# EUROPEAN PATENT SPECIFICATION

(11) **EP 4 486 452 B1**
(45) Date of publication and mention of the grant of the patent: **02.09.2026**
(21) Application number: 23707115.4
(22) Date of filing: 01.03.2023
(51) Int. Cl.: A61P 31/04, C07D 249/04, C07D 249/06, C07D 417/04

(54) **INHIBITORS OF PSEUDOMONAS AERUGINOSA VIRULENCE FACTOR LASB**
INHIBITOREN DES PSEUDOMONAS-AERUGINOSA-VIRULENZFAKTORS LASB
INHIBITEURS DE LASB FACTEUR DE VIRULENCE PSEUDOMONAS AERUGINOSA

(30) Priority: 01.03.2022 EP 22290011
(43) Date of publication of application: 08.01.2025
(73) Proprietor: Helmholtz-Zentrum für Infektionsforschung GmbH, 38124 Braunschweig (DE); Université de Lille, 59800 Lille (FR)
(72) Inventor: CAMBERLEIN, Virgyl, 38124 Braunschweig (DE); HIRSCH, Anna, 38124 Braunschweig (DE); HAUPENTHAL, Jörg, 38124 Braunschweig (DE); KANY, Andreas, 38124 Braunschweig (DE); KONSTANTINOVIC, Jelena, 38124 Braunschweig (DE); ROX, Katharina, 38124 Braunschweig (DE); PARK, Yu Mi, 38124 Braunschweig (DE); MÜLLER, Rolf, 38124 Braunschweig (DE); DEPREZ-POULAIN, Rebecca, 59006 Lille (FR); DEPREZ, Benoit, 59006 Lille (FR); ALHAYEK, Alaa, 38124 Braunschweig (DE); KLEIN, Andreas, 38124 Braunschweig (DE); SHAFIEI, Roya, 38124 Braunschweig (DE)
(74) Representative: Forstmeyer, Dietmar
(86) International application number: PCT/EP2023/055152
(87) International publication number: WO 2023/166039

(56) References cited:
- KANY ANDREAS M. ET AL: "Tackling Pseudomonas aeruginosa Virulence by a Hydroxamic Acid-Based LasB Inhibitor", vol. 13, no. 9, 8 August 2018 (2018-08-08), pages 2449 - 2455, XP055941902, ISSN: 1554-8929, Retrieved from the Internet <URL:https://pubs.acs.org/doi/pdf/10.1021/acschembio.8b00257> DOI: 10.1021/acschembio.8b00257
- HANSEN METTE R ET AL: "Triazole-containingN-acyl homoserine lactones targeting the quorum sensing system inPseudomonas aeruginosa", BIOORGANIC, ELSEVIER, AMSTERDAM, NL, vol. 23, no. 7, 31 January 2015 (2015-01-31), pages 1638 - 1650, XP029204965, ISSN: 0968-0896, DOI: 10.1016/J.BMC.2015.01.038
- YAHIAOUI SAMIR ET AL: "N -Aryl mercaptoacetamides as potential multi-target inhibitors of metallo-[beta]-lactamases (MBLs) and the virulence factor LasB from Pseudomonas aeruginosa", vol. 12, no. 10, 20 October 2021 (2021-10-20), pages 1698 - 1708, XP055941997, Retrieved from the Internet <URL:https://pubs.rsc.org/en/content/articlepdf/2021/md/d1md00187f> DOI: 10.1039/D1MD00187F
- ALHAYEK ALAA ET AL: "Discovery and Characterization of Synthesized and FDA-Approved Inhibitors of Clostridial and Bacillary Collagenases", JOURNAL OF MEDICINAL CHEMISTRY, vol. 65, no. 19, 26 September 2022 (2022-09-26), US, pages 12933 - 12955, XP093031705, ISSN: 0022-2623, Retrieved from the Internet <URL:https://pubs.acs.org/doi/pdf/10.1021/acs.jmedchem.2c00785> DOI: 10.1021/acs.jmedchem.2c00785

## Description

The present invention relates to novel inhibitors of the *Pseudomonas aeruginosa* virulence factor LasB. These compounds are useful in the treatment of bacterial infections, especially caused by *P. aeruginosa.*

*P. aeruginosa* is a Gram-negative bacterium, which is ranked by the WHO as one of the most critical pathogens today (World Health Organization. Global Priority List of Antibiotic-Resistant Bacteria to Guide Research, Discovery, and Development of New Antibiotics. WHO **2017).** This opportunistic bacterium causes around 10% of hospital-acquired infections and has a high occurrence among immunocompromised and cystic-fibrosis patients (Magill, S. S.; Edwards, J. R.; Bamberg, W.; Beldavs, Z. G.; Dumyati, G.; Kainer, M. A.; Lynfield, R.; Maloney, M.; McAllister-Hollod, L.; Nadle, J.; et al. N. Engl. J. Med. 2014, 370, 1198-1208; Richards, M. J.; Edwards, J. R.; Culver, D. H.; Gaynes, R. P. Pediatrics 1999, 103, e39; Valenza, G.; Tappe, D.; Turnwald, D.; Frosch, M.; König, C.; Hebestreit, H.; Abele-Horn, M. J. Cyst. Fibros. 2008, 7, 123-127; Sordé, R.; Pahissa, A.; Rello, J. Infect. Drug Resist. 2011, 4, 31-41). The development of potent antibiotics is urgently needed due to the lack of efficient therapeutics on the market (Mesaros, N.; Nordmann, P.; Plésiat, P.; Roussel-Delvallez, M.; Eldere, J. Van; Glupczynski, Y.; Laethem, Y. Van; Jacobs, F.; Lebecque, P.; Malfroot, A.; et al. Clin. Microbiol. Infect. 2007, 13, 560-578; Taubes, G. Science 2008, 321, 356-361). This task is complicated by the high intrinsic resistance of the pathogen (Hancock, R. E. W.; Speert, D. P. Drug Resist. Updat. 2000, 3, 247-255; Strateva, T.; Yordanov, D. J. Med. Microbiol. 2009, 58, 1133-1148).

*P. aeruginosa* has a particularly low permeability of the outer membrane, preventing the entrance of antibiotics into the cell (Nikaido, H.; Yoshimura, F. J. Bacterial. 1982, 152, 636-642). Additionally, its efflux pumps efficiently transport undesired antimicrobials out of the cell and its inducible chromosomal β-lactamases are able to inactivate the corresponding β-lactam antibiotics (Pos, K. M. Biochim. Biophys. Acta - Proteins Proteomics 2009, 1794, 782-793; Moreira, M. A. S.; Souza, E. C. de; Moraes, C. A. de. Brazilian J. Microbiol. 2004, 35, 19-28; Hancock, R. E. W.; Woodruff, W. A. Clin. Infect. Dis. 1988, 10, 770-775; Li, X. Z.; Livermore, D. M.; Nikaido, H. Antimicrob. Agents Chemother. 1994, 38, 1732-1741). An additional difficulty is the rising mutational resistance rate of *P. aeruginosa* strains (Thomson, J. M.; Bonomo, R. A. Curr. Opin. Microbiol. 2005, 8, 518-524). For example, fluoroquinolone and aminoglycoside resistance have reached up to 30% (Gasink, L. B.; Fishman, N. O.; Weiner, M. G.; Nachamkin, I.; Bilker, W. B.; Lautenbach, E. Am. J. Med. 2006, 119, 19-25; Poole, K. Antimicrob. Agents Chemother. 2005, 49, 479-487). Furthermore, resistances against almost all drugs used for the treatment of infections with *P*. *aeruginosa* (for example cephalosporins and carbapenems) are described (Obritsch, M. D.; Fish, D. N.; MacLaren, R.; Jung, R. Pharmacotherapy 2005, 25, 1353-1364; ASCP Susceptibility Testing Group. United States Geographic Bacteria Susceptibility Patterns. Am. J. Clin. Pathol. 1996, 106, 275-281). These facts emphasize the urgent need for new therapeutic options.

Besides the traditional strategy to target bacterial viability, recently, special attention has been paid to targeting bacterial virulence as an alternative approach for fighting microbial infections (Dickey, S. W.; Cheung, G. Y. C.; Otto, M. Nat. Rev. Drug Discov. 2017, 16, 457-471; Rasko, D. A.; Sperandio, V. Nat. Rev. Drug Discov. 2010, 9, 117-128). Virulence factors are common among pathogenic bacteria and act by damaging their host or evading its immune response (Strateva, T.; Mitov, I. Ann. Microbiol. 2011, 61, 717-732). Inhibitors of virulence factors reduce bacterial virulence and in this way enable clearance of the pathogen by either the host's immune system or with the help of antibiotics (Heras, B.; Scanlon, M. J.; Martin, J. L. Br. J. Clin. Pharmacol. 2015, 79, 208-215; Clatworthy, A. E.; Pierson, E.; Hung, D. T. Nat. Chem. Biol. 2007, 3, 541-548). Although only a few compounds have reached clinical approval yet, many *in vitro* and *in vivo* studies support the efficacy of this strategy (Wagner, S.; Sommer, R.; Hinsberger, S.; Lu, C.; Hartmann, R. W.; Empting, M.; Titz, A. J. Med. Chem. 2016, 59, 5929-5969). The main advantage of this new approach is the reduced selection pressure on the bacteria and thus the lower risk for resistance development. In addition, these antivirulence agents do not harm the commensal bacteria.

A well-known antivirulence target of *P*. *aeruginosa* is the elastase LasB. This extracellular zinc-containing protease plays a role in the pathogenic invasion of tissues and is thought to be predominantly relevant during acute infections (Liu, P. V. J. Infect. Dis. 1974, 130, S94-S99). It has the ability to break down elastin, which is an important component of lung tissue and blood vessels (Morihara, K.; Tsuzuki, H.; Oka, T.; Inoue, H.; Ebata, M. J. Biol. Chem. 1965, 240, 3295-3304). Additionally, LasB can degrade fibrin, collagen and surfactant proteins in the lung and is also involved in the reduction of the host's immunity by inactivation of human immunoglobulins A and G, cytokines gamma-interferon and tumor necrosis factor α as well as the degradation of the antibacterial peptide LL-37 (Heck, L. W.; Morihara, K.; McRae, W. B.; Miller, E. J. Infect. Immun. 1986, 51, 115-118; Heck, L. W.; Alarcon, P. G.; Kulhavy, R. M.; Morihara, K.; Mestecky, M. W.; Russell, J. F. J. Immunol. 1990, 144, 2253-2257; Holder, I. A.; Wheeler, R. Can. J. Microbiol. 1984, 30, 1118-1124; Galloway, D. R. Mol. Microbiol. 1991, 5, 2315-2321; Parmely, M.; Gale, A.; Clabaugh, M.; Horvat, R.; Zhou, W. Infect. Immun. 1990, 58, 3009-3014; Mariencheck, W. I.; Alcorn, J. F.; Palmer, S. M.; Wright, J. R. Am. J. Respir. Cell Mol. Biol. 2003, 28, 528-537; Schmidtchen, A. et al. Mol. Microbiol. 2002, 46, 157-168).

Since LasB is an attractive antivirulence target, several LasB inhibitors have been described in the literature up to now: natural products such as streptomyces metalloprotease inhibitor TK-23 (SMPI) from *Streptomyces nigrescens* and phosphoramidon (Oda, K.; Koyama, T.; Murao, S. Biochim. Biophys. Acta 1979, 571, 147-156; Nishino, N.; Powers, J. C. J. Biol. Chem. 1979, 255, 3482-19), small peptides containing metal-chelating motifs such as thiol or hydroxamate groups (Kessler, E.; Israel, M.; Landshman, N.; Chechick, A.; Blumberg, S. Infect. Immun. 1982, 38, 716-723; Cathcart, G. R. A.; Quinn, D.; Greer, B.; Harriott, P.; Lynas, J. F.; Gilmore, B. F.; Walker, B. Antimicrob. Agents Chemother. 2011, 55, 2670-2678; Burns, F. R.; Paterson, C. A.; Gray, R. D.; Wells, J. T. Antimicrob. Agents Chemother. 1990, 34, 2065-2069) and small synthetic molecules with hydroxamate, thiol or mercaptoacetamide groups (Zhu, J.; Cai, X.; Harris, T. L.; Gooyit, M.; Wood, M.; Lardy, M.; Janda, K. D. Chem. Biol. 2015, 22, 483-491; Adekoya, O. A.; Sjøli, S.; Wuxiuer, Y.; Bilto, I.; Marques, S. M.; Santos, M. A.; Nuti, E.; Cercignani, G.; Rossello, A.; Winberg, J. O.; et al. Eur. J. Med. Chem. 2015, 89, 340-348) as well as compounds based on tropolone (Fullagar, J. L.; Garner, A. L.; Struss, A. K.; Day, J. A.; Martin, D. P.; Yu, J.; Cai, X.; Janda, K. D.; Cohen, S. M. Chem. Commun. 2013, 49, 3197-3199).

Triazole-containing *N*-acyl homoserine lactones targeting the quorum sensing system in *Pseudomonas aeruginosa* are described in Hansen et al. Bioorg. Med. Chem. 2015, 23, 1638-1650.

Recently, a group of N-aryl mercaptoacetamides as potent LasB inhibitors has been described (Kany, A. M.; Sikandar, A.; Haupenthal, J.; Yahiaoui, S.; Maurer, C. K.; Proschak, E.; Köhnke, J.; Hartmann, R. W. ACS Infect. Dis. 2018, 4, 988-997). The crystal structure of the most promising compound described therein revealed the presence of two molecules in the binding pocket. In order to occupy the active site with a single molecule, a series of *N*-benzylamide/*N*-alkylamide derivatives were synthesized. However, this approach failed to improve the inhibitory potency of the initial ligand. Further LasB inhibitors are disclosed in WO 2022/043322 A1; Kany A. M., Sikandar A., Yahiaoui S., Haupenthal J., Walter I., Empting M., Köhnke J., Hartmann R. W. "Tackling Pseudomonas aeruginosa Virulence by a Hydroxamic Acid-Based LasB Inhibitor". ACS Chem Biol. 2018, 13, 2449-2455; Yahiaoui S., Voos K., Haupenthal J., Wichelhaus T. A., Frank D., Weizel L., Rotter M., Brunst S., Kramer J. S., Proschak E., Ducho C., Hirsch A. K. H. "N-Aryl mercaptoacetamides as potential multi-target inhibitors of metallo-β-lactamases (MBLs) and the virulence factor LasB from Pseudomonas aeruginosa". RSC Med Chem. 2021, 12, 1698-1708, doi:10.1039/D1MD00187F; and Konstantinovic J., Yahiaoui S., Alhayek A., Haupenthal J., Schönauer E., Andreas A., Kany A. M., Müller R., Koehnke J., Berger F. K., Bischoff M., Hartmann R. W., Brandstetter H., Hirsch A. K. H. "N-Aryl-3-mercaptosuccinimides as Antivirulence Agents Targeting Pseudomonas aeruginosa Elastase and Clostridium Collagenases". J Med Chem. 2020. 63, 8359-8368. doi: 10.1021/acs.jmedchem.0c00584.

It has been the object of the present invention to provide novel inhibitors of the *P*. *aeruginosa* virulence factor LasB.

The present invention provides compounds of formula (II): 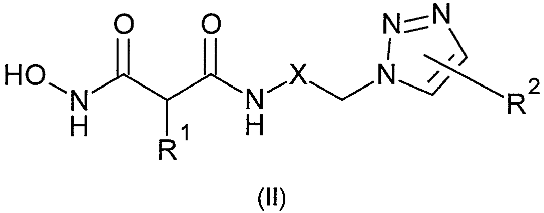 wherein
X is an optionally substituted arylene group or an optionally substituted heteroarylene group;
R¹ is a C₁₋₆ alkyl group; or a group of formula -CH₂-R¹¹, wherein R¹¹ is a phenyl group or a cyclopropyl group; and
R² is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a pharmaceutically acceptable salt thereof;
wherein the optional substituents are selected from fluorine, chlorine, bromine and iodine and OH, =O, SH, NH₂, -SO₃H, -SO₂NH₂, -COOH, -COOMe, -COMe, - NHSO₂Me, -SO₂NMe₂, -CH₂NH₂, -NHAc, -SO₂Me, -CONH₂, -CN, -NHCONH₂, - NHC(NH)NH₂, -NOHCH₃, -N₃, -NO₂, C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups.

Further preferably, X is an optionally substituted phenylene group or an optionally substituted heteroarylene group having 5 or 6 ring atoms that are selected from C, N, O and S.

Especially preferably, X is a 1,3 phenylene group.

Further especially preferably, X is a 1,4 phenylene group.

Further preferably, R' is a C₁₋₄ alkyl group; or a group of formula -CH₂-R¹¹, wherein R¹¹ is a phenyl group or a cyclopropyl group.

Especially preferably, R¹ is an iso-butyl group (i.e., a group of formula -CH₂CH(CH₃)₂).

Moreover preferably, R¹ is an iso-propyl group (i.e., a group of formula -CH(CH₃)₂).

Further preferably, R² is a group of formula -CH₂-NH-SO₂-R³ or -CH₂-N(CH₃)-SO₂-R³, wherein R³ is an optionally substituted phenyl group, an optionally substituted benzyl group, an optionally substituted C₃₋₁₀ cycloalkyl group, an optionally substituted -CH₂-C₃₋₁₀ cycloalkyl group, a C₁₋₆ alkyl group or a C₁₋₆ heteroalkyl group.

Moreover preferably, R² is a group of formula -CH₂-Y-R⁴, wherein Y is selected from a bond, O, NH, S and NHCO; and R⁴ is hydrogen, a C₁₋₆ heteroalkyl group or an optionally substituted phenyl group (preferably, R⁴ is an optionally substituted phenyl group).

Further preferably, R² is an optionally substituted phenyl group.

Preferably, the optional substituents (preferably, 1 or 2 substituent(s)) at group R², R³ or R⁴ are independently selected from halogen atoms (e.g., F, Cl, Br, I), OH, NH₂, COOH, =O, phenyl, C₁₋₆ alkyl groups and C₁₋₆ heteroalkyl groups.

Moreover preferably, R² is selected from the following groups:

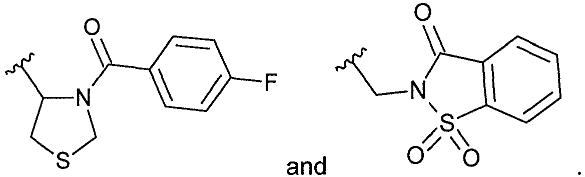

The most preferred compounds of the present invention are the compounds disclosed in the examples, or a salt thereof.

It is further preferred to combine the preferred embodiments of the present invention in any desired manner (e.g., any embodiment for R¹ may be combined with any embodiment of R²).

The term "optionally substituted" refers to a group which is unsubstituted or substituted by one or more (especially by one, two or three; preferably by one or two) substituents.

If a group (e.g., group R¹ and/or group R²) comprises more than one substituent, these substituents are independently selected, i.e., they may be the same or different.

If a group (e.g., group R¹ and/or group R²) is substituted by a cyclic group, such as e.g., a cycloalkyl group or a heterocycloalkyl group, this cyclic group may be bonded to this group (e.g., group R¹ and/or group R²) via a single or double bond or this cyclic group may be annulated or fused to said group (e.g., group R¹ and/or group R²). Isatin is an example for a substituted phenyl group.

Examples for substituents are fluorine, chlorine, bromine and iodine and OH, =O, SH, NH₂, -SO₃H, -SO₂NH₂, -COOH, -COOMe, -COMe (Ac), -NHSO₂Me, -SO₂NMe₂, - CH₂NH₂, -NHAc, -SO₂Me, -CONH₂, -CN, -NHCONH₂, -NHC(NH)NH₂, -NOHCH₃, -N₃ and -NO₂ groups. Further examples of substituents are C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups; especially C₁-C₆ alkyl, C₂-C₆ alkenyl, C₂-C₆ alkynyl, C₁-C₆ heteroalkyl, C₃-C₁₀ cycloalkyl, C₁-C₉ heterocycloalkyl, C₄-C₁₂ alkylcycloalkyl, C₁-C₁₁ heteroalkylcycloalkyl, C₆-C₁₀ aryl, C₁-C₉ heteroaryl, C₇-C₁₂ aralkyl and C₁-C₁₁ heteroaralkyl groups, further preferably C₁-C₆ alkyl and C₁-C₆ heteroalkyl groups.

Preferred substituents are halogen atoms (e.g., F, Cl, Br, I) and groups of formula - OH, =O, -O-C₁₋₆ alkyl (e.g., -OMe, -OEt, -O-*n*Pr, -O-*i*Pr, -O-*n*Bu, -O-*i*Bu and -O-*t*Bu), - NH₂, -NHC₁₋₆ alkyl, -N(C₁₋₆ alkyl)₂, -COOH, -COO-C₁₋₆ alkyl (e.g., -COOMe), -CO-C₁₋₆ alkyl (e.g., -COMe), -COCF₃, -NHSO₂Me, -SO₂NMe₂, -OCH₂CH₂OCH₃, -SO₃H, - SO₂NH₂, -CONH₂, -CH₂NH₂, -CN, -C₁₋₆ alkyl (e.g., -Me, -Et, -*n*Pr, -*i*Pr, -*n*Bu, -*i*Bu, -*t*Bu and -CF₃), -C₁₋₆ heteroalkyl, -SH, -S-CO-C₁₋₆ alkyl, -S-C₁₋₆ alkyl, -NHAc, -NO₂, -C≡CH, -NHCONH₂, -SO₂Me, -SO₂CF₃, phenyl, -CO-4-fluorophenyl, -C₃₋₆ cycloalkyl (e.g. cyclopropyl, cyclobutyl) and heterocycloalkyl containing 3 to 6 ring atoms selected from C, N, S and O.

Moreover preferred substituents are halogen atoms (e.g., F, Cl, Br, I), OH, NH₂, COOH, =O, phenyl, C₁₋₆ alkyl groups and C₁₋₆ heteroalkyl groups.

Further preferred substituents are halogen atoms (e.g., F, Cl, Br, I) and groups of formula -OH, =O, -O-C₁₋₆ alkyl (e.g., -OMe, -OEt, -O-*n*Pr, -O-*i*Pr, -O-*n*Bu, -O-*i*Bu and - O-tBu), -NH₂, -NHC₁₋₆ alkyl, -N(C₁₋₆ alkyl)₂, -COOH, -COO-C₁₋₆ alkyl (e.g., -COOMe), - CO-C₁₋₆ alkyl (e.g., -COMe), -OCH₂CH₂OCH₃, -NHSO₂Me, -SO₂NMe₂, -SO₃H, - SO₂NH₂, -CONH₂, -CH₂NH₂, -CN, -C₁₋₆ alkyl (e.g., -Me, -Et, -*n*Pr, -*i*Pr, -*n*Bu, -*i*Bu, -*t*Bu and -CF₃), -SH, -S-CO-C₁₋₆ alkyl, -S-C₁₋₆ alkyl, -NHCOOtBu, -NHAc, phenyl, -NO₂, - C≡CH, -NHCONH₂, -SO₂Me and cyclopropyl.

The substituent(s) is/are especially preferably independently selected from halogen (especially F and CI), =O, -C₁₋₆ alkyl (e.g., -Me), -CF₃, -O-C₁₋₆ alkyl (e.g., -OMe), -OH, -NH₂, NHAc, -COOH, -CONH₂, -COO-C₁₋₆ alkyl (e.g., -COOMe), -O-C₁₋₆ alkyl (e.g., - COMe) and -NO₂.

The suffix "-ene" like e.g., in "phenylene" refers to the corresponding divalent group.

The expression alkyl refers to a saturated, straight-chain or branched hydrocarbon group that contains from 1 to 20 carbon atoms, preferably from 1 to 15 carbon atoms, especially from 1 to 10 (e.g., 1, 2, 3 or 4) carbon atoms, for example a methyl (Me, CH₃), ethyl (Et), *n*-propyl (nPr), *iso*-propyl (iPr), *n*-butyl (*n*Bu), *iso*-butyl (*i*Bu), *sec*-butyl (sBu), *tert-*butyl (tBu), *n*-pentyl, *iso*-pentyl, *n*-hexyl, 2,2-dimethylbutyl or *n-*octyl group.

Especially preferred alkyl groups are C₁₋₆ alkyl groups; moreover preferred alkyl groups are C₁₋₄ alkyl groups.

The expression C₁₋₆ alkyl refers to a saturated, straight-chain or branched hydrocarbon group that contains from 1 to 6 carbon atoms. The expression C₁₋₄ alkyl refers to a saturated, straight-chain or branched hydrocarbon group that contains from 1 to 4 carbon atoms. Examples are a methyl, ethyl, *n*-propyl, *iso*-propyl, *n*-butyl, *iso*-butyl, sec-butyl or *tert-*butyl group.

The expressions alkenyl and alkynyl refer to at least partially unsaturated, straight-chain or branched hydrocarbon groups that contain from 2 to 20 carbon atoms, preferably from 2 to 15 carbon atoms, especially from 2 to 10 (e.g., 2, 3 or 4) carbon atoms, for example an ethenyl (vinyl), propenyl (allyl), isopropenyl, butenyl, ethynyl (acetylenyl), propynyl (e.g., propargyl), butynyl, isoprenyl or hex-2-enyl group. Preferably, alkenyl groups have one or two (especially preferably one) double bond(s), and alkynyl groups have one or two (especially preferably one) triple bond(s).

Furthermore, the terms alkyl, alkenyl and alkynyl refer to groups in which one or more hydrogen atoms have been replaced by a halogen atom (preferably F or Cl) such as, for example, a 2,2,2-trichloroethyl or a trifluoromethyl group.

The expression heteroalkyl refers to an alkyl, alkenyl or alkynyl group in which one or more (preferably 1 to 8; especially preferably 1, 2, 3 or 4) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy. Furthermore, the term heteroalkyl refers to groups in which one or more hydrogen atoms have been replaced by a halogen atom (preferably F or CI).

Preferably, a heteroalkyl group contains from 1 to 12 carbon atoms and from 1 to 8 heteroatoms selected from oxygen, nitrogen and sulfur (especially oxygen and nitrogen). Especially preferably, a heteroalkyl group contains from 1 to 6 (e.g., 1, 2, 3 or 4) carbon atoms and 1, 2, 3 or 4 (especially 1, 2 or 3) heteroatoms selected from oxygen, nitrogen and sulfur (especially oxygen and nitrogen). The term C₁-C₁₀ heteroalkyl refers to a heteroalkyl group containing from 1 to 10 carbon atoms and 1, 2, 3, 4, 5 or 6 heteroatoms selected from O, S and/or N (especially O and/or N). The term C₁-C₆ heteroalkyl refers to a heteroalkyl group containing from 1 to 6 carbon atoms and 1, 2, 3 or 4 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N).

Further preferably, the expression heteroalkyl refers to an alkyl group as defined above (straight-chain or branched) in which one or more (preferably 1 to 6; especially preferably 1, 2, 3 or 4) carbon atoms have been replaced by an oxygen, sulfur or nitrogen atom or a CO group or a SO group or a SO₂ group; this group preferably contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen); this group may preferably be substituted by one or more (preferably 1 to 6; especially preferably 1, 2, 3 or 4) fluorine, chlorine, bromine or iodine atoms or OH, =O, SH, =S, NH₂, =NH, N₃, CN or NO₂ groups.

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-SO₂-Y^{a}-, R^{a}-SO₂-N(R^{b})-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, *n*-propyloxy, *iso*-propyloxy, *n*-butoxy, *tert*-butyloxy, methoxymethyl, ethoxymethyl, -CH₂CH₂OH, -CH₂OH, -SO₂Me, -NHAc, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, *N*-ethyl-*N*-methylcarbamoyl or *N*-methylcarbamoyl. Further examples of heteroalkyl groups are nitrile (-CN), isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, propellane (e.g., [1.1.1]propellane) tetraline, cyclopentylcyclohexyl, fluorocyclohexyl or cyclohex-2-enyl group. Preferably, the expression cycloalkyl refers to a saturated cyclic group that contains one or more rings (preferably 1 or 2), and contains from 3 to 14 ring carbon atoms, preferably from 3 to 10 (especially 3, 4, 5, 6 or 7) ring carbon atoms.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) and 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl (e.g., -N(CH₂CH₂)₂O), urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings and from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings and from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, NH₂, N₃ or NO₂ groups. Examples are the phenyl (Ph), naphthyl, biphenyl, 2-fluorophenyl, anilinyl, 3-nitrophenyl or 4-hydroxyphenyl group.

The expression heteroaryl refers to an aromatic group that contains one or more rings and from 5 to 14 ring atoms, preferably from 5 to 10 (especially 5 or 6 or 9 or 10) ring atoms, comprising one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g., 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, 4-hydroxypyridyl (4-pyridonyl), 3,4-hydroxypyridyl (3,4-pyridonyl), oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g., 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are phenylcyclopentyl, cyclohexylphenyl as well as groups derived from toluene, xylene, mesitylene, styrene, benzyl chloride, o-fluorotoluene, 1*H*-indene, tetraline, dihydronaphthalene, indanone, cumene, fluorene and indane. An aralkyl group preferably contains one or two aromatic ring systems (especially 1 or 2 rings), each containing from 6 to 10 carbon atoms and one or two alkyl, alkenyl and/or alkynyl groups containing from 1 or 2 to 6 carbon atoms and/or a cycloalkyl group containing 3, 4, 5, 6 or 7 ring carbon atoms.

The expression heteroaralkyl refers to groups containing both aryl and/or heteroaryl groups and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (especially 1 or 2 rings), each containing from 5 or 6 to 9 or 10 ring atoms (preferably selected from C, N, O and S) and one or two alkyl, alkenyl and/or alkynyl groups containing 1 or 2 to 6 carbon atoms and/or one or two heteroalkyl groups containing 1 to 6 carbon atoms and 1, 2 or 3 heteroatoms selected from O, S and N and/or one or two cycloalkyl groups each containing 3, 4, 5, 6 or 7 ring carbon atoms and/or one or two heterocycloalkyl groups, each containing 3, 4, 5, 6 or 7 ring atoms comprising 1, 2, 3 or 4 oxygen, sulfur or nitrogen atoms.

Examples are arylheteroalkyl, arylheterocycloalkyl, arylheterocycloalkenyl, arylalkylheterocycloalkyl, arylalkenyl heterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroaryl-heterocycloalkyl, heteroarylheterocycloalkenyl, heteroarylalkylcycloalkyl, heteroaryl-alkylheterocycloalkenyl, heteroarylheteroalkylcycloalkyl, heteroarylheteroalkyl-cycloalkenyl and heteroarylheteroalkylheterocycloalkyl groups, the cyclic groups being saturated or mono-, di- or tri-unsaturated. Specific examples are a tetrahydroisoquinolinyl, benzoyl, phthalidyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, NH₂, =NH, N₃ or NO₂ groups.

The term halogen refers to F, Cl, Br or I.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated or fused or bridged.

Owing to their substitution, the compounds of the present invention may contain one or more centers of chirality. The present invention therefore includes both all pure enantiomers and all pure diastereomers and also mixtures thereof in any mixing ratio. The present invention moreover also includes all *cis*/*trans*-isomers of the compounds of the present invention and also mixtures thereof. The present invention moreover includes all tautomeric forms of the compounds of the present invention.

The present invention further provides pharmaceutical compositions comprising one or more compounds described herein or a pharmaceutically acceptable salt, solvate or hydrate thereof, optionally in combination with one or more carrier substances and/or one or more adjuvants. The pharmaceutical composition of the present invention may contain a further antibacterial compound.

The compounds or pharmaceutical compositions of the present invention may be administered in combination with a further antibacterial compound.

The present invention furthermore provides compounds or pharmaceutical compositions as described herein for use in the treatment of bacterial infections, especially caused by *P. aeruginosa.*

The present invention further provides a compound as described herein or a pharmaceutical composition as defined herein for the preparation of a medicament for the treatment of bacterial infections, especially caused by *P. aeruginosa.*

Examples of pharmacologically acceptable salts of sufficiently basic compounds are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein.

The compounds described herein may be solvated, especially hydrated. The solvation/ hydration may occur during the process of production or as a consequence of the hygroscopic nature of the initially water-free compounds. The solvates and/or hydrates may e.g. be present in solid or liquid form.

The therapeutic use of the compounds described herein, their pharmacologically acceptable salts, solvates and hydrates, respectively, as well as formulations and pharmaceutical compositions also lie within the scope of the present invention.

In general, the compounds and pharmaceutical compositions described herein will be administered by using the established and acceptable modes known in the art.

For oral administration, such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g., liquid aerosol), transdermal, for example via an transdermal drug delivery system (TDDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules, one may use excipients as are e.g., vegetable, petroleum, animal or synthetic oils, wax, fat, and polyols. For the production of liquid solutions, emulsions or suspensions or syrups one may use as excipients e.g., water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations, one may use compressed gases suitable for this purpose, e.g., oxygen, nitrogen and carbon dioxide. The pharmaceutically useful agents may also contain additives for conservation, stabilization, e.g., UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 1 mg to about 10,000 mg, preferably from about 5 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

As can be taken from the following examples, besides their high activity, the advantages of the compounds of the present invention are for example their excellent selectivity towards human off-targets, their chemical stability and extremely low toxicity.

### EXAMPLES

### I. Chemistry

### 1. General information

Solvents for synthesis, analysis and purification were purchased as analytical-grade from commercial suppliers and used directly without further purification. Chemical reagents were purchased as reagent-grade and used without further purification.

NMR spectra were recorded on a Bruker UltraShield Plus 500 MHz device. LC-MS analysis were measured on a LC-MS system, consisting of a Thermo Scientific Dionex UltiMate 3000 pump, autosampler, column compartment, and detector (Thermo Fisher Scientific, Dreieich, Germany) and ESI quadrupole MS (MSQ Plus or ISQ EC, Thermo Fisher Scientific, Dreieich, Germany). High-resolution mass was determined by LCMS/MS using Thermo Scientific Q Exactive Focus Orbitrap LC-MS/ MS system. Flash chromatography was performed using either a Teledyne ISCO CombiFlash Rf+ 150 or a Teledyne ISCO CombiFlash NEXTGEN 300+ equipped with RediSepRf silica columns. Preparative HPLC was performed on a Thermo Scientific Dionex Ultimate 3000 system.

### 2. General synthesis schemes

The compounds of the present invention can be prepared according to the following synthesis schemes:

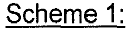

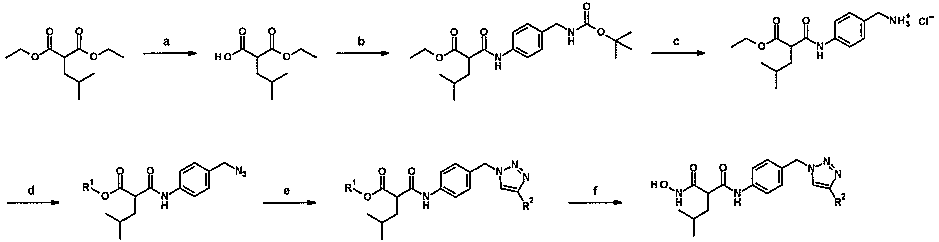

Reagents and conditions: a) NaOH, EtOH/H₂O (4 :1), rt., overnight, 80%; b) tert-butyl *N*-[(4-aminophenyl)methyl]carbamate, HBTU, Et₃N, DMF, rt., overnight, 47% ; c) HCl 4M in dioxane, EtOH, 0 °C to rt., overnight, quantit. yield; d) azide-*N*-diazoimidazole-1-sulfonamide hydrogen sulfate, K₂CO₃, ZnCl₂, DIPEA, MeOH, 0 °C to rt., overnight, 58%; f) alkynes, CuSO₄ (5H₂O), sodium ascorbate, DMF/H₂O (1.2:1), rt., overnight, 45-quant. yield; e) aq. hydroxylamine (50% in water w/w), KCN (cat.), MeOH, rt., overnight to 72 h, 10-85%.

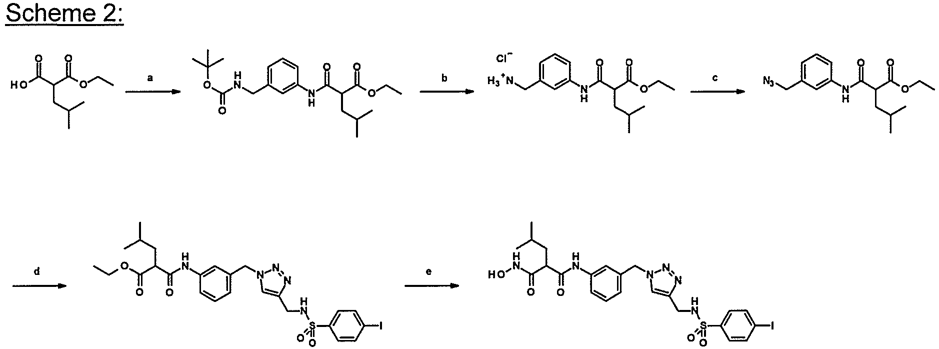

Reagents and conditions: a) tert-butyl *N*-[(3-aminophenyl)methyl]carbamate, HBTU, Et₃N, DMF, rt., overnight, 74%; b) HCl 4M in dioxane, EtOH, 0 °C to rt., overnight, quantit. yield; c) azide-*N*-diazoimidazole-1-sulfonamide hydrogen chloride, K₂CO₃, ZnCl₂, DIPEA, EtOH, 0 °C to rt., overnight, 31%; d) **93,** CuSO₄ (5H₂O), sodium ascorbate, DMF/H₂O (1.2:1), rt., overnight, 86%; e) aq. hydroxylamine (50% in water w/w), KCN (cat.), MeOH, 48 h, overnight, 7%.

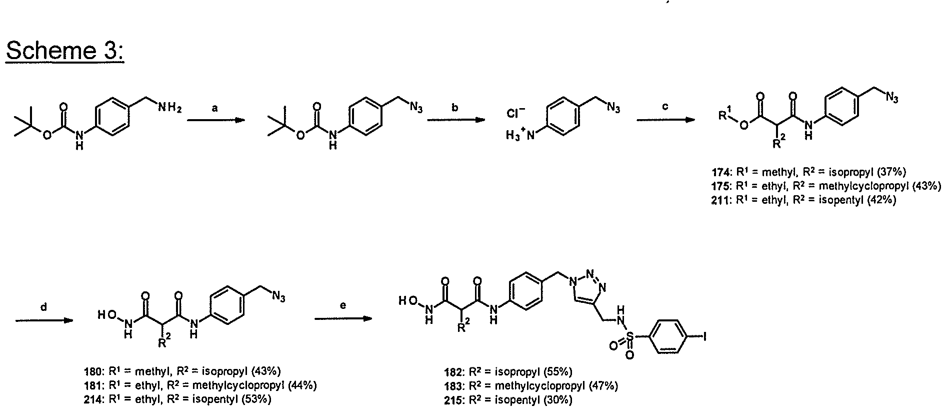

Reagents and conditions : a) azide-*N*-diazoimidazole-1-sulfonamide hydrogen sulfate, K₂CO₃, ZnCl₂, DIPEA, MeOH, 0 °C to rt., overnight, quantit. yield; b) HCl 4 M in dioxane, CH₂Cl₂, MeOH, 0 °C to rt., overnight, quant. yield; c) carboxylic acids **(174a-175a, 211a),** HBTU, Et₃N, DMF, rt., overnight, 37-43%; d) aq. hydroxylamine (50% in water w/w), KCN (cat.), MeOH, rt., overnight, 43-53%.; e) **93,** CuSO₄ (5H₂O), sodium ascorbate, DMF/H₂O (1.2:1), rt., overnight, 30-56%.

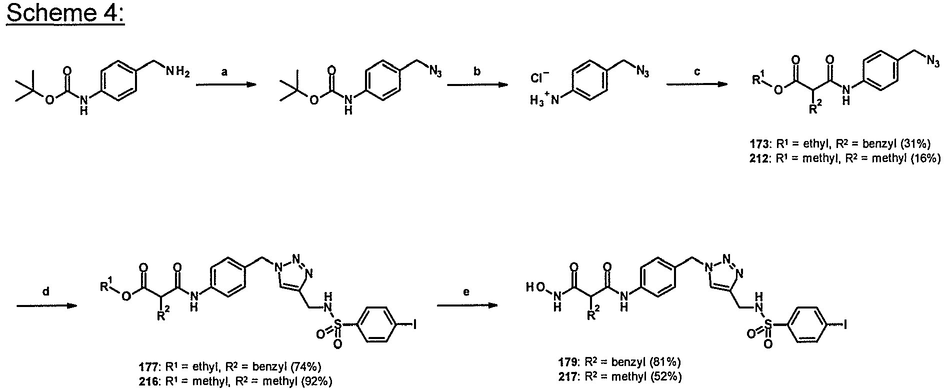

Reagents and conditions : a) azide-N-diazoimidazole-1-sulfonamide hydrogen sulfate, K₂CO₃, ZnCl₂, DIPEA, MeOH, 0 °C to rt., overnight, quantit. yield; b) HCl 4 M in dioxane, CH₂Cl₂, MeOH, 0 °C to rt., overnight, quant. yield; c) carboxylic acids **(174a-175a, 211a),** HBTU, Et₃N, DMF, rt., overnight, 37-43%; d) **93,** CuSO₄ (5H₂O), sodium ascorbate, DMF/H₂O (1.2:1), rt., overnight, 74-92%; e) aq. hydroxylamine (50% in water w/w), KCN (cat.), MeOH, rt., 16-96 h, 52-81%.

93, CuSO₄ (5H₂O), sodium ascorbate, DMF/H₂O (1.2:1), rt., overnight, 30-56%.

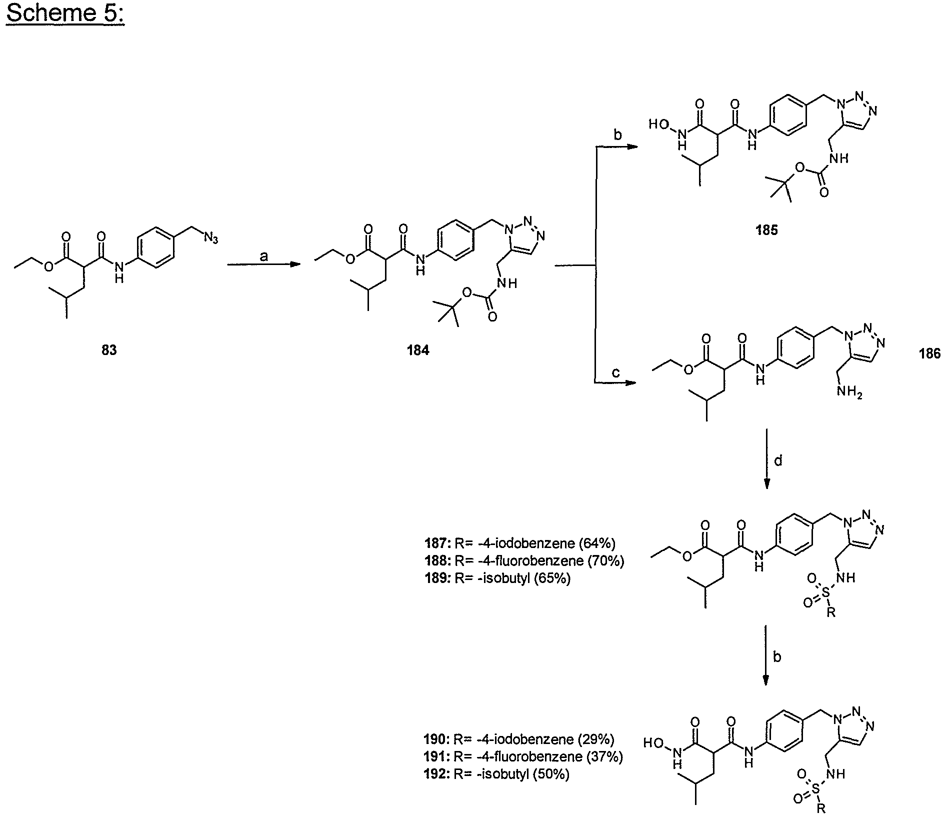

Reagents and conditions: a) *N*-Boc-propargylamine, Cp*RuCl (COD), dioxane, 80 °C, overnight, 73%; b) aq. hydroxylamine (50% in water w/w), KCN (cat.), MeOH, rt., 48 h, 26-50%; c) HCl 4M in dioxane, CH₂Cl₂, EtOH, 0 °C to rt., overnight, quant. yield; d) R-SO₂-Cl, DIPEA, DMF, 0 °c to rt., overnight, 64-70%.

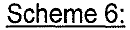

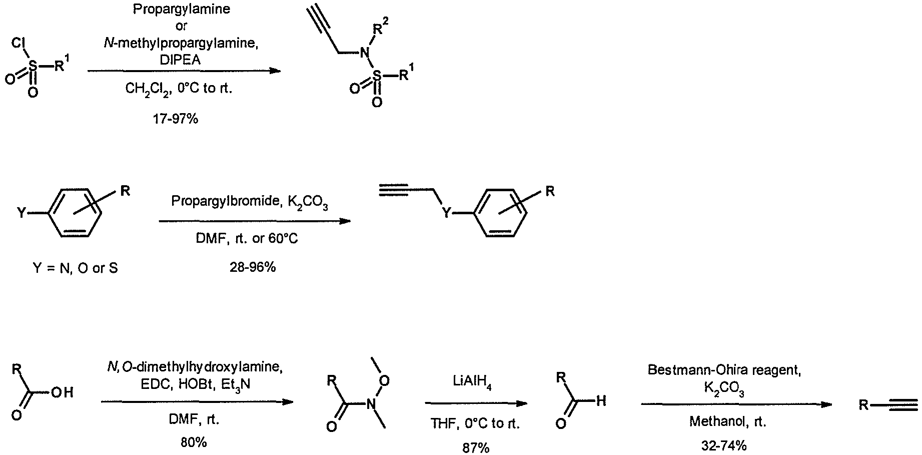

### 3. General procedures

### General procedure A: azide formation

A suspension of amine (1 eq.), ZnCl₂ (0.06 eq.) and K₂CO₃ (2-4 eq.) in anhydrous methanol (0.18-0.25 M) under inert atmosphere was cooled down to 0 °C with an icebath. Besides, anhydrous *N,N*-diisopropylethylamine (1.1-2.1 eq.) was slowly added to a solution of 1*H*-imidazole-1-sulfonyl azide; hydrogen sulfate or hydrogen chloride (1.2 eq.) solubilized in anhydrous methanol (0.3 M) under inert atmosphere (solution A). The azide-containing solution A was immediately added dropwise to the first mixture at 0 °C. Then, the cooling bath was removed, and the white mixture was stirred at room temperature overnight. The mixture was then cooled down to 0 °C, diluted with water and carefully acidified to pH = 2 with diluted aq. HCl (1 N). It was finally extracted with ethyl acetate. The combined organic layers were dried over MgSO₄, filtered and concentrated under reduced pressure or the residue was finally purified through flash chromatography to give the desired azide.

### General procedure B: amide formation

Carboxylic acid (1 eq.) and amine (1.1-1.6 eq.) were dissolved in *N,N-*dimethylformamide (0.28-0.45M) or CH₂Cl₂ (0.2 M). HBTU (1.1-1.7 eq.) or HOBt (0.1 eq.) and EDC·HCl (1.5 eq.), and trimethylamine or diisopropylethylamine (3-5 eq.) were then added, and the mixture was stirred overnight. The mixture was then washed with diluted aq. HCl (1 M), sat. aq. NaHCO₃ and sat. aq. NaCl. Combined organic layers were dried over MgSO₄, filtered and concentrated under reduced pressure or the residue was finally purified through flash chromatography to give the desired amide.

### General procedure C: sulfonamide formation

The amine (1-1.5 eq.) was dissolved in *N,N*-dimethylformamide or dichloromethane (0.04-0.10 M) and cooled down to 0°C. R1-sulfonyl chloride (1-1.2 eq.) was then carefully added followed by *N,N*-diisopropylethylamine (1.2-3 eq.). The mixture was allowed to reach room temperature and was stirred overnight. Then, the crude was diluted in aq. HCl (0.5 M) and extracted with ethyl acetate. Combined organic layers were dried over MgSO₄, filtered and concentrated under reduced pressure. The residue was finally purified through flash chromatography to afford the desired sulfonamide.

### General procedure D: aminolysis

To an ester solution (1 eq.) in methanol (0.04-0.35M), was added aq. hydroxylamine (50% w/w in water, 0.04-0.35 M) and KCN (0.1-0.5 eq.). The mixture was stirred for 16 to 96 h. Then, the solvents were removed under reduced pressure, and the residue was purified through flash chromatography.

### General procedure E: copper-catalyzed click reaction

Azide (1 eq.) and alkyne (1.0-2.5 eq.) were dissolved in *N,N*-dimethylformamide (0.03-0.09 M) before an addition of copper sulfate pentahydrate (0.2-0.4 eq.) in water (0.05-0.09 M) and sodium ascorbate (0.5-1 eq.). The resulting mixture was stirred at room temperature overnight. The mixture was then diluted in water and extracted with ethyl acetate. The combined organic layers were dried over MgSO₄, filtered and concentrated under reduced pressure. The residue was finally purified through flash chromatography affording the desired 1,4-triazole.

### General procedure F: monosaponification

Malonate diester (1 eq.) was dissolved in a mixture of methanol/water or ethanol/water (9:1-8:2, 0.32-0.58 M) and sodium hydroxide (1.2 eq.) or potassium hydroxide (1.0 eq.) was added. The reaction mixture was stirred at room temperature overnight. Then, solvents were evaporated under reduced pressure, and the remaining aqueous mixture was diluted with sat. aq. NaHCO₃ and washed with CH₂Cl₂. The aqueous layer was then carefully acidified (pH~1) with aq. HCl and extracted with CH₂Cl₂. The combined organic layers were dried over MgSO₄, filtered and concentrated under reduced pressure affording the desired product.

### General procedure G: Boc deprotection

The Boc-protected intermediate was dissolved in a mixture of ethanol and dichloromethane (1:1, 0.09-0.11 M), and the reaction was cooled down to 0 °C before addition of 4 N HCl in dioxane (0.18-0.22 M). The mixture was stirred at room temperature overnight. Then, solvents were evaporated to give the desired compound.

### 4. Synthesis of compounds

### 1-(4-Chlorophenyl)-N-prop-2-ynyl-methanesulfonamide (147)

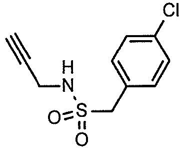

Compound **147** was synthesized according to the general procedure C, using propargylamine (94 µL, 1.47 mmol), (4-chlorophenyl)methanesulfonyl chloride (300 mg, 1.33 mmol) and *N,N*-diisopropylethylamine (464 µL, 2.67 mmol) in CH₂Cl₂ (5 mL) overnight. The crude product was purified by flash chromatography on silica gel (cHex to cHex/EtOAc: 7:3), affording compound **147** as a light yellow solid (187 mg, 45%). LC tr = 3.88 min, MS (ESI-): m/z = 242 and 244 [M - H]⁻. ¹H NMR (500 MHz, DMSO-ds) *δ* ppm: 7.68 (t, *J* = 5.8 Hz, 1H), 7.46-7.45 (m, 2H), 7.42-7.40 (m, 2H), 4.40 (s, 2H), 3.79 (dd, J = 5.8 and 2.4 Hz, 2H), 3.34 (t, *J* = 2.4 Hz, 1H). ¹³C NMR (126 MHz, DMSO-*d₆*) *δ* ppm: 133.0, 132.7 (2C), 129.2, 128.4 (2C), 80.5, 74.8, 57.0, 31.8.

### 4-Iodo-N-prop-2-ynyl-benzenesulfonamide (93)

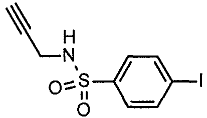

Compound **93** was synthesized according to the general procedure C, using propargylamine (70 µL, 1.09 mmol), 4-iodobenzenesulfonyl chloride (300 mg, 0.99 mmol) and *N,N*-diisopropylethylamine (345 µL, 1.98 mmol) in CH₂Cl₂ (5 mL) overnight. The crude product was purified by flash chromatography on silica gel (cHex to cHex/EtOAc: 7/3) affording compound **93** as a white solid (207 mg, 64%). LC tr = 4.04 min, MS (ESI-): m/z = 320 [M - H]⁻. ¹H NMR (500 MHz, DMSO-*d₆*) *δ* ppm: 8.23 (br s, 1H), 7.98-7.97 (m, 2H), 7.56-7.55 (m, 2H), 3.71 (s, 2H), 3.06 (t, *J* = 2.5 Hz, 1H). ¹³C NMR (126 MHz, DMSO-*d₆*) *δ* ppm: 140.2, 138.0 (2C), 128.5 (2C), 100.6, 79.2, 74.9, 31.9.

### 4-Fluoro-N-prop-2-ynyl-benzenesulfonamide (141)

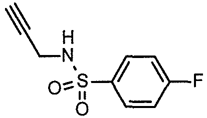

Compound **141** was synthesized according to the general procedure C, using propargylamine (217 µL, 3.39 mmol), 4-fluorobenzenesulfonyl chloride (600 mg, 3.08 mmol) and *N,N*-diisopropylethylamine (1070 µL, 6.17 mmol) in CH₂Cl₂ (10 mL) overnight. The crude product was purified by flash chromatography on silica gel (cHex to cHex/EtOAc: 7/3) affording compound **141** as a white solid (263 mg, 39%). LC tr = 3.40 min, MS (ESI+): m/z = 214 [M + H]⁺. ¹H NMR (500 MHz, DMSO-*d₆*) *δ* ppm: 8.18 (br s, 1H), 7.88-7.85 (m, 2H), 7.44-7.41 (m, 2H), 3.71 (d, *J* = 2.5 Hz, 2H), 3.03 (t, *J =* 2.5 Hz, 1H). ¹³C NMR (126 MHz, DMSO-*d₆*) *δ* ppm: 164.2 (d, *J* = 250.3 Hz), 137.0 (d, *J* = 2.8 Hz), 129.8 (d, *J = 9.2* Hz, 2C), 116.2 (d, *J* = 22.8 Hz, 2C), 79.2, 74.8, 31.9.

### 4-Chloro-N-prop-2-ynyl-benzenesulfonamide (142)

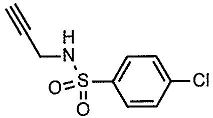

Compound **142** was synthesized according to the general procedure C, using propargylamine (100 µL, 1.56 mmol), 4-chlorobenzenesulfonyl chloride (300 mg, 1.42 mmol) and *N,N*-diisopropylethylamine (495 µL, 2.84 mmol) in CH₂Cl₂ (5 mL) overnight. The crude product was purified by flash chromatography on silica gel (cHex to cHex/EtOAc: 7/3) affording compound **142** as a white solid (283 mg, 85%). LC tr = 3.81 min, MS (ESI+): m/z = 230 [M + H]⁺. ¹H NMR (500 MHz, DMSO-*d₆*) *δ* ppm: 8.25 (br s, 1H), 7.82-7.80 (m, 2H), 7.67-7.66 (m, 2H), 3.73 (d, *J* = 2.5 Hz, 2H), 3.05 (t, *J =* 2.5 Hz, 1H). ¹³C NMR (126 MHz, DMSO-*d₆*) *δ* ppm: 139.5, 137.4, 129.2 (2C), 128.7 (2C), 79.1, 74.9, 31.9.

### 4-Methoxy-N-prop-2-ynyl-benzenesulfonamide (143)

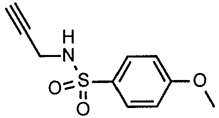

Compound **143** was synthesized according to the general procedure C, using propargylamine (205 µL, 3.19 mmol), 4-methoxybenzenesulfonyl chloride (600 mg, 2.90 mmol) and *N,N-*diisopropylethylamine (1010 µL, 5.81 mmol) in CH₂Cl₂ (10 mL) overnight. The crude product was purified by flash chromatography on silica gel (cHex to cHex/EtOAc: 5/5) affording compound **143** as a yellowish solid (528 mg, 76%). LC tr = 3.35 min, MS (ESI+): m/z = 226 [M + H]⁺. ¹H NMR (500 MHz, DMSO-*d₆*) *δ* ppm: 7.95 (br s, 1H), 7.74-7.71 (m, 2H), 7.11-7.08 (m, 2H), 3.83 (s, 3H), 3.64 (d, *J* = 2.5 Hz, 2H), 3.04 (t, *J* = 2.5 Hz, 1H). ¹³C NMR (126 MHz, DMSO-*d₆*) *δ* ppm: 162.0, 132.1, 128.9 (2C), 114.2 (2C), 79.5, 74.6, 55.6, 31.9.

### 4-Phenyl-N-prop-2-ynyl-benzenesulfonamide (95)

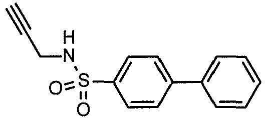

Compound **95** was synthesized according to the general procedure C, using propargylamine (83.6 µL, 1.31 mmol), 4-phenylbenzenesulfonyl chloride (300 mg, 1.19 mmol) and *N,N*-diisopropylethylamine (414 µL, 2.37 mmol) in CH₂Cl₂ (5 mL) overnight. Compound **95** was obtained as a white solid (298 mg, 91%) and used in the next step without further purifications. LC tr = 4.39 min, MS (ESI+): m/z = 272 [M + H]⁺. ¹H NMR (500 MHz, DMSO-*d₆*) *δ* ppm: 8.20 (t, *J* = 5.7 Hz, 1H), 7.89 (s, 4H), 7.74 (d, *J* = 7.5 Hz, 2H), 7.51 (t, *J =* 7.4 Hz, 2H), 7.44 (t, *J* = 7.2 Hz, 1H), 3.73 (dd, J = 6.3 and 2.6 Hz, 2H), 3.06 (t, *J* = 2.6 Hz, 1H). ¹³C NMR (126 MHz, DMSO-*d₆*) *δ* ppm: 144.0, 139.2, 138.6, 129.1 (2C), 128.5, 127.4 (2C), 127.3 (2C), 127.1 (2C), 79.4, 74.8, 32.0.

### 2-Chloro-N-prop-2-ynyl-benzenesulfonamide (144)

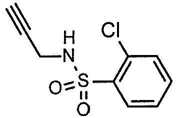

Compound **144** was synthesized according to the general procedure C, using propargylamine (167 µL, 2.21 mmol), 2-chlorobenzenesulfonyl chloride (500 mg, 2.37 mmol) and *N,N-*diisopropylethylamine (825 µL, 4.74 mmol) in CH₂Cl₂ (10 mL) overnight. The crude product was purified by flash chromatography on silica gel (cHex to cHex/EtOAc: 7/3) affording compound **144** as a white solid (478 mg, 87%). LC tr = 3.49 min, MS (ESI+): m/z = 230 [M + H]⁺. ¹H NMR (500 MHz, DMSO-*d₆*) *δ* ppm: 8.33 (br s, 1H), 7.99-7.97 (m, 1H), 7.66-7.62 (m, 2H), 7.54-7.51 (m, 1H), 3.78 (d, *J =* 2.5 Hz, 2H), 2.96 (t, *J* = 2.5 Hz, 1H). ¹³C NMR (126 MHz, DMSO-*d₆*) *δ* ppm: 138.1, 134.1, 131.6, 130.9, 130.5, 127.5, 79.1, 74.3, 31.8.

### 3-Chloro-N-prop-2-ynyl-benzenesulfonamide (145)

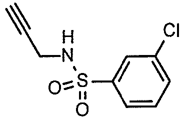

Compound **145** was synthesized according to the general procedure C, using propargylamine (167 µL, 2.21 mmol), 3-chlorobenzenesulfonyl chloride (500 mg, 2.37 mmol) and *N,N*-diisopropylethylamine (825 µL, 4.74 mmol) in CH₂Cl₂ (10 mL) overnight. The crude product was purified by flash chromatography on silica gel (cHex to cHex/EtOAc: 7/3) affording compound **145** as a white solid (300 mg, 54%). LC tr = 3.81 min, MS (ESI-): m/z = 228 [M - H]⁻. ¹H NMR (500 MHz, DMSO-*d₆*) *δ* ppm: 8.30 (br s, 1H), 7.83-7.82 (m, 1H), 7.78-7.72 (m, 2H), 7.64-7.61 (m, 1H), 3.76 (d, *J =* 2.4 Hz, 2H), 3.05 (t, *J* = 2.4 Hz, 1H). ¹³C NMR (126 MHz, DMSO-*d₆*) *δ* ppm: 142.5, 133.6, 132.5, 131.2, 126.4, 125.4, 79.1, 74.9, 31.9.

### 3,4-Dichloro-N-prop-2-ynyl-benzenesulfonamide (146)

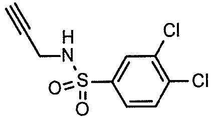

Compound **146** was synthesized according to the general procedure C, using propargylamine (172 µL, 2.69 mmol), 3,4-chlorobenzenesulfonyl chloride (600 mg, 2.44 mmol) and *N,N*-diisopropylethylamine (851 µL, 4.89 mmol) in CH₂Cl₂ (10 mL) overnight. The crude product was purified by flash chromatography on silica gel (cHex to cHex/EtOAc: 7/3) affording compound **146** as a white solid (436 mg, 66%). LC tr = 4.22 min, MS (ESI-): m/z = 262, 264 and 266 [M - H]⁻. ¹H NMR (500 MHz, DMSO-*d₆*) *δ* ppm: 8.38 (br s, 1H), 8.00-7.99 (m, 1H), 7.89-7.88 (m, 1H), 7.77-7.76 (m, 1H), 3.78 (d, *J* = 2.3 Hz, 2H), 3.09 (t, *J* = 2.3 Hz, 1H). ¹³C NMR (126 MHz, DMSO-*d₆*) *δ* ppm: 141.0, 135.6, 131.9, 131.5, 128.6, 127.0, 79.0, 75.1, 31.9.

### N-Prop-2-ynylbenzenesulfonamide (148)

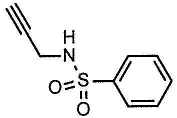

Compound **148** was synthesized according to the general procedure C, using propargylamine (239 µL, 3.74 mmol), benzenesulfonyl chloride (600 mg, 3.4 mmol) and *N,N*-diisopropylethylamine (1180 µL, 6.79 mmol) in CH₂Cl₂ (10 mL) overnight. The crude product was purified by flash chromatography on silica gel (cHex to cHex/EtOAc: 7/3) affording compound **148** as light yellow oil (527 mg, 78%). LC tr = 3.17 min, MS (ESI+): m/z = 196 [M + H]⁺. ¹H NMR (500 MHz, DMSO-*d₆*) *δ* ppm: 8.16 (br s, 1H), 7.82-7.80 (m, 2H), 7.66-7.63 (m, 1H), 7.60-7.57 (m, 2H), 3.69 (d, *J* = 2.5 Hz, 2H), 3.04 (t, *J* = 2.5 Hz, 1H). ¹³C NMR (126 MHz, DMSO-*d₆*) *δ* ppm: 140.5, 132.6, 129.1 (2C), 126.7 (2C), 79.3, 74.6, 31.9.

### N-Prop-2-ynylcyclohexanesulfonamide (149)

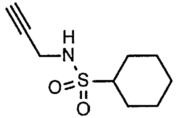

Compound **149** was synthesized according to the general procedure C, using propargylamine (193 µL, 3.01 mmol), cyclohexanesulfonyl chloride (500 mg, 2.74 mmol) and *N,N-*diisopropylethylamine (954 µL, 5.47 mmol) in CH₂Cl₂ (10 mL) overnight. The crude product was purified by flash chromatography on silica gel (cHex to cHex/EtOAc: 4/6) affording compound **149** as yellow oil (116 mg, 20%). LC tr = not visible (254 nm), MS (ESI+): no ionization [M + H]⁺ ; MS (ESI-): no ionization [M - H]⁻ . ¹H NMR (500 MHz, DMSO-*d₆*) *δ* ppm: 7.57 (t, *J* = 6.0 Hz, 1H), 3.77 (dd, *J* = 6.0 and 2.5 Hz, 2H), 3.29 (t, *J* = 2.5 Hz, 1H), 2.95 (tt, *J* = 17.6 and 3.4 Hz, 1H), 2.07-2.05 (m, 2H), 1.79 (dt, *J* = 12.7 and 2.9 Hz, 2H), 1.63 (dt, *J* = 12.7 and 3.4 Hz, 1H), 1.38-1.08 (m, 5H). ¹³C NMR (126 MHZ, DMSO-*d₆*) *δ* ppm: 80.6, 74.3, 59.7, 31.6, 25.9 (2C), 24.8, 24.6 (2C).

### 2-Methyl-N-prop-2-ynyl-propane-1-sulfonamide (106)

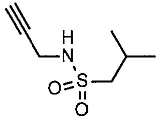

Compound **106** was synthesized according to the general procedure C, using propargylamine (135 µL, 2.11 mmol), 2-methylpropane-1-sulfonyl chloride (300 mg, 1.92 mmol) and *N,N*-diisopropylethylamine (667 µL, 3.83 mmol) in CH₂Cl₂ (10 mL) overnight. The crude product was purified by flash chromatography on silica gel (cHex to cHex/EtOAc: 4/6) affording compound **106** as yellow oil (271 mg, 79%). LC tr = not visible (254 nm), MS (ESI+): no ionization [M + H]⁺ ; MS (ESI-): no ionization [M - H]⁻. ¹H NMR (500 MHz, DMSO-*d₆*) *δ* ppm: 7.58 (t, *J* = 5.9 Hz, 1H), 3.78 (dd, *J* = 5.9 and 2.5 Hz, 2H), 3.30 (t, *J* = 2.5 Hz, 1H), 2.97 (d, *J* = 6.4 Hz, 2H), 2.12 (sep, *J* = 6.8 Hz, 1H), 1.03-1.01 (m, 6H). ¹³C NMR (126 MHz, DMSO-*d₆*) *δ* ppm: 80.5, 74.6, 59.6, 31.5, 24.2, 22.2 (2C).

### 1-(7,7-Dimethyl-2-oxo-norbornan-1-yl)-N-prop-2-ynyl-methanesulfonamide (94)

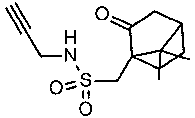

Compound **94** was synthesized according to the general procedure C, using propargylamine (84.3 µL, 1.32 mmol), (7,7-dimethyl-2-oxo-norbornan-1-yl)methanesulfonyl chloride (300 mg, 1.2 mmol) and *N,N*-diisopropylethylamine (417 µL, 2.39 mmol) in CH₂Cl₂ (5 mL) overnight. Compound **94** was obtained as a white solid (210 mg, 64%) and used in the next step without further purifications. LC tr = 3.59 min, MS (ESI+): m/z = 270 [M + H]⁺. ¹H NMR (500 MHz, DMSO-*d₆*) *δ* ppm: 7.57 (t, *J = 6.0* Hz, 1H), 3.84 (dd, *J* = 6.0 and 2.5 Hz, 2H), 3.42 (d, *J* = 14.9 Hz, 1H), 3.04 (d, *J* = 14.9 Hz, 1H), 2.37-2.32 (m, 2H), 2.05 (t, *J* = 4.5 Hz, 1H), 1.99-1.90 (m, 2H), 1.54-1.48 (m, 1H), 1.42-1.37 (m, 1H), 1.03 (s, 3H), 0.80 (s, 3H). ¹³C NMR (126 MHz, DMSO-*d₆*) *δ* ppm: 215.0, 80.5, 75.0, 58.0, 49.2, 47.6, 42.1, 42.0, 31.9, 26.3, 24.6, 19.6, 19.4.

### 4-Iodo-N-methyl-N-prop-2-ynyl-benzenesulfonamide (115)

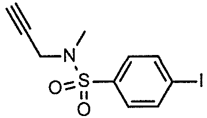

Compound **115** was synthesized according to the general procedure C, using *N-*methylpropargylamine (92 µL, 1.09 mmol), 4-iodobenzenesulfonyl chloride (300 mg, 0.99 mmol) and *N,N*-diisopropylethylamine (345 µL, 1.98 mmol) in CH₂Cl₂ (5 mL) overnight. Compound **94** was obtained as a white solid (327 mg, 97%) and used in the next step without further purifications. LC tr = 4.55 min, MS (ESI+): m/z = 336 [M + H]⁺. ¹H NMR (500 MHz, DMSO-*d₆*) *δ* ppm: 8.01-7.99 (m, 2H), 7.57-7.54 (m, 2H), 4.03 (d, *J* = 2.4 Hz, 2H), 3.16 (t, *J* = 2.4 Hz, 1H), 2.73 (s, 3H). ¹³C NMR (126 MHz, DMSO-*d₆*) *δ* ppm: 138.1 (2C), 136.3, 129.2 (2C), 101.6, 76.8, 76.6, 39.3, 34.1.

### 4-Iodo-N-prop-2-ynyl-benzamide (116)

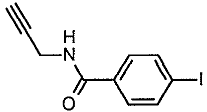

Propargylamine (79.3 µL, 1.24 mmol) and triethylamine (308 µL, 2.25 mmol) were solubilized in CH₂Cl₂ (5 mL). The reaction media was cooled at 0 °C, and 4-iodobenzoyl chloride (300 mg, 1.13 mmol) was added. The mixure was allowed to reach room temperature and was stirred overnight. Then, it was diluted in water and washed twice with 0.25 M aq. HCl, with sat. aq. NaHCO₃ and brine. The organic layer was dried over MgSO₄, filtered and concentrated under reduced pressure affording compound **116** as an orange powder (159 mg, 49%). LC tr = 3.71 min, MS (ESI+): m/z= 286 [M + H]⁺. ¹H NMR (500 MHz, DMSO-*d₆*) *δ* ppm: 9.01 (t, *J* = 5.4 Hz, 1H), 7.87-7.85 (m, 2H), 7.64-7.62 (m, 2H), 4.04 (dd, *J* = 5.4 and 2.4 Hz, 2H), 3.12 (t, *J =* 2.4 Hz, 1H). ¹³C NMR (126 MHz, DMSO-*d₆*) *δ* ppm: 165.3, 137.3 (2C), 133.2, 129.3 (2C), 99.2, 81.1, 73.0, 28.5.

### 4-Iodo-N-prop-2-ynyl-aniline (118)

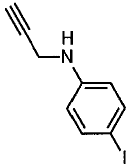

To a solution of 4-iodoaniline (300 mg, 1.37 mmol) in DMF (3 mL) were added K₂CO₃ (379 mg, 2.74 mmol) and propargylbromide (143 µL, 1.51 mmol). The resulting solution was stirred at room temperature for 48h. After 48h, the conversion was not complete and propargylbromide (195 µL, 2.05 mmol) was added and the mixture was stirred overnight at 60°C. The conversion was not significantly increased and the reaction was therefore stopped at 50% of conversion (LC-MS monitoring). The mixture was then diluted in water and extracted three times with ethyl acetate. Combined organic layers were dried over MgSO4, filtered and concentrated under reduced pressure. Finally, the residue was purified through two successive flash chromatographies on silica gel (cHex to cHex/EtOAc: 6/4) and (cHex/CH₂Cl₂ to cHex/CH₂Cl₂: 7/3 to 5/5) affording compound **118** as brown oil (100 mg, 28%). LC tr = 3.61 min, MS (ESI+): m/z= 258 [M + H]⁺. ¹H NMR (500 MHz, DMSO-*d₆*) δ: 7.39-7.37 (m, 2H), 6.49-6.48 (m, 2H), 6.22 (t, *J* = 6.1 Hz, 1H), 3.83 (dd, *J* = 6.1 and 2.4 Hz, 2H), 3.08 (t, J = 2.4 Hz, 1H). ¹³C NMR (126 MHz, DMSO-*d₆*) δ: 147.5, 137.1 (2C), 115.5 (2C), 81.8, 77.4, 73.2, 31.9.

### 1-Chloro-4-prop-2-ynylsulfanyl-benzene (120)

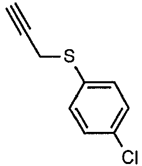

To a solution of 4-chlorobenzenethiol (300 mg, 2.07 mmol) in DMF (3 mL) were added K₂CO₃ (573 mg, 4.15 mmol) and propargylbromide (216 µL, 2.28 mmol). The resulting solution was heated at 60°C and stirred overnight. The mixture was then diluted in water and extracted three times with ethyl acetate. Combined organic layers were dried over MgSO₄, filtered and concentrated under reduced pressure affording the desired product as a brown solid (352 mg, 92%). LC tr = 4.81 min, MS (ESI+): m/z = no ionisation [M + H]⁺, (ESI-): m/z = no ionisation [M - H]⁻. ¹H NMR (500 MHz, DMSO-*d₆*) *δ* ppm: 7.41 (s, 4H), 3.87 (d, *J* = 2.6 Hz, 2H), 3.16 (t, *J* = 2.6 Hz, 1H). ¹³C NMR (126 MHz, DMSO-*d₆*) *δ* ppm: 134.0, 131.0, 130.2 (2C), 128.9 (2C), 80.1, 74.0, 20.6.

### 1-Chloro-4-prop-2-ynoxy-benzene (119)

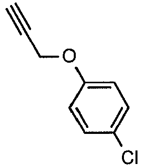

To a solution of 4-chlorophenol (300 mg, 2.33 mmol) in DMF (3 mL) were added K₂CO₃ (645 mg, 4.67 mmol) and propargylbromide (243 µL, 2.57 mmol). The resulting solution was heated at 60°C and stirred overnight. The mixture was then diluted in water and extracted three times with ethyl acetate. Organic layers were combined and solvents were evaporated under reduced pressure and the residue was purified through flash chromatography on silica gel (cHex to cHex/EtOAc: 8/2) affording compound **119** as a brown solid (376 mg, 96%). LC tr = 4.55 min, MS (ESI+): m/z = no ionisation [M + H]⁺, (ESI-): m/z = no ionisation [M - H]⁻. ¹H NMR (500 MHz, DMSO-*d₆*) *δ* ppm: 7.37-7.34 (m, 2H), 7.03-6.99 (m, 2H), 4.80 (d, *J* = 2.4 Hz, 2H), 3.59 (t, *J* = 2.3 Hz, 1H). ¹³C NMR (126 MHz, DMSO-*d₆*) *δ* ppm: 156.0, 129.2 (2C), 125.0, 116.7 (2C), 78.9, 78.5, 55.7.

### N-(2-Prop-2-ynoxyphenyl)acetamide (100)

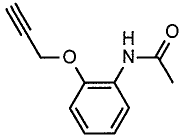

To a solution of N-(2-hydroxyphenyl) acetamide (150 mg, 0.99 mmol) in DMF (5 mL) were added K₂CO₃ (274 mg, 1.98 mmol) and propargylbromide (103 µL, 1.09 mmol). The resulting solution was heated at 60°C and stirred overnight. The mixture was then diluted in water and extracted three times with ethyl acetate. Organic layers were combined and solvents were evaporated under reduced pressure affording compound **100** as a brown solid (182 mg, 96%). LC tr = 3.03 min, MS (ESI+): m/z= 190 [M + H]⁺. ¹H NMR (500 MHz, DMSO-*d₆*) *δ* ppm: 9.13 (s, 1H), 7.91 (d, *J* = 7.5 Hz, 1H), 7.12-7.11 (m, 1H), 7.06 (t, *J* = 7.5 Hz, 1H), 6.93 (t, *J* = 7.5 Hz, 1H), 4.86 (d, *J* = 2.0 Hz, 2H), 3.60 (t, *J = 2.2* Hz, 1H), 2.08 (s, 3H). ¹³C NMR (126 MHz, DMSO-*d₆*) *δ* ppm: 168.5, 147.6, 127.9, 124.1, 122.6, 121.0, 112.9, 79.2, 78.6, 56.0, 23.8.

### 1,1-Dioxo-2-prop-2-ynyl-1,2-benzothiazol-3-one (117)

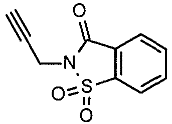

Saccharin (500 mg, 2.73 mmol) was dissolved in dry DMF (3 mL). Sodium carbonate (289 mg, 2.73 mmol) was added portion wise followed by propargyl bromide (441 µL, 80 wt% in toluene, 4.09 mmol). The reaction media was stirred overnight at 80°C. The mixture was then diluted in ethyl acetate and washed with brine. The organic layer was dried over MgSO₄, filtered and concentrated under reduced pressure affording compound **117** as a brown solid (548 mg, 89%). LC tr = 3.47 min, MS (ESI+): m/z = 222 [M + H]⁺. ¹H NMR (500 MHz, DMSO-*d₆*) *δ* ppm: 8.34 (dt, J = 7.7 and 0.8 Hz, 1H), 8.14 (dt, J = 7.7 and 0.8 Hz, 1H), 8.07 (td, *J =* 11.4 and 1.1 Hz, 1H), 8.01 (td, J *=* 11.4 and 0.9 Hz, 1H), 4.58 (d, *J* = 2.5 Hz, 2H), 3.40 (t, *J* = 2.5 Hz, 1H). ¹³C NMR (126 MHz, DMSO-*d₆*) *δ* ppm: 157.9, 136.9, 136.1, 135.4, 126.0, 125.3, 121.7, 77.0, 75.2, 27.5.

### 1-Chloro-4-prop-2-ynyl-benzene (121)

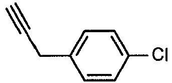

2-(4-chlorophenyl)acetaldehyde (500 mg, 3.23 mmol) and potassium carbonate (894 mg, 6.47 mmol) were mixed in anhydrous methanol (7 mL), followed by a dropwise addition of the Ohira-Bestmann reagent (647 µL, 4.31 mmol), and the reaction was stirred overnight at room temperature overnight. The mixture was then diluted in CH₂Cl₂ and washed with 5% aq. NaHCOs.The organic layer was concentrated under reduced pressure and the residue was purified through flash silica gel column (cH to cH/EtOAc 9:1), affording compound **121** as yellow oil (169 mg, 32%). LC tr = 5.14 min, MS (ESI+): m/z = no ionization [M + H]⁺; MS (ESI-): m/z = no ionization [M - H]⁻. ¹H NMR (500 MHz, DMSO-*d₆*) *δ* ppm: 7.30 (s, 4H), 3.59 (d, *J =* 2.7 Hz, 2H), 2.21 (t, *J* = 2.7 Hz, 1H). ¹³C NMR (126 MHz, DMSO-*d₆*) *δ* ppm: 134.7, 132.7, 129.3 (2C), 128.8 (2C), 81.5, 71.0, 24.4.

### 1-Ethynyl-4-iodo-benzene (122)

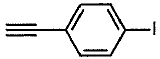

4-iodobenzaldehyde (600 mg, 2.59 mmol, 1 eq) and potassium carbonate (715 mg, 5.17 mmol, 2 eq) were mixed in anhydrous methanol (7.0 mL) followed by a dropwise addition of Ohira-Bestmann reagent (466 µL, 3.10 mmol, 1.2 eq). The resulting mixture was stirred overnight at room temperature. The mixture was then diluted with CH₂Cl₂ and washed with 5% aq. NaHCO₃. The organic layer was dried over MgSO₄, filtered and concentrated under reduced pressure. Finally, the residue was purified through flash silica gel column (cH to cH/EtOAc: 9:1) affording the desired product as a white solid (256 mg, 43%). LC tr = 4.96 min, MS (ESI+): m/z= no ionization [M + H]⁺ ; MS (ESI-): m/z= no ionization [M - H]⁻. ¹H NMR (500 MHz, CDCl₃) *δ* ppm: 7.68-7.66 (m, 2H), 7.22-7.20 (m, 2H), 3.13 (s, 1H). ¹³C NMR (126 MHz, CDCl₃) *δ* ppm: 137.6 (2C), 133.6 (2C), 121.6, 94.9, 82.7, 78.6.

### N-[2-(4-Hydroxyphenyl)ethyl]pent-4-ynamide (109)

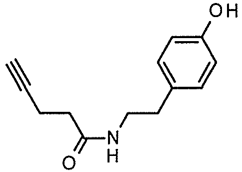

Pentynoic acid (100 mg, 1.02 mmol), tyramine (210.0 mg, 1.53 mmol, 1.5 eq) and HBTU (464 mg, 1.22 mmol, 1.2 eq) were put in solution in DMF (5.0 mL) and triethylamine (424 µL, 3.06 mmol, 3 eq), and the mixture was stirred overnight at room temperature. The resulting mixture was then diluted with water and extracted with EtOAc (three times). The organic layers were combined and solvents were evaporated in vacuo. The residue was then purified through flash silica gel column (cH/EtOAc: 9:1 to 5:5), affording compound **109** as a colorless oil (107 mg, 47%). LC tr = 2.57 min, MS (ESI+): m/z= 218 [M + H]⁺. ¹H NMR (500 MHz, DMSO-*d₆*) *δ* ppm: 9.19 (s, 1H), 7.95 (t, *J* = 5.5 Hz, 1H), 6.99-6.97 (m, 2H), 6.68-6.65 (m, 2H), 3.20-3.16 (m, 2H), 2.78 (t, *J* = 2.6 Hz, 1H), 2.58-2.55 (m, 2H), 2.35-2.31 (m, 2H), 2.25-2.22 (m, 2H). ¹³C NMR (126 MHz, DMSO-*d₆*) *δ* ppm: 170.1, 155.7, 129.6 (2C), 129.5, 115.1 (2C), 83.9, 71.4, 40.7, 34.4, 34.2, 14.3.

### 2-Ethoxycarbonyl-4-methyl-pentanoic acid (80)

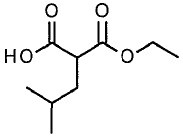

Compound **80** was synthesized according to the general procedure F, using diethylmalonate (1000 mg, 4.62 mmol) and NaOH (222 mg, 5.55 mmol) in EtOH/H₂O (10 mL, 8:2 v/v) overnight. Compound **80** was obtained as colorless oil (711 mg, 80%) and was used in the next step without further purification. LC tr = 2.10 min, MS (ESI+): m/z = 189 [M + H]⁺. ¹H NMR (MeOD-*d₄*) *δ* ppm: 4.18 (q, *J* = 7.1 Hz, 2H), 3.41 (t, *J =* 7.6 Hz, 1H), 1.77-1.72 (m, 2H), 1.64-1.50 (m, 1H), 1.26 (t, *J* = 7.1 Hz, 3H), 0.93 (d, *J* = 6.5 Hz, 6H). ¹³C NMR (MeOD-*d₄*) *δ* ppm: 173.0, 171.6, 62.3, 51.4, 38.8, 27.4, 22.7, 22.5, 14.4.

### 2-Methoxycarbonyl-3-methyl-butanoic acid (174-a)

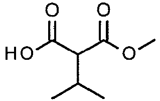

Compound **174-a** was synthesized according to the general procedure F, using dimethylmalonate (745 mg, 4.28 mmol) and KOH (240 mg, 4.28 mmol) in MeOH/H₂O (11 mL, 10:1 v/v) overnight. Compound **174-a** was obtained as colorless oil (711 mg, 80%) and was used in the next step without further purification. LC tr = 2.71 min, MS (ESI+): m/z= 161 [M + H]⁺. ¹H NMR (500 MHz, DMSO-*d₆*) *δ* ppm: 12.85 (s, 1H), 3.63 (s, 3H), 3.11 (d, *J* = 8.6 Hz, 1H), 2.23-2.14 (m, 1H), 0.93 (d, *J* = 6.7 Hz, 3H), 0.91 (d, *J* = 6.7 Hz, 3H). ¹³C NMR (126 MHz, DMSO-*d₆*) *δ* ppm: 169.7, 169.2, 58.2, 51.9, 28.0, 20.1, 20.0.

### 2-(Cyclopropylmethyl)-3-ethoxy-3-oxo-propanoic acid (175-a)

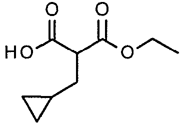

Compound **175-a** was synthesized according to the general procedure F, using dimethylmalonate (550 mg, 2.57 mmol) and KOH (144 mg, 2.57 mmol) in EtOH/H₂O (9 mL, 8:1 v/v) overnight. Compound **175-a** was obtained as colorless oil (347 mg, 71%) and was used in the next step without further purification. LC tr = not visible, MS (ESI+): m/z= 187 [M + H]⁺. ¹H NMR (500 MHz, DMSO-*d₆*) *δ* ppm: 12.80 (br s, 1H), 4.11 (q, *J* = 7.1 Hz, 2H), 3.40-3.35 (m, 1H), 1.69-1.60 (m, 2H), 1.18 (t, *J* = 7.1 Hz, 3H), 0.71-0.63 (m, 1H), 0.39-0.37 (m, 2H), 0.09-0.03 (m, 2H). ¹³C NMR (126 MHz, DMSO-*d₆*) *δ* ppm: 170.4, 169.5, 60.6, 51.9, 33.2, 14.0, 8.7, 4.4, 4.2.

### 2-Benzyl-3-ethoxy-3-oxo-propanoic acid (173-a)

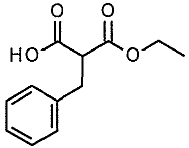

Compound **173-a** was synthesized according to the general procedure F, using dimethylmalonate (1070 mg, 4.28 mmol) and KOH (240 mg, 4.28 mmol) in EtOH/H₂O (11 mL, 10:1 v/v) overnight. Compound **173-a** was obtained as colorless oil (694 mg, 72%) and was used in the next step without further purification. LC tr = 3.72 min, MS (ESI+): m/z= no ionization [M + H]⁺. ¹H NMR (500 MHz, CDCl₃) *δ* ppm: 10.23 (br s, 1H), 7.36-7.31 (m, 2H), 7.30-7.26 (m, 3H), 4.22 (q, *J* = 7.1 Hz, 2H), 3.76 (t, *J* = 7.7 Hz, 1H), 3.30 (dd, *J* = 7.7 and 2.8 Hz, 2H), 1.26 (t, *J* = 7.1 Hz, 3H). ¹³C NMR (126 MHz, CDCl₃) *δ* ppm: 174.3, 168.9, 137.4, 128.9 (2C), 128.7 (2C), 127.1, 62.0, 53.6, 34.9, 14.1.

### Ethyl 2-[[4-[(tert-butoxycarbonylamino)methyl]phenyl]carbamoyl]-4-methyl-pentanoate (81)

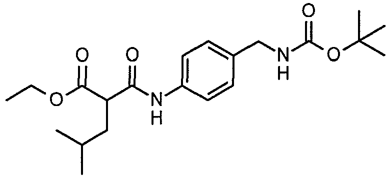

Compound **81** was synthesized according to the general procedure B, using carboxylic acid **80** (450 mg, 2.39 mmol), *tert-*butyl *N*-[(4-aminophenyl)methyl]carbamate (638 mg, 2.87 mmol), HBTU (1540 mg, 4.06 mmol) and trimethylamine (981 µL, 7.17 mmol) in DMF (8 mL) overnight. The crude product was purified by flash chromatography on silica gel (cHex/EtOAc: 9/1 to 7/3) affording compound **81** as an off-white solid (456 mg, 47%). LC tr = 3.03 min, MS (ESI-): m/z= 391 [M - H]⁻. ¹H NMR (DMSO-*d₆*) *δ* ppm: 10.18 (s, 1H), 7.52-7.49 (m, 2H), 7.33 (t, *J* = 6.0 Hz, 1H), 7.18-7.15 (m, 2H), 4.15-4.02 (m, 4H), 3.56 (dd, *J* = 8.6 Hz and 6.3 Hz, 1H), 1.80-1.60 (m, 2H), 1.57-1.45 (m, 1H), 1.39 (s, 9H), 1.16 (t, *J* = 7.1 Hz, 3H), 0.90 (d, *J* = 6.6 Hz, 3H), 0.87 (d, *J = 6.6* Hz, 3H). ¹³C NMR (DMSO-*d₆*) *δ* ppm: 169.8, 166.9, 155.7, 137.4, 135.4, 127.4 (2C), 119.2 (2C), 77.7, 60.6, 50.9, 43.0, 37.4, 28.2 (3C), 25.6, 22.6, 21.9, 14.0.

### Ethyl 2-[[3-[(tert-butoxycarbonylamino)methyl]phenyl]carbamoyl]-4-methyl-pentanoate (110)

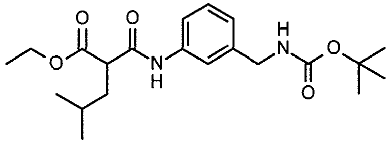

Compound **110** was synthesized according to the general procedure B, using carboxylic acid **80** (320 mg, 1.7 mmol), *tert-*butyl *N*-[(3-aminophenyl)methyl]carbamate (416 mg, 1.87 mmol), HBTU (967 mg, 2.55 mmol) and trimethylamine (697 µL, 5.10 mmol) in DMF (5 mL) overnight. The crude product was purified by flash chromatography on silica gel (cHex/EtOAc: 9/1 to 7/3) affording compound **110** as a yellowish solid (507 mg, 74%). LC tr = 4.77 min, MS (ESI+): m/z= 393 [M + H]⁺, 337 [M - tBu]⁺. ¹H NMR (500 MHz, DMSO-*d₆*) *δ* ppm: 10.24 (s, 1H), 7.47-7.45 (m, 2H), 7.42 (t, *J* = 6.1 Hz, 1H), 7.24 (t, *J* = 8.0 Hz, 1H), 6.93 (d, *J* = 7.6 Hz, 1H), 4.13-4.04 (m, 4H), 3.57 (dd, *J* = 8.9 and 6.0 Hz, 1H), 1.78-1.72 (m, 1H), 1.66-1.60 (m, 1H), 1.48 (sep, *J* = 6.7 Hz, 1H), 1.39 (s, 9H), 1.18-1.14 (m, 3H), 0.89 (d, *J* = 6.6 Hz, 3H), 0.87 (d, *J* = 6.6 Hz, 3H). ¹³C NMR (126 MHz, DMSO-*d₆*) *δ* ppm: 169.9, 167.1, 155.8, 141.0, 138.8, 128.7, 122.3, 117.8, 117.7, 77.9, 60.7, 50.9, 43.4, 37.5, 28.3 (3C), 25.7, 22.8, 21.9, 14.1.

### [4-[(2-Ethoxycarbonyl-4-methyl-pentanoyl)amino]phenyl]methylammonium chloride (82)

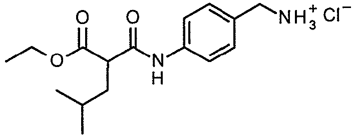

Compound **82** was synthesized according to the general procedure G, using the Boc-protected intermediate **81** (440 mg, 1.12 mmol), 4 N HCl in dioxane (5 mL) in a mixture CH₂Cl₂/EtOH (10 mL, 5:5 v/v) overnight. Compound **82** was obtained as yellow oil (369 mg, quant. yield) and was used in the next step without further purification. LC tr = 2.15 min, MS (ESI-): m/z = 291 [M - H]⁻. ¹H NMR (DMSO-*d₆*) *δ* ppm: 10.55 (s, 1H), 8.44 (br s, 3H), 7.65-7.60 (m, 2H), 7.44-7.41 (m, 2H), 4.13-4.04 (m, 2H), 3.96-3.91 (m, 2H), 3.68-3.63 (m, 1H), 1.79-1.61 (m, 2H), 1.50 (sep, J = 6.7 Hz, 1H), 1.15 (t, *J =* 7.1 Hz, 3H), 0.89 (d, *J* = 6.7 Hz, 3H), 0.87 (d, *J* = 6.7 Hz, 3H). ¹³C NMR (DMSO-*d₆*) *δ* ppm: 169.8, 167.4, 139.1, 129.6 (2C), 129.0, 119.2 (2C), 60.6, 50.9, 41.8, 37.5, 25.7, 22.7, 22.1, 14.1.

### [3-[(2-Ethoxycarbonyl-4-methyl-pentanoyl)amino]phenyl]methylammonium chloride (111)

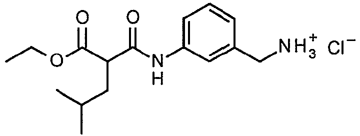

Compound **111** was synthesized according to the general procedure G, using the Boc-protected intermediate **110** (495 mg, 1.26 mmol), 4 N HCl in dioxane (7 mL) in a mixture CH₂Cl₂/EtOH (14 mL, 7:7 v/v) overnight. Compound **111** was obtained as yellow oil (415 mg, quant. yield) and was used in the next step without further purification. LC tr = 2.82 min, MS (ESI+): m/z = 293 [M + H]⁺, 585 [2M + H]⁺. ¹H NMR (500 MHz, DMSO-*d₆*) *δ* ppm: 10.54 (s, 1H), 8.39 (br s, 3H), 7.79-7.78 (m, 1H), 7.54-7.53 (m, 1H), 7.36 (t, *J* = 7.9 Hz, 1H), 7.21-7.20 (m, 1H), 4.13-4.05 (m, 2H), 3.72-3.65 (m, 2H), 3.51-3.46 (m, 1H), 1.79-1.73 (m, 1H), 1.67-1.61 (m, 1H), 1.49 (sep, *J* = 6.6 Hz, 1H), 1.16 (t, *J* = 7.1 Hz, 3H), 0.90 (d, *J* = 6.6 Hz, 3H), 0.88 (d, *J =* 6.6 Hz, 3H). ¹³C NMR (126 MHz, DMSO-*d₆*) *δ* ppm: 169.9, 167.4, 139.2, 134.7, 129.2, 124.2, 119.9, 119.4, 60.7, 53.6, 50.9, 37.5, 25.7, 22.9, 22.0, 14.1.

### Ethyl 2-[[4-(azidomethyl)phenyl]carbamoyl]-4-methyl-pentanoate (83)

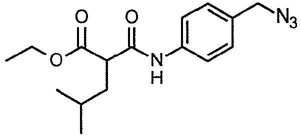

Compound **83** was synthesized according to the general procedure A, using amine **82** (350 mg, 0.93 mmol), ZnCl₂ (7.6 mg, 0.05 mmol), K₂CO₃ (512 mg, 3.7 mmol), anhydrous *N,N*-diisopropylethylamine (177 µL, 1.02 mmol) and diazo transfer reagent (301 mg, 1.11 mmol) in anhydrous MeOH (5 mL) overnight. The crude product was purified by flash chromatography on silica gel (cHex to cHex/EtOAc: 8/2), affording compound **84** as a yellowish solid (176 mg, 58%). Trans esterification was also observed, leading to the formation of the methyl ester analog (87). LC tr = 3.02 min, MS (ESI-): m/z = 317 [M - H]⁻. ¹H NMR (300 MHz, CDCl₃+TMS) *δ* ppm: 8.75 (s, 1H), 7.58-7.56 (m, 2H), 7.28-7.24 (m, 2H), 4.28 (br s, 2H), 4.26-4.19 (m, 2H), 3.46 (t, *J =* 7.7 Hz, 1H), 1.90-1.85 (m, 2H), 1.64 (sep, J = 6.7 Hz, 1H), 1.30 (t, *J* = 7.1 Hz, 3H), 0.95 (d, *J* = 6.7 Hz, 6H). ¹³C NMR (75 MHz, CDCl₃+TMS) *δ* ppm: 172.8, 166.9, 137.8, 131.3, 129.0 (2C), 120.2 (2C), 61.8, 54.3, 52.4, 40.5, 26.4, 22.4, 22.1, 14.1.

### Ethyl 2-[[3-(azidomethyl)phenyl]carbamoyl]-4-methyl-pentanoate (112)

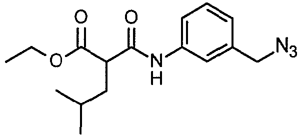

Compound **112** was synthesized according to the general procedure A, using amine **111** (405 mg, 1.07 mmol), ZnCl₂ (8.8 mg, 0.06 mmol), K₂CO₃ (296 mg, 2.14 mmol), anhydrous *N,N*-diisopropylethylamine (392 µL, 2.25 mmol) and diazo transfer reagent (270 mg, 1.29 mmol) in anhydrous MeOH (5 mL) overnight. The crude product was purified by flash chromatography on silica gel (cHex to cHex/EtOAc: 8/2) affording compound **112** as white crystals (121 mg, 31%). LC tr = 4.75 min, MS (ESI+): m/z = 319 [M + H]⁺. ¹H NMR (500 MHz, DMSO-ds) *δ* ppm: 10.31 (s, 1H), 7.65-7.64 (m, 1H), 7.55-7.53 (m, 1H), 7.34 (t, *J* = 7.8 Hz, 1H), 7.07-7.06 (m, 1H), 4.44 (s, 2H), 4.16-4.06 (m, 2H), 3.58 (dd, *J* = 8.8 and 6.2 Hz, 1H), 1.78-1.74 (m, 1H), 1.69-1.63 (m, 1H), 1.50 (sep, *J* = 6.7 Hz, 1H), 1.16 (t, *J* = 7.0 Hz, 3H), 0.90 (d, *J* = 6.7 Hz, 3H), 0.88 (d, *J =* 6.7 Hz, 3H). ¹³C NMR (126 MHz, DMSO-*d₆*) *δ* ppm: 169.7, 167.2, 139.2, 136.4, 129.2, 123.5, 119.0, 118.9, 60.7, 53.6, 51.0, 37.4, 25.6, 22.7, 21.9, 14.0.

### Tert-butyl N-[4-(azidomethyl)phenyl]carbamate (170)

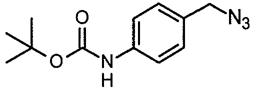

Compound **170** was synthesized according to the general procedure A, using *tert-*butyl *N*-[4-(aminomethyl)phenyl]carbamate (500 mg, 2.25 mmol), ZnCl₂ (18.4 mg, 0.14 mmol), K₂CO₃ (1240 mg, 9.0 mmol), anhydrous N,N-diisopropylethylamine (240 µL, 2.47 mmol) and diazo transfer reagent (566 mg, 2.7 mmol) in anhydrous MeOH (5 mL) overnight. Compound **170** was obtained as orange oil (558 mg, quant. yield) and was used in the next step without further purification. LC tr = 4.72 min, MS (ESI+): m/z = no ionization [M + H]⁺. ¹H NMR (500 MHz, DMSO-*d₆*) *δ* ppm: 9.42 (s, 1H), 7.48-7.45 (m, 2H), 7.26-7.24 (m, 2H), 4.33 (s, 2H), 1.47 (s, 9H). ¹³C NMR (126 MHz, DMSO-*d₆*) *δ* ppm: 152.7, 139.5, 129.1 (3C), 128.8, 118.2, 79.1, 53.3, 28.1 (3C).

### [4-(Azidomethyl)phenyl]ammonium;chloride (171)

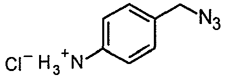

Compound **171** was synthesized according to the general procedure G, using the Boc-protected intermediate **170** (550 mg, 2.22 mmol), 4 N HCl in dioxane (10 mL) in a mixture CH₂Cl₂/EtOH (20 mL, 10:10 v/v) overnight. Compound **171** was obtained as an orange solid (409 mg, quant. yield) and was used in the next step without further purification. LC tr = 1.40 min, MS (ESI+): m/z = 149 [M + H]⁺. ¹H NMR (500 MHz, DMSO-*d₆*) *δ* ppm: 9.88 (br s, 3H), 7.45-7.43 (m, 2H), 7.33-7.32 (m, 2H), 4.46 (s, 2H).

### Methyl 2-[[4-(azidomethyl)phenyl]carbamoyl]-3-methyl-butanoate (174)

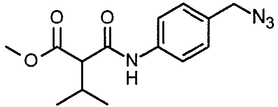

Compound **174** was synthesized according to the general procedure B, using carboxylic acid **174-a** (300 mg, 1.87 mmol), aniline **171** (415 mg, 2.27 mmol), HBTU (1070 mg, 2.81 mmol) and trimethylamine (1020 µL, 7.49 mmol) in DMF (5 mL) overnight. The crude product was purified by flash chromatography on silica gel (cHex to cHex/EtOAc: 6/4) affording compound **174** as a white solid (203 mg, 37%). LC tr = 4.18 min, MS (ESI+): m/z = 291 [M + H]⁺. ¹H NMR (500 MHz, DMSO-*d₆*) *δ* ppm: 10.28 (s, 1H), 7.62-7.60 (m, 2H), 7.33-7.31 (m, 2H), 4.38 (s, 2H), 3.64 (s, 3H), 3.24 (d, *J =* 9.7 Hz, 1H), 2.36-2.28 (m, 1H), 0.95 (d, *J* = 6.7 Hz, 3H), 0.92 (d, *J* = 6.7 Hz, 3H). ¹³C NMR (126 MHz, DMSO-*d₆*) *δ* ppm: 169.4, 166.3, 138.6, 130.6, 129.2 (2C), 119.4 (2C), 60.0, 53.2, 51.9, 28.4, 20.6, 19.8.

### Ethyl 3-[4-(azidomethyl)anilino]-2-(cyclopropylmethyl)-3-oxo-propanoate (175)

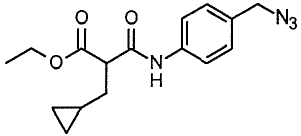

Compound **175** was synthesized according to the general procedure B, using carboxylic acid **175-a** (340 mg, 1.83 mmol), aniline **171** (405 mg, 2.19 mmol), HBTU (1040 mg, 2.74 mmol) and trimethylamine (1250 µL, 9.13 mmol) in DMF (5 mL) overnight. The crude product was purified by flash chromatography on silica gel (cHex to cHex/EtOAc: 5/5) affording compound **175** as a pale yellow solid (277 mg, 43%). LC tr = 4.49 min, MS (ESI+): m/z = 317 [M + H]⁺. ¹H NMR (500 MHz, DMSO-*d₆*) *δ* ppm: 10.30 (s, 1H), 7.62-7.61 (m, 2H), 7.33-7.31 (m, 2H), 4.38 (s, 2H), 4.12-4.09 (m, 2H), 3.59 (dd, *J* = 8.6 and 6.8 Hz, 1H), 1.81-1.63 (m, 2H), 1.17 (t, *J* = 7.1 Hz, 3H), 0.74-0.65 (m, 1H), 0.44-0.34 (m, 2H), 0.11-0.04 (m, 2H). ¹³C NMR (126 MHz, DMSO-*d₆*) *δ* ppm: 169.5, 167.2, 138.8, 130.5, 129.2 (2C), 119.4 (2C), 60.6, 53.2, 53.2, 33.5, 14.0, 8.8, 4.4, 4.3.

### Ethyl 3-[4-(azidomethyl)anilino]-2-benzyl-3-oxo-propanoate (173)

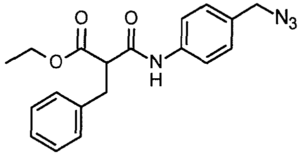

Compound **173** was synthesized according to the general procedure B, using carboxylic acid **173-a** (300 mg, 1.35 mmol), aniline **171** (299 mg, 1.62 mmol), HBTU (768 mg, 2.02 mmol) and trimethylamine (738 µL, 5.4 mmol) in DMF (3 mL) overnight. The crude product was purified by flash chromatography on silica gel (cHex to cHex/EtOAc: 7/3) affording compound **173** as a white solid (152 mg, 31%). LC tr = 4.69 min, MS (ESI+): m/z= 353 [M + H]⁺. ¹H NMR (500 MHz, DMSO-*d₆*) *δ* ppm: 10.25(s, 1H), 7.54-7.53 (m, 2H), 7.30-7.29 (m, 2H), 7.26-7.15 (m, 5H), 4.36 (s, 2H), 4.11-4.04 (m, 2H), 3.84 (dd, *J* = 8.5 and 6.8 Hz, 1H), 3.14-3.12 (m, 2H), 1.13 (t, *J =* 7.1 Hz, 3H). ¹³C NMR (126 MHz, DMSO-*d₆*) *δ* ppm: 169.0, 166.4, 138.5, 138.4, 130.6, 129.1 (2C), 128.8 (2C), 128.3 (2C), 126.4, 119.4 (2C), 60.8, 54.3, 53.2, 34.2, 14.0.

### N-[4-(Azidomethyl)phenyl]-2-(hydroxycarbamoyl)-4-methyl-pentanamide (Z9)

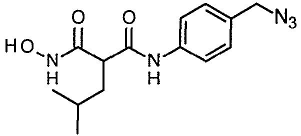

Compound **Z9** was synthesized according to the general procedure D, using ester **83** (176 mg, 0.55 mmol), KCN (7.13 mg, 0.11 mmol) and aq. NH₂OH (2.5 mL, 50% w/w in water) in MeOH (2.5 mL) overnight. The crude product was purified by flash chromatography on silica gel (CH₂Cl₂ to CH₂Cl₂/MeOH: 95/5) affording compound **Z9** as a white solid after lyophilization (65 mg, 38%).

Purity: 100%, LC tr = 2.35 min, MS (ESI-): m/z = 304 [M - H]⁻. ¹H NMR (300 MHz, DMSO-*d₆*) *δ* ppm: 10.52 (s, 1H), 9.82 (s, 1H), 8.97 (s, 1H), 7.62-7.59 (m, 2H), 7.32-7.30 (m, 2H), 4.37 (s, 2H), 3.22 (t, *J* = 7.6 Hz, 1H), 1.69 (t, *J* = 7.3 Hz, 2H), 1.49 (sep, *J = 6.7* Hz, 1H), 0.89 (d, *J* = 2.0 Hz, 3H), 0.87 (d, *J* = 2.0 Hz, 3H). ¹³C NMR (75 MHz, DMSO-*d₆*) *δ* ppm: 167.8, 166.3, 138.7, 130.4, 129.1 (2C), 119.5 (2C), 53.2, 50.0, 38.0, 25.7, 22.4, 22.3.

### N-[4-(Azidomethyl)phenyl]-2-(hydroxycarbamoyl)-3-methyl-butanamide (180)

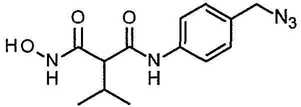

Compound **180** was synthesized according to the general procedure D, using ester **174** (100 mg, 0.34 mmol), KCN (4.44 mg, 0.07 mmol) and aq. NH₂OH (2.5 mL, 50% w/w in water) in MeOH (2.5 mL) overnight. The crude product was purified by flash chromatography on silica gel (CH₂Cl₂ to CH₂Cl₂/MeOH: 95/5) affording compound **180** as a white solid after lyophilization (43 mg, 43%). LC tr = 3.24 min, MS (ESI+): m/z = 292 [M + H]⁺. ¹H NMR (500 MHz, DMSO-*d₆*) *δ* ppm: 10.55 (s, 1H), 9.78 (s, 1H), 9.02 (s, 1H), 7.62-7.60 (m, 2H), 7.32-7.30 (m, 2H), 4.37 (s, 2H), 2.73 (d, *J* = 10.5 Hz, 1H), 2.40-2.33 (m, 1H), 0.90 (d, *J* = 6.6 Hz, 3H), 0.89 (d, J = 6.6 Hz, 3H). ¹³C NMR (126 MHz, DMSO-*d₆*) *δ* ppm: 167.3, 165.9, 138.6, 130.5, 129.1 (2C), 119.6 (2C), 59.7, 53.2, 29.7, 20.4, 20.3.

### N-[4-(Azidomethyl)phenyl]-2-(cyclopropylmethyl)-3-(hydroxyamino)-3-oxo-propanamide (181)

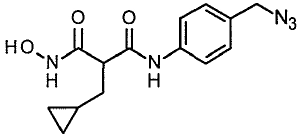

Compound **181** was synthesized according to the general procedure D, using ester **175** (135 mg, 0.42 mmol), KCN (5.5 mg, 0.08 mmol) and aq. NH₂OH (3.2 mL, 50% w/w in water) in MeOH (3.2 mL) overnight. The crude product was purified by flash chromatography on silica gel (CH₂Cl₂ to CH₂Cl₂/MeOH: 95/5) affording compound **181** as a white solid after lyophilization (58 mg, 44%). LC tr = 3.41 min, MS (ESI+): m/z = 304 [M + H]⁺. ¹H NMR (500 MHz, DMSO-*d₆*) *δ* ppm: 10.51 (br s, 1H), 9.86 (s, 1H), 8.96 (s, 1H), 7.62-7.60 (m, 2H), 7.32-7.30 (m, 2H), 4.37 (s, 2H), 3.23 (t, *J* = 7.5 Hz, 1H), 1.72-1.69 (m, 2H), 0.68-0.61 (m, 1H), 0.40-0.34 (m, 2H), 0.09-0.08 (m, 2H). ¹³C NMR (126 MHz, DMSO-*d₆*) *δ* ppm: 167.8, 166.2, 138.8, 130.3, 129.1 (2C), 119.4 (2C), 53.3, 52.0, 34.1, 9.0, 4.3, 4.3.

### Methyl 2-[[4-[[4-[[(4-iodophenyl)sulfonylamino]methyl]triazol-1-yl]methyl]phenyl]carbamoyl]-4-methyl-pentanoate (88)

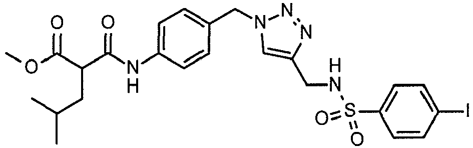

Compound **88** was synthesized according to the general procedure E, using azide **87** (100 mg, 0.33 mmol), alkyne **93** (106 mg, 0.33 mmol), copper (II) sulfate pentahydrate (16.4 mg, 0.07 mmol) and sodium ascorbate (32.5 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **88** as a white powder (119 mg, 51%). LC tr = 4.48 min, MS (ESI+): m/z = 626 [M + H]⁺. ¹H NMR (500 MHz, DMSO-*d₆*) *δ* ppm: 10.32 (s, 1H), 8.19 (t, *J* = 5.9 Hz, 1H), 7.93-7.91 (m, 2H), 7.85 (s, 1H), 7.59-7.57 (m, 2H), 7.51-7.50 (m, 2H), 7.27-7.25 (m, 2H), 5.45 (s, 2H), 4.03 (d, *J* = 5.9 Hz, 2H), 3.63 (s, 3H), 3.58 (dd, *J* = 8.9 and 6.1 Hz, 1H), 1.78-1.73 (m, 1H), 1.68-1.62 (m, 1H), 1.48 (sep, *J* = 6.6 Hz, 1H), 0.89 (d, *J* = 6.6 Hz, 3H), 0.87 (d, *J* = 6.6 Hz, 3H). ¹³C NMR (126 MHz, DMSO-*d₆*) *δ* ppm: 170.2, 167.1, 143.4, 140.0, 138.7, 138.0 (2C), 130.9, 128.8 (2C), 128.3 (2C), 123.2, 119.4 (2C), 100.4, 52.4, 52.1, 50.8, 38.0, 37.4, 25.6, 22.7, 21.9.

### Methyl 2-[[4-[[4-[[(7,7-dimethyl-2-oxo-norbornan-1-yl)methylsulfonylamino]methyl]triazol-1-yl]methyl]phenyl]carbamoyl]-4-methyl-pentanoate (89)

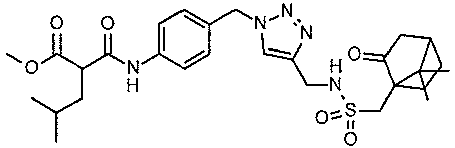

Compound **89** was synthesized according to the general procedure E, using azide **87** (100 mg, 0.33 mmol), alkyne **94** (88.5 mg, 0.33 mmol), copper (II) sulfate pentahydrate (16.4 mg, 0.07 mmol) and sodium ascorbate (32.5 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **89** as colorless oil (137 mg, 64%). LC tr= 4.29 min, MS (ESI-): m/z = 572 [M - H]⁻. ¹H NMR (500 MHz, DMSO-*d₆*) *δ* ppm: 10.31 (s, 1H), 8.01 (s, 1H), 7.58-7.56 (m, 2H), 7.29-7.28 (m, 2H), 5.52 (s, 2H), 4.26-4.24 (m, 2H), 3.62 (s, 3H), 3.58 (dd, *J* = 8.9 and 6.0 Hz, 1H), 3.21 (d, *J= 14.9* Hz, 1H), 3.17 (d, *J* = 5.2 Hz, 1H), 2.84 (d, *J* = 14.9 Hz, 1H), 2.32-2.27 (m, 2H), 2.01 (t, *J = 4.5* Hz, 1H), 1.89 (d, *J* = 18.3 Hz, 2H), 1.78-1.73 (m, 1H), 1.67-1.62 (m, 1H), 1.50-1.44 (m, 2H), 1.38-1.33 (m, 1H), 0.95 (s, 3H), 0.88 (d, *J* = 6.6 Hz, 3H), 0.87 (d, *J* = 6.6 Hz, 3H), 0.70 (s, 3H). ¹³C NMR (126 MHz, DMSO-*d₆*) *δ* ppm: 214.6, 170.2, 167.1, 144.6, 138.7, 131.1, 128.8 (2C), 123.2, 119.4 (2C), 57.9, 52.4, 52.1, 50.8, 48.7, 47.6, 42.0, 41.9, 38.0, 37.4, 26.3, 25.6, 24.5, 22.7, 21.9, 19.4, 19.2.

### Methyl 4-methyl-2-[[4-[[4-[[(4-phenylphenyl)sulfonylamino]methyl]triazol-1-yl]methyl]phenyl]carbamoyl]pentanoate (90)

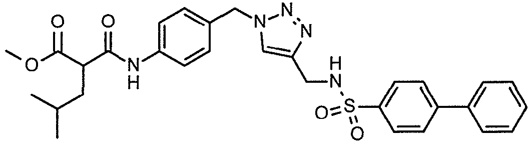

Compound **90** was synthesized according to the general procedure E, using azide **87** (100 mg, 0.33 mmol), alkyne **95** (89.2 mg, 0.33 mmol), copper (II) sulfate pentahydrate (16.4 mg, 0.07 mmol) and sodium ascorbate (32.5 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **90** as a greenwish powder (139 mg, 62%). LC tr= 4.69 min, MS (ESI-): m/z = 574 [M - H]⁻. ¹H NMR (500 MHz, DMSO-*d₆*) *δ* ppm: 10.31 (s, 1H), 8.15 (t, *J* = 5.9 Hz, 1H), 7.89 (s, 1H), 7.84 (s, 4H), 7.74-7.72 (m, 2H), 7.57-7.55 (m, 2H), 7.53-7.50 (m, 2H), 7.46-7.43 (m, 1H), 7.26-7.24 (m, 2H), 5.45 (s, 2H), 4.06 (d, *J* = 5.9 Hz, 2H), 3.62 (s, 3H), 3.58 (dd, *J =* 8.9 and 6.0 Hz, 1H), 1.78-1.72 (m, 1H), 1.68-1.62 (m, 1H), 1.48 (sep, *J* = 6.7 Hz, 1H), 0.88 (d, *J* = 6.7 Hz, 3H), 0.87 (d, *J* = 6.7 Hz, 3H). ¹³C NMR (126 MHz, DMSO-*d₆*) *δ* ppm: 170.1, 167.1, 143.9, 143.7, 139.1, 138.7, 138.5, 131.0, 129.1 (2C), 128.8 (2C), 128.5, 127.3 (2C), 127.2 (2C), 127.1 (2C), 123.2, 119.4 (2C), 52.4, 52.1, 50.8, 38.1, 37.4, 25.6, 22.6, 21.9.

### Methyl 2-[[4-[[4-[3-(4-fluorobenzoyl)thiazolidin-4-yl]triazol-1-yl]methyl]phenyl]carbamoyl]-4-methyl-pentanoate (91)

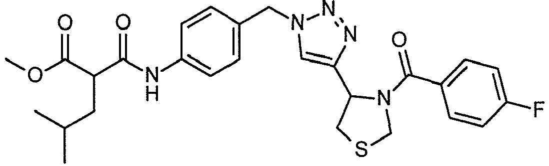

Compound **91** was synthesized according to the general procedure E, using azide **87** (80 mg, 0.25 mmol), alkyne **99** (59.1 mg, 0.25 mmol), copper (II) sulfate pentahydrate (12.5 mg, 0.05 mmol) and sodium ascorbate (24.9 mg, 0.13 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **91** as brown oil (128 mg, 76%). LC tr= 4.40 min, MS (ESI+): m/z = 540 [M + H]⁺. ¹H NMR (500 MHz, DMSO-*d₆*) *δ* ppm: 10.35 (s, 1H), 8.17 (s, 1H), 7.59-7.58 (m, 4H), 7.30-7.28 (m, 4H), 5.96-5.74 (m, 1H), 5.50 (s, 2H), 4.60 (br s, 2H), 3.62 (s, 3H), 3.47-3.42 (m, 1H), 3.29-3.19 (m, 1H), 1.78-1.72 (m, 1H), 1.67-1.61 (m, 1H), 1.51-1.43 (m, 1H), 1.16-0.97 (m, 1H), 0.88 (d, *J* = 6.6 Hz, 3H), 0.86 (d, *J* = 6.8 Hz, 3H). ¹³C NMR (126 MHz, DMSO-*d₆*) *δ* ppm: 170.2, 167.7, 167.1, 163.1 (d, *J* = 248.7 Hz), 146.7, 138.7, 131.0, 129.9 (d, *J* = 12.4 Hz), 128.7 (2C), 122.7 (2C), 120.2, 119.4 (2C), 115.4 (d, *J* = 21.8 Hz, 2C), 52.5, 52.1, 50.8, 48.6, 37.4, 35.5, 25.7, 25.6, 22.7, 21.9.

### Methyl 2-[[4-[[4-[(2-acetamidophenoxy)methyl]triazol-1-yl]methyl]phenyl]carbamoyl]-4-methyl-pentanoate (92)

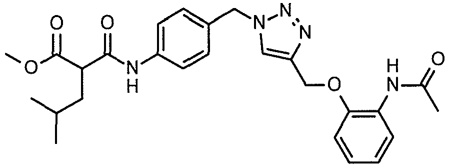

Compound **92** was synthesized according to the general procedure E, using azide **87** (100 mg, 0.33 mmol), alkyne **100** (62.2 mg, 0.33 mmol), copper (II) sulfate pentahydrate (16.4 mg, 0.07 mmol) and sodium ascorbate (32.5 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **92** as a beige powder (139 mg, 62%). LC tr = 4.05 min, MS (ESI-): m/z = 492 [M - H]⁻. ¹H NMR (500 MHz, DMSO-*d₆*) *δ* ppm: 10.32 (s, 1H), 9.01 (s, 1H), 8.22 (s, 1H), 7.87 (d, *J* = 7.7 Hz, 1H), 7.59-7.57 (m, 2H), 7.29-7.27 (m, 2H), 7.20 (d, *J* = 7.9 Hz, 1H), 7.04 (t, *J* = 7.4 Hz, 1H), 6.90 (t, *J* = 7.4 Hz, 1H), 5.55 (s, 2H), 5.20 (s, 2H), 3.62 (s, 3H), 3.58 (dd, *J* = 8.9 and 6.1 Hz, 1H), 2.03 (s, 3H), 1.78-1.73 (m, 1H), 1.68-1.62 (m, 1H), 1.48 (sep, *J* = 6.7 Hz, 1H), 0.89 (d, *J* = 6.7 Hz, 3H), 0.87 (d, *J* = 6.7 Hz, 3H). ¹³C NMR (126 MHz, DMSO-*d₆*) *δ* ppm: 170.2, 168.3, 167.1, 148.5, 143.2, 138.7, 131.0, 128.7 (2C), 127.9, 124.4, 124.2, 122.5, 120.8, 119.4 (2C), 113.3, 62.3, 52.5, 52.1, 50.8, 37.4, 25.6, 23.8, 22.7, 21.9.

### Methyl 2-[[4-[[4-[[(4-iodophenyl)sulfonyl-methyl-amino]methyl]triazol-1-yl]methyl]phenyl]carbamoyl]-4-methyl-pentanoate (123)

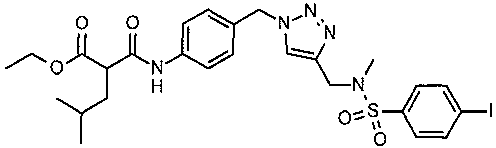

Compound **123** was synthesized according to the general procedure E, using azide **83** (100 mg, 0.31 mmol), alkyne **115** (105 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **123** as a white powder (132 mg, 59%). LC tr = 4.93 min, MS (ESI+): m/z = 654 [M + H]⁺. ¹H NMR (500 MHz, DMSO-*d₆*) *δ* ppm: 10.29 (s, 1H), 8.04 (s, 1H), 7.96-7.94 (m, 2H), 7.59-7.57 (m, 2H), 7.52-7.51 (m, 2H), 7.28-7.26 (m, 2H), 5.49 (s, 2H), 4.26 (s, 2H), 4.13-4.05 (m, 2H), 3.56 (dd, *J* = 8.7 and 6.2 Hz, 1H), 2.64 (s, 3H), 1.77-1.72 (m, 1H), 1.67-1.62 (m, 1H), 1.49 (sep, *J* = 6.7 Hz, 1H), 1.15 (t, *J* = 7.1 Hz, 3H), 0.88 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 141.8, 138.8, 138.2 (2C), 136.5, 130.9, 128.9 (2C), 128.7 (2C), 124.0, 119.5 (2C), 101.3, 60.7, 52.5, 50.9, 44.7, 37.4, 34.7, 25.7, 22.7, 21.9, 14.0.

### Ethyl 2-[[4-[[4-[[(4-iodobenzoyl)amino]methyl]triazol-1-yl]methyl]phenyl]carbamoyl]-4-methyl-pentanoate (124)

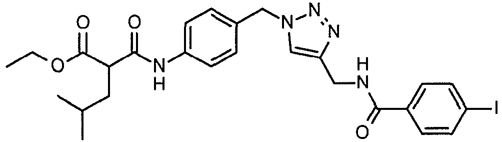

Compound **124** was synthesized according to the general procedure E, using azide **83** (100 mg, 0.31 mmol), alkyne **116** (89.5 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **124** as a white solid (141 mg, 60%). LC tr = 4.61 min, MS (ESI+): m/z = 604 [M+H]⁺. ¹H NMR (500 MHz, DMSO-*d₆*) *δ* ppm: 10.28 (s, 1H), 9.05 (t, *J* = 5.6 Hz, 1H), 7.98 (s, 1H), 7.85-7.83 (m, 2H), 7.65-7.63 (m, 2H), 7.57-7.55 (m, 2H), 7.30-7.28 (m, 2H), 5.49 (s, 2H), 4.47 (d, *J* = 5.7 Hz, 2H), 4.12-4.05 (m, 2H), 3.55 (dd, *J* = 8.7 and 6.2 Hz, 1H), 1.77-1.71 (m, 1H), 1.67-1.62 (m, 1H), 1.48 (sep, *J* = 6.7 Hz, 1H), 1.15 (t, *J* = 7.1 Hz, 3H), 0.88 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 165.4, 145.1, 138.7, 137.2 (2C), 133.5, 131.1, 129.3 (2C), 128.8 (2C), 122.9, 119.4 (2C), 98.9, 60.6, 52.4, 50.9, 37.4, 34.9, 25.6, 22.6, 21.9, 14.0.

### Ethyl 2-[[4-[[4-[(tert-butoxycarbonylamino)methyl]triazol-1-yl]methyl]phenyl]carbamoyl]-4-methyl-pentanoate (125)

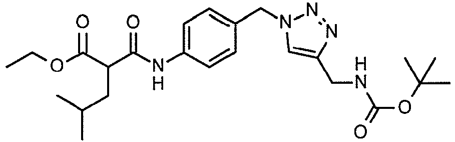

Compound **125** was synthesized according to the general procedure E, using azide **83** (100 mg, 0.31 mmol), *N*-boc-propargylamine (48.7 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **125** as a colorless solid (115 mg, 72%). LC tr = 4.35 min, MS (ESI+): m/z = 474 [M + H]⁺, 418 [M + H - tBu]⁺, 374 [M + H - boc]⁺. ¹H NMR (500 MHz, DMSO-*d₆*) *δ* ppm: 10.31 (s, 1H), 7.87 (s, 1H), 7.57-7.56 (m, 2H), 7.31-7.27 (m, 3H), 5.49 (s, 2H), 4.13 (d, *J* = 5.7 Hz, 2H), 4.10-4.07 (m, 2H), 3.55 (dd, *J* = 8.7 and 6.2 Hz, 1H), 1.77-1.71 (m, 1H), 1.66-1.61 (m, 1H), 1.47 (sep, *J* = 6.7 Hz, 1H), 1.36 (s, 9H), 1.14 (t, *J* = 7.1 Hz, 3H), 0.88 (d, *J* = 6.7 Hz, 3H), 0.86 (d, *J* = 6.7 Hz, 3H). ¹³C NMR (126 MHz, DMSO-*d₆*) *δ* ppm: 169.8, 167.2, 155.6, 145.9, 138.7, 131.2, 128.8 (2C), 122.6, 119.4 (2C), 78.0, 60.7, 52.3, 51.0, 37.4, 35.7, 28.3 (3C), 25.7, 22.7, 22.0, 14.1.

### Ethyl 2-[[4-[[4-[(4-iodoanilino)methyl]triazol-1-yl]methyl]phenyl]carbamoyl]-4-methyl-pentanoate (126)

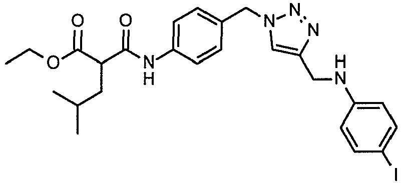

Compound **126** was synthesized according to the general procedure E, using azide **83** (100 mg, 0.31 mmol), alkyne **118** (80.7 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **126** as yellow oil (170 mg, 85%). LC tr = 4.96 min, MS (ESI+): m/z = 576 [M + H]⁺. ¹H NMR (500 MHz, DMSO-*d₆*) *δ* ppm: 10.31 (s, 1H), 7.96 (s, 1H), 7.56-7.55 (m, 2H), 7.33-7.31 (m, 2H), 7.27-7.25 (m, 2H), 6.48-6.46 (m, 2H), 6.29 (t, *J* = 8.1 Hz, 1H), 5.49 (s, 2H), 4.23 (d, *J* = 5.4 Hz, 2H), 4.12-4.08 (m, 2H), 3.55 (dd, *J* = 8.8 and 6.2 Hz, 1H), 1.77-1.71 (m, 1H), 1.66-1.61 (m, 1H), 1.47 (sep, *J* = 6.7 Hz, 1H), 1.15 (t, *J* = 7.1 Hz, 3H), 0.88 (d, *J* = 6.7 Hz, 3H), 0.86 (d, *J* = 6.7 Hz, 3H). ¹³C NMR (126 MHz, DMSO-*d₆*) *δ* ppm: 169.8, 167.2, 148.1, 145.6, 138.7, 137.1 (2C), 131.2, 128.8 (2C), 122.8, 119.5 (2C), 115.0 (2C), 76.6, 60.7, 52.4, 50.9, 38.3, 37.4, 25.7, 22.7, 22.0, 14.1.

### Ethyl 2-[[4-[[4-[(4-chlorophenoxy)methyl]triazol-1-yl]methyl]phenyl]carbamoyl]-4-methyl-pentanoate (127)

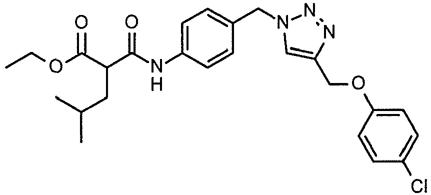

Compound **127** was synthesized according to the general procedure E, using azide **83** (70 mg, 0.22 mmol), alkyne **119** (36.6 mg, 0.22 mmol), copper (II) sulfate pentahydrate (11 mg, 0.04 mmol) and sodium ascorbate (21.8 mg, 0.11 mmol) in DMF (5 mL) and H₂O (4 mL) overnight. The crude product was purified by flash chromatography on silica gel (CH₂Cl₂ to CH₂Cl₂/MeOH: 95/5) affording compound **127** as a white solid (59 mg, 54%). LC tr = 4.99 min, MS (ESI+): m/z = 485 and 487 [M + H]⁺. ¹H NMR (500 MHz, DMSO-*d₆*) *δ* ppm: 10.30 (s, 1H), 8.23 (s, 1H), 7.59-7.57 (m, 2H), 7.33-7.29 (m, 4H), 7.07-7.02 (m, 2H), 5.54 (s, 2H), 5.12 (s, 2H), 4.13-4.05 (m, 2H), 3.56 (dd, *J* = 8.7 and 6.2 Hz, 1H), 1.77-1.72 (m, 1H), 1.67-1.62 (m, 1H), 1.48 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 156.9, 142.7, 138.8, 130.9, 129.2 (3C), 128.8 (2C), 124.6, 119.5 (2C), 116.5 (2C), 61.4, 60.7, 52.5, 50.9, 37.4, 25.6, 22.6, 21.9, 14.0.

### Ethyl 2-[[4-[[4-[(4-chlorophenyl)sulfanylmethyl]triazol-1-yl]methyl]phenyl]carbamoyl]-4-methyl-pentanoate (128)

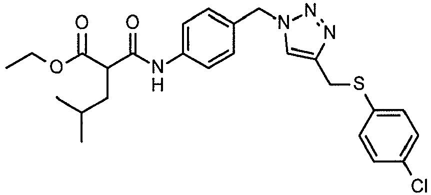

Compound **128** was synthesized according to the general procedure E, using azide **83** (70 mg, 0.22 mmol), alkyne **120** (40.2 mg, 0.22 mmol), copper (II) sulfate pentahydrate (11 mg, 0.04 mmol) and sodium ascorbate (21.8 mg, 0.11 mmol) in DMF (5 mL) and H₂O (4 mL) overnight. The crude product was purified by flash chromatography on silica gel (CH₂Cl₂ to CH₂Cl₂/MeOH: 95/5) affording compound **128** as a white solid (77 mg, 69%). LC tr = 5.01 min, MS (ESI+): m/z = 501 and 503 [M + H]⁺. ¹H NMR (500 MHz, DMSO-*d₆*) *δ* ppm: 10.28 (s, 1H), 7.92 (s, 1H), 7.57-7.55 (m, 2H), 7.36-7.31 (m, 4H), 7.21-7.19 (m, 2H), 5.47 (s, 2H), 4.27 (s, 2H), 4.13-4.05 (m, 2H), 3.56 (dd, *J* = 8.7 and 6.2 Hz, 1H), 1.77-1.72 (m, 1H), 1.68-1.62 (m, 1H), 1.49 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 143.5, 138.7, 134.7, 131.0, 130.7, 130.2 (2C), 128.8 (2C), 128.5 (2C), 123.3, 119.4 (2C), 60.6, 52.4, 50.9, 37.4, 27.3, 25.6, 22.6, 21.9, 14.0.

### Ethyl 2-[[4-[[4-[(4-chlorophenyl)methyl]triazol-1-yl]methyl]phenyl]carbamoyl]-4-methyl-pentanoate (129)

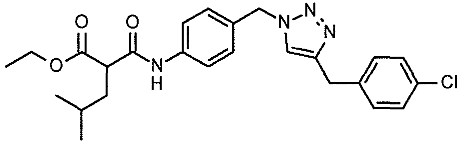

Compound **129** was synthesized according to the general procedure E, using azide **83** (100 mg, 0.31 mmol), alkyne **121** (47.3 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (cHex to cHex/EtOAc: 5/5) affording compound **129** as a white solid (85 mg, 54%). LC tr = 4.92 min, MS (ESI+): m/z = 469 and 471 [M+H]⁺. ¹H NMR (500 MHz, DMSO-*d₆*) *δ* ppm: 10.29 (s, 1H), 7.85 (s, 1H), 7.57-7.55 (m, 2H), 7.34-7.33 (m, 2H), 7.28-7.24 (m, 4H), 5.47 (s, 2H), 4.13-4.05 (m, 2H), 3.96 (s, 2H), 3.55 (dd, *J* = 8.7 and 6.2 Hz, 1H), 1.77-1.71 (m, 1H), 1.67-1.62 (m, 1H), 1.48 (sep, *J* = 6.7 Hz, 1H), 1.15 (t, *J* = 7.1 Hz, 3H), 0.88 (d, *J* = *6.7* Hz, 3H), 0.87 (d, *J* = 6.7 Hz, 3H). ¹³C NMR (126 MHz, DMSO-*d₆*) *δ* ppm: 169.7, 167.1, 145.9, 138.7, 138.6, 131.1, 130.8, 130.4 (2C), 128.7 (2C), 128.3 (2C), 122.5, 119.4 (2C), 60.7, 52.4, 50.9, 37.4, 30.5, 25.6, 22.6, 21.9, 14.0.

### Ethyl 2-[[4-[[4-(4-iodophenyl)triazol-1-yl]methyl]phenyl]carbamoyl]-4-methyl-pentanoate (130)

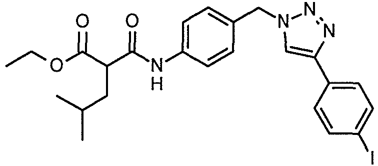

Compound **130** was synthesized according to the general procedure E, using azide **83** (100 mg, 0.31 mmol), alkyne **122** (71.6 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (cHex to cHex/EtOAc: 7/3) affording compound **130** as a white solid (151 mg, 82%). LC tr = 5.21 min, MS (ESI+): m/z = 547 [M + H]⁺. ¹H NMR (500 MHz, DMSO-*d₆*) *δ* ppm: 10.33 (s, 1H), 8.64 (s, 1H), 7.80-7.78 (m, 2H), 7.66-7.64 (m, 2H), 7.60-7.59 (m, 2H), 7.34-7.32 (m, 2H), 5.58 (s, 2H), 4.12-4.05 (m, 2H), 3.56 (dd, *J* = 8.8 and 6.1 Hz, 1H), 1.77-1.71 (m, 1H), 1.66-1.61 (m, 1H), 1.47 (sep, *J* = 6.7 Hz, 1H), 1.14 (t, *J* = 7.1 Hz, 3H), 0.88 (d, *J* = 6.7 Hz, 3H), 0.86 (d, *J* = 6.7 Hz, 3H). ¹³C NMR (126 MHz, DMSO-*d₆*) *δ* ppm: 169.7, 167.2, 145.7, 138.8, 137.7 (2C), 130.8, 130.2, 128.8 (2C), 127.2 (2C), 121.7, 119.5 (2C), 93.7, 60.7, 52.7, 50.9, 37.4, 25.6, 22.6, 21.9, 14.0.

### Ethyl 2-[[3-[[4-[[(4-iodophenyl)sulfonylamino]methyl]triazol-1-yl]methyl]phenyl]carbamoyl]-4-methyl-pentanoate (113)

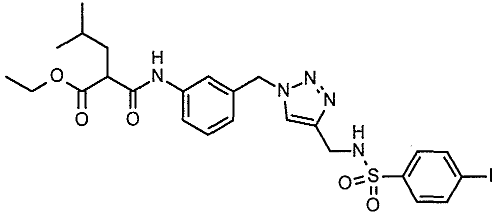

Compound **113** was synthesized according to the general procedure E, using azide **112** (110 mg, 0.35 mmol), alkyne **93** (111 mg, 0.35 mmol), copper (II) sulfate pentahydrate (17.3 mg, 0.07 mmol) and sodium ascorbate (34.2 mg, 0.17 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **113** as yellow oil (218 mg, 86%). LC tr = 4.71 min, MS (ESI+): m/z = 640 [M + H]⁺. ¹H NMR (500 MHz, DMSO-*d₆*) *δ* ppm: 10.30 (s, 1H), 8.21 (br s, 1H), 7.93-7.92 (m, 2H), 7.90 (s, 1H), 7.56-7.55 (m, 2H), 7.52-7.50 (m, 2H), 7.33-7.30 (m, 1H), 6.97-6.96 (m, 1H), 5.50 (s, 2H), 4.13-4.06 (m, 2H), 4.05-4.04 (m, 2H), 3.56 (dd, *J* = 8.7 and 6.2 Hz, 1H), 1.78-1.72 (m, 1H), 1.67-1.62 (m, 1H), 1.48 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 143.5, 140.0, 139.2, 138.0 (2C), 136.6, 129.3, 128.3 (2C), 123.5, 123.2, 119.0, 118.7, 100.4, 60.7, 52.7, 50.9, 38.0, 37.4, 25.7, 22.7, 21.9, 14.1.

### Ethyl 2-[[4-[[4-[[(4-fluorophenyl)sulfonylamino]methyl]triazol-1-yl]methyl]phenyl]carbamoyl]-4-methyl-pentanoate (150)

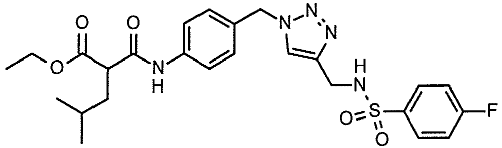

Compound **150** was synthesized according to the general procedure E, using azide **83** (100 mg, 0.31 mmol), alkyne **141** (67 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **150** as a pale yellow solid (137 mg, 76%). LC tr = 4.38 min, MS (ESI+): m/z = 532 [M+H]⁺. ¹H NMR (500 MHz, DMSO-*d₆*) *δ* ppm: 10.29 (s, 1H), 8.15 (t, *J* = 5.8 Hz, 1H), 7.85 (s, 1H), 7.82-7.79 (m, 2H), 7.58-7.56 (m, 2H), 7.37-7.33 (m, 2H), 7.25-7.23 (m, 2H), 5.45 (s, 2H), 4.13-4.06 (m, 2H), 4.04 (d, *J* = 5.8 Hz, 2H), 3.56 (dd, *J* = 8.7 and 6.2 Hz, 1H), 1.77-1.72 (m, 1H), 1.68-1.62 (m, 1H), 1.49 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 164.1 (d, *J* = 250.8 Hz), 143.5, 138.7, 136.8 (d, *J* = 2.9 Hz), 130.9, 129.6 (d, *J* = 9.2 Hz, 2C), 128.8 (2C), 123.2, 119.4 (2C), 116.2 (d, *J* = 22.1 Hz, 2C), 60.7, 52.4, 51.0, 38.0, 37.4, 25.7, 22.7, 21.9, 14.0.

### Ethyl 2-[[4-[[4-[[(4-chlorophenyl)sulfonylamino]methyl]triazol-1-yl]methyl]phenyl]carbamoyl]-4-methyl-pentanoate (151)

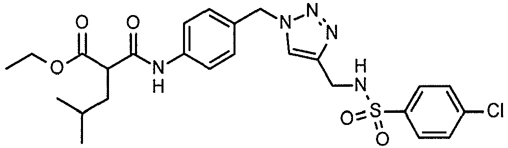

Compound **151** was synthesized according to the general procedure E, using azide **83** (100 mg, 0.31 mmol), alkyne **142** (72.1 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **151** as a colorless wax (138 mg, 73%). LC tr = 4.55 min, MS (ESI+): m/z = 548 and 550 [M + H]⁺. ¹H NMR (500 MHz, DMSO-*d₆*) *δ* ppm: 10.29 (s, 1H), 8.22 (t, *J* = 5.9 Hz, 1H), 7.86 (s, 1H), 7.75-7.73 (m, 2H), 7.59-7.57 (m, 4H), 7.25-7.24 (m, 2H), 5.45 (s, 2H), 4.11-4.05 (m, 4H), 3.56 (dd, *J* = 8.7 and 6.2 Hz, 1H), 1.78-1.72 (m, 1H), 1.68-1.62 (m, 1H), 1.49 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 143.4, 139.3, 138.7, 137.2, 130.9, 129.2 (2C), 128.8 (2C), 128.5 (2C), 123.2, 119.4 (2C), 60.7, 52.3, 50.9, 38.0, 37.4, 25.6, 22.6, 21.9, 14.0.

### Ethyl 2-[[4-[[4-[[(4-methoxyphenyl)sulfonylamino]methyl]triazol-1-yl]methyl]phenyl]carbamoyl]-4-methyl-pentanoate (152)

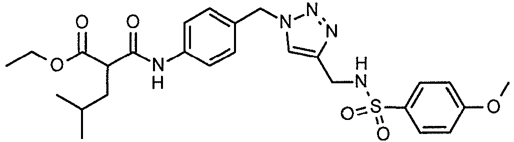

Compound **152** was synthesized according to the general procedure E, using azide **83** (100 mg, 0.31 mmol), alkyne **143** (70.8 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **152** as a white solid (141 mg, 76%). LC tr = 4.34 min, MS (ESI+): m/z = 544 [M + H]⁺. ¹H NMR (500 MHz, DMSO-*d₆*) *δ* ppm: 10.29 (s, 1H), 7.92 (t, *J* = 5.8 Hz, 1H), 7.85 (s, 1H), 7.70-7.69 (m, 2H), 7.58-7.56 (m, 2H), 7.25-7.24 (m, 2H), 7.07-7.05 (m, 2H), 5.46 (s, 2H), 4.13-4.05 (m, 2H), 3.98 (d, *J* = 5.9 Hz, 2H), 3.82 (s, 3H), 3.56 (dd, *J* = 8.9 and 6.5 Hz, 1H), 1.77-1.72 (m, 1H), 1.68-1.62 (m, 1H), 1.48 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 162.1, 143.7, 138.7, 131.9, 131.0, 128.7 (4C), 123.2, 119.4 (2C), 114.3 (2C), 60.7, 55.6, 52.4, 51.0, 38.1, 37.4, 25.7, 22.7, 21.9, 14.0.

### Ethyl 2-[[4-[[4-[[(2-chlorophenyl)sulfonylamino]methyl]triazol-1-yl]methyl]phenyl]carbamoyl]-4-methyl-pentanoate (153)

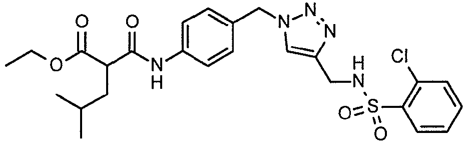

Compound **153** was synthesized according to the general procedure E, using azide **83** (100 mg, 0.31 mmol), alkyne **144** (72.1 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **153** as colorless oil (170 mg, 82%). LC tr = 4.40 min, MS (ESI+): m/z = 548 and 550 [M + H]⁺. ¹H NMR (500 MHz, DMSO-*d₆*) *δ* ppm: 10.31 (s, 1H), 8.37 (br s, 1H), 7.88 (dd, *J* = 7.9 and 1.6 Hz, 1H), 7.73 (s, 1H), 7.59-7.57 (m, 2H), 7.53 (td, *J* = 11.5 and 1.6 Hz, 1H), 7.48 (dd, *J* = 7.9 and 1.3 Hz, 1H), 7.42 (td, *J* = 11.3 and 1.3 Hz, 1H), 7.23-7.22 (m, 2H), 5.42 (s, 2H), 4.15 (br s, 2H), 4.11-4.08 (m, 2H), 3.56 (dd, *J* = 9.0 and 6.4 Hz, 1H), 1.78-1.73 (m, 1H), 1.68-1.63 (m, 1H), 1.49 (sep, *J* = 6.6 Hz, 1H), 1.15 (t, *J* = 7.0 Hz, 3H), 0.89 (d, *J* = 6.6 Hz, 3H), 0.87 (d, *J* = 6.6 Hz, 3H). ¹³C NMR (126 MHz, DMSO-*d₆*) *δ* ppm: 169.7, 167.2, 143.6, 138.7, 138.0, 133.8, 131.5, 130.9, 130.5, 130.3, 128.8 (2C), 127.4, 123.0, 119.5 (2C), 60.7, 52.3, 51.0, 37.8, 37.4, 25.7, 22.6, 21.9, 14.0.

### Ethyl 2-[[4-[[4-[[(3-chlorophenyl)sulfonylamino]methyl]triazol-1-yl]methyl]phenyl]carbamoyl]-4-methyl-pentanoate (154)

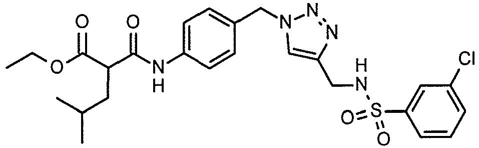

Compound **154** was synthesized according to the general procedure E, using azide **83** (100 mg, 0.31 mmol), alkyne **145** (72.1 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **154** as a white solid (136 mg, 74%). LC tr = 4.55 min, MS (ESI+): m/z = 548 and 550 [M + H]⁺. ¹H NMR (500 MHz, DMSO-*d₆*) *δ* ppm: 10.30 (s, 1H), 8.28 (br s, 1H), 7.82 (s, 1H), 7.74 (t, *J* = 1.7 Hz, 1H), 7.70-7.68 (m, 1H), 7.66-7.64 (m, 1H), 7.58-7.56 (m, 2H), 7.53 (t, *J* = 7.9 Hz, 1H), 7.25-7.23 (m, 2H), 5.44 (s, 2H), 4.13-4.06 (m, 4H), 3.56 (dd, *J* = 8.7 and 6.2 Hz, 1H), 1.78-1.72 (m, 1H), 1.68-1.62 (m, 1H), 1.49 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 143.3, 142.4, 138.7, 133.7, 132.3, 131.1, 130.9, 128.8 (2C), 126.1, 125.2, 123.1, 119.5 (2C), 60.7, 52.4, 50.9, 38.0, 37.4, 25.7, 22.6, 21.9, 14.0.

### Ethyl 2-[[4-[[4-[[(3,4-dichlorophenyl)sulfonylamino]methyl]triazol-1-yl]methyl]phenyl]carbamoyl]-4-methyl-pentanoate (155)

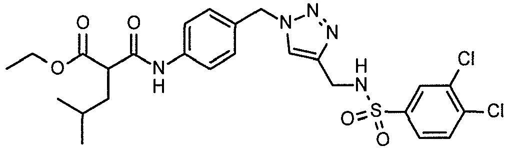

Compound **155** was synthesized according to the general procedure E, using azide **83** (70 mg, 0.22 mmol), alkyne **146** (58.1 mg, 0.22 mmol), copper (II) sulfate pentahydrate (11 mg, 0.04 mmol) and sodium ascorbate (21.8 mg, 0.11 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **155** as a white powder (108 mg, 77%). LC tr = 4.76 min, MS (ESI+): m/z = 582, 584 and 586 [M + H]⁺. ¹H NMR (500 MHz, DMSO-*d₆*) *δ* ppm: 10.29 (s, 1H), 8.36 (br s, 1H), 7.91 (d, *J* = 2.1 Hz, 1H), 7.88 (s, 1H), 7.77 (d, *J* = 8.4 Hz, 1H), 7.67 (dd, *J* = 8.4 and 2.1 Hz, 1H), 7.58-7.57 (m, 2H), 7.25-7.23 (m, 2H), 5.45 (s, 2H), 4.11-4.05 (m, 4H), 3.56 (dd, *J* = 8.8 and 6.2 Hz, 1H), 1.78-1.72 (m, 1H), 1.68-1.62 (m, 1H), 1.49 (sep, *J* = 6.6 Hz, 1H), 1.15 (t, *J* = 7.1 Hz, 3H), 0.89 (d, *J* = 6.6 Hz, 3H), 0.87 (d, *J* = 6.6 Hz, 3H). ¹³C NMR (126 MHz, DMSO-*d₆*) *δ* ppm: 169.7, 167.1, 143.2, 140.9, 138.7, 135.4, 131.9, 131.4, 130.8, 128.7 (2C), 128.4, 126.6, 123.2, 119.5 (2C), 60.7, 52.4, 50.9, 38.0, 37.4, 25.6, 22.6, 21.9, 14.0.

### Ethyl 2-[[4-[[4-[[(4-chlorophenyl)methylsulfonylamino]methyl]triazol-1-yl]methyl]phenyl]carbamoyl]-4-methyl-pentanoate (156)

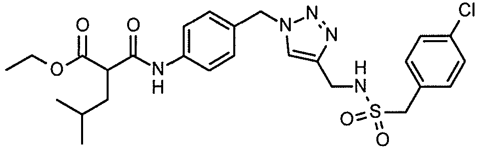

Compound **156** was synthesized according to the general procedure E, using azide **83** (70 mg, 0.22 mmol), alkyne **147** (53.6 mg, 0.22 mmol), copper (II) sulfate pentahydrate (11 mg, 0.04 mmol) and sodium ascorbate (21.8 mg, 0.11 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **156** as colorless oil (65 mg, 45%). LC tr = 4.60 min, MS (ESI+): m/z = 562 and 564 [M + H]⁺. ¹H NMR (500 MHz, DMSO-*d₆*) *δ* ppm: 10.29 (s, 1H), 7.99 (s, 1H), 7.63 (t, *J* = 5.9 Hz, 1H), 7.59-7.57 (m, 2H), 7.37-7.36 (m, 2H), 7.32-7.30 (m, 2H), 7.28-7.27 (m, 2H), 5.53 (s, 2H), 4.34 (s, 2H), 4.17 (d, *J* = 5.9 Hz, 2H), 4.13-4.05 (m, 2H), 3.55 (dd, *J* = 8.6 and 6.2 Hz, 1H), 1.77-1.71 (m, 1H), 1.68-1.62 (m, 1H), 1.48 (sep, *J* = 6.7 Hz, 1H), 1.15 (t, *J* = 7.1 Hz, 3H), 0.88 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 144.7, 138.8, 132.9, 132.5 (2C), 131.1, 129.3, 128.8 (2C), 128.3 (2C), 123.2, 119.4 (2C), 60.7, 56.7, 52.4, 50.9, 38.0, 37.4, 25.6, 22.6, 21.9, 14.0.

### Ethyl 2-[[4-[[4-(benzenesulfonamidomethyl)triazol-1-yl]methyl]phenyl]carbamoyl]-4-methyl-pentanoate (157)

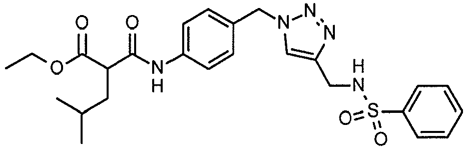

Compound **157** was synthesized according to the general procedure E, using azide **83** (100 mg, 0.31 mmol), alkyne **148** (61.3 mg, 0.31 mmol), copper (II) sulfate pentahydrate (15.7 mg, 0.06 mmol) and sodium ascorbate (31.1 mg, 0.17 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **157** as colorless wax (123 mg, 68%). LC tr = 4.32 min, MS (ESI+): m/z = 514 [M+H]⁺. ¹H NMR (500 MHz, DMSO-*d₆*) *δ* ppm: 10.33 (s, 1H), 8.13 (t, *J* = 5.9 Hz, 1H), 7.81 (s, 1H), 7.76-7.74 (m, 2H), 7.61-7.51 (m, 5H), 7.25-7.23 (m, 2H), 5.44 (s, 2H), 4.12-4.05 (m, 2H), 4.03 (d, *J* = 5.9 Hz, 2H), 3.56 (dd, *J* = 9.0 and 6.1 Hz, 1H), 1.78-1.72 (m, 1H), 1.67-1.62 (m, 1H), 1.48 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 170.2, 167.6, 141.1, 140.8, 139.2, 132.8, 131.4, 129.5 (2C), 129.2 (2C), 126.9 (2C), 123.6, 119.9 (2C), 61.1, 52.8, 51.4, 38.6, 37.8, 26.1, 23.1, 22.4, 14.5.

### Ethyl 2-[[4-[[4-[(cyclohexylsulfonylamino)methyl]triazol-1-yl]methyl]phenyl]carbamoyl]-4-methyl-pentanoate (158)

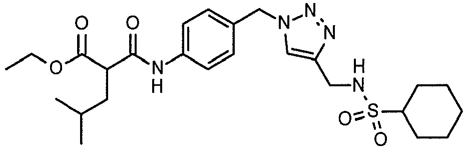

Compound **158** was synthesized according to the general procedure E, using azide **83** (70 mg, 0.22 mmol), alkyne **149** (44.3 mg, 0.22 mmol), copper (II) sulfate pentahydrate (11 mg, 0.04 mmol) and sodium ascorbate (21.8 mg, 0.11 mmol) in DMF (5.5 mL) and H₂O (4.5 mL) overnight. The crude product was purified by flash chromatography on silica gel (CH₂Cl₂ to CH₂Cl₂/MeOH: 9/1) affording compound **158** as colorless oil (58 mg, 47%). LC tr = 4.39 min, MS (ESI+): m/z = 520 [M + H]⁺. ¹H NMR (500 MHz, DMSO-*d₆*) *δ* ppm: 10.28 (s, 1H), 8.01 (s, 1H), 7.58-7.56 (m, 2H), 7.50 (t, *J* = 5.4 Hz, 1H), 7.31-7.29 (m, 2H), 5.52 (s, 2H), 4.18 (d, *J* = 4.6 Hz, 2H), 4.10-4.07 (m, 2H), 3.56-3.53 (m, 1H), 2.71-2.64 (m, 1H), 1.89-1.87 (m, 2H), 1.77-1.71 (m, 1H), 1.67-1.64 (m, 3H), 1.53-1.52 (m, 1H), 1.49-1.46 (m, 1H), 1.23-1.21 (m, 2H), 1.15 (t, *J* = 6.8 Hz, 3H), 1.03-1.00 (m, 3H), 0.89-0.86 (m, 6H). ¹³C NMR (126 MHz, DMSO-*d₆*) *δ* ppm: 169.7, 167.1, 144.9, 138.7, 131.1, 128.7 (2C), 123.1, 119.4 (2C), 60.6, 59.4, 52.4, 50.9, 37.7, 37.4, 25.9 (2C), 25.6, 24.8, 24.4 (2C), 22.6, 21.9, 14.0.

### Ethyl 2-[[4-[[4-[(isobutylsulfonylamino)methyl]triazol-1-yl]methyl]phenyl]carbamoyl]-4-methyl-pentanoate (159)

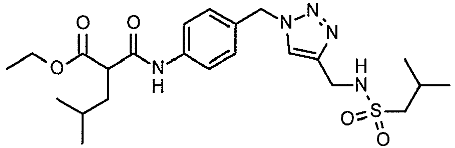

Compound **159** was synthesized according to the general procedure E, using azide **83** (100 mg, 0.31 mmol), alkyne **106** (55 mg, 0.31 mmol), copper (II) sulfate pentahydrate (15.7 mg, 0.06 mmol) and sodium ascorbate (31.1 mg, 0.17 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **159** as colorless oil (85 mg, 51%). LC tr = 4.29 min, MS (ESI+): m/z = 494 [M + H]⁺. ¹H NMR (500 MHz, DMSO-*d₆*) *δ* ppm: 10.29 (s, 1H), 8.01 (s, 1H), 7.58-7.56 (m, 2H), 7.53 (t, *J* = 5.9 Hz, 1H), 7.30-7.28 (m, 2H), 5.52 (s, 2H), 4.18 (d, *J* = 5.9 Hz, 2H), 4.10-4.07 (m, 2H), 3.55 (dd, *J* = 8.6 and 6.3 Hz, 1H), 2.80 (d, *J* = 6.4 Hz, 2H), 1.97 (sep, *J* = 6.7 Hz, 1H), 1.77-1.71 (m, 1H), 1.67-1.62 (m, 1H), 1.48 (sep, *J* = 6.7 Hz, 1H), 1.15 (t, *J* = 7.1 Hz, 3H), 0.91 (d, *J* = 6.7 Hz, 6H), 0.88 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 144.6, 138.7, 131.1, 128.7 (2C), 123.2, 119.4 (2C), 60.7, 59.2, 52.4, 50.9, 37.6, 37.4, 25.6, 24.1, 22.7, 22.1 (2C), 21.9, 14.0.

### Ethyl 2-benzyl-3-[4-[[4-[[(4-iodophenyl)sulfonylamino]methyl]triazol-1-yl]methyl]anilino]-3-oxo-propanoate (177)

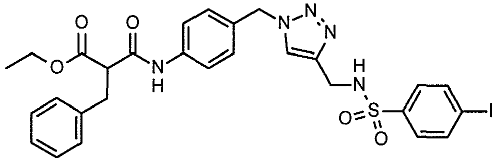

Compound **177** was synthesized according to the general procedure E, using azide **173** (65 mg, 0.18 mmol), alkyne **93** (59.2 mg, 0.18 mmol), copper (II) sulfate pentahydrate (9.21 mg, 0.04 mmol) and sodium ascorbate (18.3 mg, 0.09 mmol) in DMF (5 mL) and H₂O (4 mL) overnight. The crude product was purified by flash chromatography on silica gel (CH₂Cl₂ to CH₂Cl₂/MeOH: 95/5) affording compound **176** as a colorless solid (94 mg, 74%). LC tr = 4.59 min, MS (ESI+): m/z = 674 [M + H]⁺. ¹H NMR (500 MHz, DMSO-*d₆*) *δ* ppm: 10.20 (s, 1H), 8.18 (t, *J* = 5.6 Hz, 1H), 7.93-7.91 (m, 2H), 7.84 (s, 1H), 7.51-7.49 (m, 4H), 7.26-7.22 (m, 6H), 7.18-7.15 (m, 1H), 5.44 (s, 2H), 4.08 (q, *J* = 7.0 Hz, 2H), 4.02 (d, *J* = 5.5 Hz, 2H), 3.82 (dd, *J* = 8.2 and 7.1 Hz, 1H), 3.16-3.08 (m, 2H), 1.12 (t, *J* = 7.0 Hz, 3H). ¹³C NMR (126 MHz, DMSO-*d₆*) *δ* ppm: 169.0, 166.4, 143.4, 140.0, 138.5, 138.4, 138.0 (2C), 130.9, 128.8 (2C), 128.7 (2C), 128.3 (2C), 128.2 (2C), 126.4, 123.2, 119.5 (2C), 100.4, 60.8, 54.3, 52.3, 38.0, 34.2, 14.0.

### 2-(Hydroxycarbamoyl)-N-[4-[[4-[[(4-iodophenyl)sulfonylamino]methyl]triazol-1-yl]methyl]phenyl]-4-methyl-pentanamide (Hit L1)

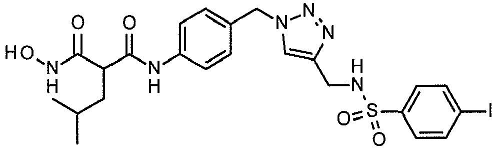

**Hit L1** was synthesized according to the general procedure D, using ester **88** (110 mg, 0.17 mmol), KCN (2.27 mg, 0.03 mmol) and NH₂OH (1.5 mL, 50% w/w in water) in MeOH (1.5 mL) overnight. The crude product was purified by flash chromatography on silica gel (CH₂Cl₂ to CH₂Cl₂/MeOH: 95/5) affording compound **Hit L1** as a white solid after lyophilization (96 mg, 85%). Purity: 97%, LC tr = 3.77 min, MS (ESI+): m/z = 627 [M + H]⁺. ¹H NMR (300 MHz, DMSO-*d₆*) *δ* ppm: 10.54 (br s, 1H), 9.92 (s, 1H), 8.96 (br s, 1H), 8.34-8.08 (m, 1H), 7.93-7.91 (m, 2H), 7.83 (s, 1H), 7.58-7.57 (m, 2H), 7.51-7.49 (m, 2H), 7.24-7.23 (m, 2H), 5.44 (s, 2H), 4.03 (s, 2H), 3.23 (t, *J* = 7.5 Hz, 1H), 1.67 (t, *J* = 7.1 Hz, 2H), 1.46 (sep, *J* = 6.6 Hz, 1H), 0.85-0.87 (m, 6H). ¹³C NMR (126 MHz, DMSO-*d₆*) *δ* ppm: 167.9, 166.3, 143.5, 140.1, 138.8, 138.0 (2C), 130.7, 128.7 (2C), 128.3 (2C), 123.2, 119.6 (2C), 100.4, 52.4, 49.9, 38.1, 38.0, 25.7, 22.4, 22.3. HRMS-ESI+ (m/z): calcd. for C₂₃H₂₈IN₆O₅S [M + H]⁺ 627.0887, found 627.0877.

### N-[4-[[4-[[(7,7-Dimethyl-2-oxo-norbornan-1-yl)methylsulfonylamino]methyl] triazol-1-yl]methyl]phenyl]-2-(hydroxycarbamoyl)-4-methyl-pentanamide (Hit L2)

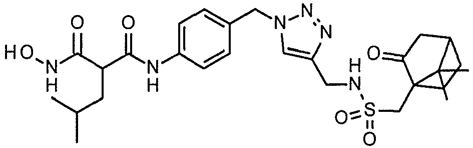

**Hit L2** was synthesized according to the general procedure D, using ester **89** (127 mg, 0.22 mmol), KCN (2.85 mg, 0.04 mmol) and NH₂OH (2 mL, 50% w/w in water) in MeOH (2 mL) overnight. The crude product was purified by flash chromatography on silica gel (CH₂Cl₂ to CH₂Cl₂/MeOH: 95/5) followed by preparative HPLC (H₂O+0.05%FA / ACN+0.05%FA: 95:5 to 5:95) affording compound **Hit L2** as a white solid after lyophilization (13 mg, 10%). Purity: 100%, LC tr = 3.57 min, MS (ESI+): m/z = 575 [M + H]⁺. ¹H NMR (500 MHz, DMSO-*d₆*) *δ* ppm: 10.52 (br s, 1H), 9.81 (s, 1H), 8.96 (s, 1H), 8.00 (s, 1H), 7.57-7.55 (m, 3H), 7.28-7.26 (m, 2H), 5.51 (s, 2H), 4.26-4.24 (m, 2H), 3.24-3.18 (m, 2H), 2.87-2.84 (m, 1H), 2.33-2.28 (m, 2H), 2.01 (t, *J* = 4.4 Hz, 1H), 1.91-1.87 (m, 2H), 1.69-1.65 (m, 2H), 1.50-1.46 (m, 2H), 1.39-1.34 (m, 1H), 0.96 (s, 3H), 0.87 (d, *J* = 6.6 Hz, 3H), 0.86 (d, *J* = 6.6 Hz, 3H), 0.71 (s, 3H). ¹³C NMR (126 MHz, DMSO-*d₆*) *δ* ppm: 214.6, 167.8, 168.3, 144.6, 138.7, 130.9, 128.6 (2C), 123.2, 119.5 (2C), 57.9, 52.4, 50.0, 48.6, 47.6, 42.0, 41.9, 38.0, 38.0, 26.3, 25.7, 24.5, 22.4, 22.3, 19.4, 19.2. HRMS-ESI+ (m/z): calcd. for C₂₇H₃₉N₆O₆S [M + H]⁺: 575.2652, found 575.2645.

### 2-(Hydroxycarbamoyl)-4-methyl-N-[4-[[4-[[(4-phenylphenyl)sulfonylamino] methyl]triazol-1-yl]methyl]phenyl]pentanamide (Hit L3)

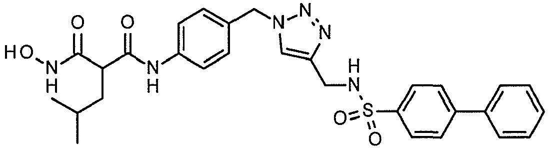

**Hit L3** was synthesized according to the general procedure D, using ester **90** (129 mg, 0.22 mmol), KCN (2.85 mg, 0.04 mmol) and NH₂OH (2 mL, 50% w/w in water) in MeOH (2 mL) overnight. The crude product was purified by flash chromatography on silica gel (CH₂Cl₂ to CH₂Cl₂/MeOH: 95/5) affording compound **Hit L3** as a white solid after lyophilization (42 mg, 32%). Purity: 100%, LC tr = 4.02 min, MS (ESI+): m/z= 577 [M + H]⁺. ¹H NMR (500 MHz, DMSO-*d₆*) *δ* ppm: 10.52 (s, 1H), 9.82 (s, 1H), 8.97 (s, 1H), 7.62-7.59 (m, 2H), 7.32-7.30 (m, 2H), 4.37 (s, 2H), 3.22 (t, *J* = 7.6 Hz, 1H), 1.69 (t, *J* = 7.3 Hz, 2H), 1.49 (sep, *J* = 6.7 Hz, 1H), 0.89 (d, *J* = 2.0 Hz, 3H), 0.87 (d, *J* = 2.0 Hz, 3H). ¹³C NMR (126 MHz, DMSO-*d₆*) *δ* ppm: 167.9, 166.2, 143.9, 143.6, 139.1, 138.8, 138.5, 130.7, 129.2 (2C), 128.6 (2C), 128.5, 127.3 (2C), 127.3 (2C), 127.1 (2C), 123.2, 119.5 (2C), 52.4, 49.8, 38.2, 37.9, 25.7, 22.4, 22.3. HRMS-ESI+ (m/z): calcd. for C₂₉H₃₃N₆O₅S [M + H]⁺ 577.2233, found 577.2221.

### N-[4-[[4-[3-(4-Fluorobenzoyl)thiazolidin-4-yl]triazol-1-yl]methyl]phenyl]-2-(hydroxycarbamoyl)-4-methyl-pentanamide (Hit L4)

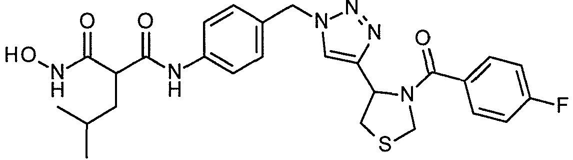

Compound **Hit L4** was synthesized according to the general procedure D, using ester **91** (154 mg, 0.28 mmol), KCN (3.7 mg, 0.06 mmol) and NH₂OH (2.5 mL, 50% w/w in water) in MeOH (2.5 mL) overnight. The crude product was purified by flash chromatography on silica gel (CH₂Cl₂ to CH₂Cl₂/MeOH: 95/5) affording compound **Hit L4** as a white solid after lyophilization (26 mg, 21%). Purity: 95%, LC tr = 3.63 min, MS (ESI+): m/z = 541 [M + H]⁺. ¹H NMR (500 MHz, DMSO-*d₆*) *δ* ppm: 10.53 (s, 1H), 9.82 (s, 1H), 8.96 (s, 1H), 8.16 (s, 1H), 7.58-7.57 (m, 4H), 7.28-7.26 (m, 4H), 5.98-5.60 (m, 1H), 5.50 (s, 2H), 4.57 (br s, 2H), 3.44 (s, 1H), 3.22-3.21 (m, 2H), 1.69-1.66 (m, 2H), 1.50-1.44 (m, 1H), 1.23-0.97 (m, 1H), 0.88-0.86 (m, 6H). ¹³C NMR (126 MHz, DMSO-*d₆*) *δ* ppm: 167.8, 167.7, 166.3, 163.0 (d, *J* = 239.2 Hz), 146.7, 138.7, 130.8, 129.9 (d, *J* = 11.9 Hz), 129.3, 128.6 (2C), 122.8 (2C), 119.6 (2C), 115.4 (d, *J* = 21.9 Hz, 2C), 53.0, 52.5, 50.0, 38.0, 35.7, 25.7, 23.7, 22.4, 22.3. HRMS-ESI+ (m/z): calcd. for C₂₆H₃₀FN₆O₄S [M + H]⁺ 541,2033, found 541.2024.

### N-[4-[[4-[(2-Acetamidophenoxy)methyl]triazol-1-yl]methyl]phenyl]-2-(hydroxycarbamoyl)-4-methyl-pentanamide (Hit L5)

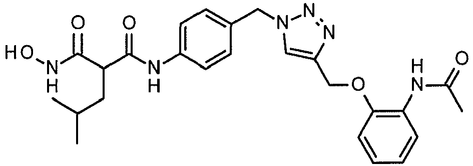

**Hit L5** was synthesized according to the general procedure D, using ester **92** (66 mg, 0.13 mmol), KCN (1.7 mg, 0.03 mmol) and NH₂OH (1.3 mL, 50% w/w in water) in MeOH (1.3 mL) overnight. The crude product was purified by preparative HPLC (H₂O+0.05%FA / ACN+0.05%FA: 95:5 to 5:95) affording compound **Hit L5** as a white solid after lyophilization (18 mg, 27%). Purity: 100%, LC tr = 3.30 min, MS (ESI+): m/z = 495 [M + H]⁺. ¹H NMR (500 MHz, DMSO-*d₆*) δ ppm: 10.51 (br s, 1H), 9.83 (s, 1H), 9.01 (s, 1H), 8.97 (br s, 1H), 8.22 (s, 1H), 7.86 (d, *J* = 7.6 Hz, 1H), 7.57-7.56 (m, 2H), 7.27-7.25 (m, 2H), 7.20 (d, *J* = 8.1 Hz, 1H), 7.04 (t, *J* = 7.6 Hz, 1H), 6.90 (t, *J* = 7.5 Hz, 1H), 5.54 (s, 2H), 5.20 (s, 2H), 3.21 (t, *J* = 7.6 Hz, 1H), 2.03 (s, 3H), 1.67 (t, *J* = 7.0 Hz, 2H), 1.47 (sep, *J* = 6.6 Hz, 1H), 0.87 (d, *J* = 6.5 Hz, 3H), 0.86 (d, *J* = 6.5 Hz, 3H). ¹³C NMR (126 MHz, DMSO-*d₆*) δ ppm: 168.4, 167.8, 166.2, 148.5, 143.1, 138.7, 130.8, 128.6 (2C), 127.9, 124.5, 124.3, 122.5, 120.8, 119.6 (2C), 113.3, 62.3, 52.5, 49.9, 38.0, 25.7, 23.9, 22.4, 22.3. HRMS-ESI+ (m/z): calcd. for C₂₅H₃₁N₆O₅ [M + H]⁺ 495.2278, found 495.2352.

### 2-(Hydroxycarbamoyl)-N-[4-[[4-[[(4-iodophenyl)sulfonyl-methyl-amino]methyl]triazol-1-yl]methyl]phenyl]-4-methyl-pentanamide (131)

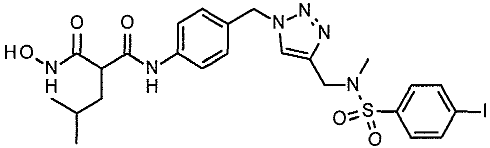

Compound **131** was synthesized according to the general procedure D, using ester **123** (120 mg, 0.18 mmol), KCN (3.55 mg, 0.05 mmol) and NH₂OH (2.5 mL, 50% w/w in water) in MeOH (2.5 mL) for 72h. The crude product was purified by flash chromatography on silica gel (CH₂Cl₂ to CH₂Cl₂/MeOH: 95/5) affording compound **131** as a white solid after lyophilization (64 mg, 55%). Purity: 100%, LC tr = 4.07 min, MS (ESI+): m/z = 641 [M + H]⁺. ¹H NMR (500 MHz, DMSO-*d₆*) *δ* ppm: 10.52 (s, 1H), 9.81 (s, 1H), 8.97 (s, 1H), 8.03 (s, 1H), 7.96-7.94 (m, 2H), 7.59-7.57 (m, 2H), 7.52-7.51 (m, 2H), 7.26-7.24 (m, 2H), 5.48 (s, 2H), 4.26 (s, 2H), 3.20 (t, *J* = 7.6 Hz, 1H), 2.63 (s, 3H), 1.68 (t, *J* = 7.3 Hz, 2H), 1.47 (sep, *J* = 6.6 Hz, 1H), 0.87 (d, *J* = 6.5 Hz, 3H), 0.86 (d, *J* = 6.5 Hz, 3H). ¹³C NMR (126 MHz, DMSO-*d₆*) *δ* ppm: 167.8, 166.3, 141.8, 138.7, 138.2 (2C), 136.5, 130.7, 128.9 (2C), 128.6 (2C), 124.0, 119.6 (2C), 101.3, 52.5, 50.0, 44.7, 38.0, 34.7, 25.7, 22.4, 22.3. HRMS-ESI+ (m/z): calcd. for C₂₄H₃₀IN₆O₅S [M+H]⁺ 641.1043, found 641.1033.

### N-[[1-[[4-[[2-(Hydroxycarbamoyl)-4-methyl-pentanoyl]amino]phenyl]methyl] triazol-4-yl]methyl]-4-iodo-benzamide (132)

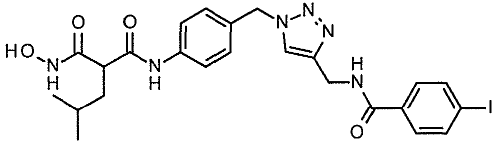

Compound **132** was synthesized according to the general procedure D, using ester **124** (132 mg, 0.22 mmol), KCN (4.23 mg, 0.07 mmol) and NH₂OH (2 mL, 50% w/w in water) in MeOH (2 mL) for 72h. The crude product was purified by flash chromatography on silica gel (CH₂Cl₂ to CH₂Cl₂/MeOH: 9/1) affording compound **132** as a white solid after lyophilization (53 mg, 41%). Purity: 100%, LC tr = 3.73 min, MS (ESI+): m/z = 591 [M + H]⁺. ¹H NMR (500 MHz, DMSO-*d₆*) *δ* ppm: 10.52 (s, 1H), 9.80 (s, 1H), 9.05 (t, *J* = 7.1 Hz, 1H), 8.97 (s, 1H), 7.97 (s, 1H), 7.85-7.83 (m, 2H), 7.64-7.63 (m, 2H), 7.56-7.54 (m, 2H), 7.28-7.26 (m, 2H), 5.48 (s, 2H), 4.47 (d, *J* = 4.3 Hz, 2H), 3.20 (t, *J* = 6.6 Hz, 1H), 1.67 (t, *J* = 7.3 Hz, 2H), 1.49-1.44 (m, 1H), 0.88-0.85 (m, 6H). ¹³C NMR (126 MHz, DMSO-*d₆*) *δ* ppm: 167.8, 166.3, 165.5, 145.1, 138.7, 137.2 (2C), 133.5, 131.0, 129.3 (2C), 128.7 (2C), 122.9, 119.6 (2C), 99.0, 52.4, 49.9, 38.0, 34.9, 25.7, 22.4, 22.3. HRMS-ESI+ (m/z): calcd. for C₂₄H₂₈IN₆O₄ [M + H]⁺ 591.1217, found 591.1208.

### Tert-butyl N-[[1-[[4-[[2-(hydroxycarbamoyl)-4-methyl-pentanoyl]amino]phenyl] methyl]triazol-4-yl]methyl]carbamate (133)

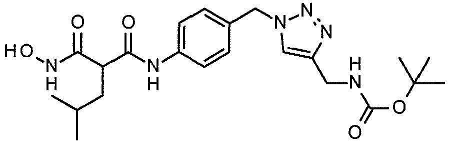

Compound **133** was synthesized according to the general procedure D, using ester **125** (105 mg, 0.22 mmol), KCN (4.29 mg, 0.07 mmol) and NH₂OH (2.3 mL, 50% w/w in water) in MeOH (2.3 mL) for 48h. The crude product was purified by preparative HPLC (H₂O+0.05%FA / ACN+0.05%FA: 95:5 to 5:95) affording compound **133** as a white solid after lyophilization (36 mg, 35%). Purity: 100%, LC tr = 3.41 min, MS (ESI+): m/z = 921 [2M + H]⁺, 461 [M + H]⁺, 405 [M - tBu + H]⁺, 361 [M - Boc + H]⁺. ¹H NMR (500 MHz, DMSO-*d₆*) *δ* ppm: 10.54 (s, 1H), 9.83 (s, 1H), 8.99 (s, 1H), 7.87 (s, 1H), 7.57-7.55 (m, 2H), 7.31 (t, *J* = 5.8 Hz, 1H), 7.26-7.25 (m, 2H), 5.49 (s, 2H), 4.13 (d, *J* = 5.9 Hz, 2H), 3.20 (t, *J* = 7.6 Hz, 1H), 1.67 (t, *J* = 7.3 Hz, 2H), 1.46 (sep, *J* = 6.6 Hz, 1H), 1.36 (s, 9H), 0.87 (d, *J* = 6.5 Hz, 3H), 0.86 (d, *J* = 6.5 Hz, 3H). ¹³C NMR (126 MHz, DMSO-*d₆*) *δ* ppm: 167.9, 166.3, 155.6, 145.9, 138.7, 131.0, 128.6 (2C), 122.6, 119.5 (2C), 77.9, 52.3, 50.0, 38.0, 35.7, 28.3 (3C), 25.7, 22.4, 22.3. HRMS-ESI+ (m/z): calcd. for C₂₂H₃₃N₆O₅ [M + H]⁺ 461.2512, found 461.2480.

### 2-(Hydroxycarbamoyl)-N-[4-[[4-[(4-iodoanilino)methyl]triazol-1-yl]methyl] phenyl]-4-methyl-pentanamide (134)

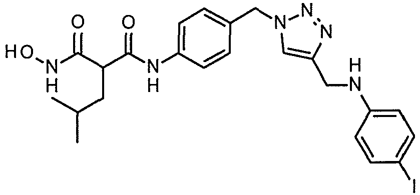

Compound **134** was synthesized according to the general procedure D, using ester **126** (158 mg, 0.27 mmol), KCN (5.31 mg, 0.08 mmol) and NH₂OH (2.5 mL, 50% w/w in water) in MeOH (2.5 mL) for 72 h. The crude product was purified by flash chromatography on silica gel (CH₂Cl₂ to CH₂Cl₂/MeOH: 9/1) affording compound **134** as a white solid after lyophilization (31 mg, 20%). Purity: 100%, LC tr = 4.13 min, MS (ESI+): m/z = 563 [M + H]⁺. ¹H NMR (500 MHz, DMSO-*d₆*) *δ* ppm: 10.52 (s, 1H), 9.80 (s, 1H), 8.97 (s, 1H), 7.94 (s, 1H), 7.56-7.54 (m, 2H), 7.33-7.31 (m, 2H), 7.26-7.24 (m, 2H), 6.49-6.47 (m, 2H), 6.27 (t, *J* = 5.8 Hz, 1H), 5.48 (s, 2H), 4.24 (d, *J* = 5.8 Hz, 2H), 3.20 (t, *J* = 7.6 Hz, 1H), 1.67 (t, *J* = 7.2 Hz, 2H), 1.47 (sep, *J* = 6.7 Hz, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.8, 166.1, 148.1, 145.5, 138.6, 137.1 (2C), 131.0, 128.6 (2C), 122.7, 119.6 (2C), 115.0 (2C), 76.5, 52.4, 49.9, 38.3, 38.0, 25.7, 22.4, 22.3. HRMS-ESI+ (m/z): calcd. for C₂₃H₂₈IN₆O₃ [M + H]⁺ 563.1268, found 563.1232.

### N-[4-[[4-[(4-Chlorophenoxy)methyl]triazol-1-yl]methyl]phenyl]-2-(hydroxycarbamoyl)-4-methyl-pentanamide (135)

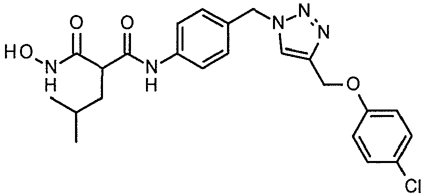

Compound **135** was synthesized according to the general procedure D, using ester **127** (50 mg, 0.1 mmol), KCN (2.0 mg, 0.03 mmol) and NH₂OH (1.5 mL, 50% w/w in water) in MeOH (1.5 mL) for 72 h. The crude product was purified by preparative HPLC (H₂O+0.05%FA / ACN+0.05%FA: 95:5 to 5:95) affording compound **135** as a white solid after lyophilization (21 mg, 43%). Purity: 100%, LC tr = 4.13 min, MS (ESI+): m/z = 472 and 474 [M + H]⁺. ¹H NMR (500 MHz, DMSO-*d₆*) δ ppm: 10.53 (s, 1H), 9.83 (s, 1H), 8.97 (s, 1H), 8.23 (s, 1H), 7.58-7.56 (m, 2H), 7.33-7.32 (m, 2H), 7.29-7.27 (m, 2H), 7.06-7.04 (m, 2H), 5.53 (s, 2H), 5.11 (s, 2H), 3.21 (t, *J* = 7.6 Hz, 1H), 1.69-1.67 (m, 2H), 1.47 (sep, *J* = 6.7 Hz, 1H), 0.87 (d, *J* = 6.6 Hz, 3H), 0.86 (d, *J* = 6.6 Hz, 3H). ¹³C NMR (126 MHz, DMSO-*d₆*) δ ppm: 167.8, 166.3, 156.9, 142.7, 138.7, 130.8, 129.2 (2C), 128.7 (2C), 124.6, 124.6, 119.6 (2C), 116.5 (2C), 61.4, 52.5, 49.9, 38.0, 25.7, 22.4, 22.3. HRMS-ESI+ (m/z): calcd. for C₂₃H₂₇ClN₅O₄ [M + H]⁺ 472.1752, found 472.1718.

### N-[4-[[4-[(4-Chlorophenyl)sulfanylmethyl]triazol-1-yl]methyl]phenyl]-2-(hydroxycarbamoyl)-4-methyl-pentanamide (136)

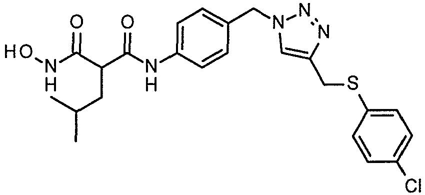

Compound **136** was synthesized according to the general procedure D, using ester **128** (70 mg, 0.14 mmol), KCN (2.7 mg, 0.04 mmol) and NH₂OH (1.5 mL, 50% w/w in water) in MeOH (1.5 mL) for 72 h. The crude product was purified by preparative HPLC (H₂O+0.05%FA / ACN+0.05%FA: 95:5 to 5:95) affording compound **136** as a white solid after lyophilization (11 mg, 15%). Purity: 95%, LC tr = 4.13 min, MS (ESI+): m/z = 488 and 490 [M + H]⁺. ¹H NMR (500 MHz, DMSO-*d₆*) *δ* ppm: 10.55 (s, 1H), 9.83 (s, 1H), 8.97 (br s, 1H), 7.93 (s, 1H), 7.56-7.54 (m, 2H), 7.36-7.32 (m, 4H), 7.19-7.18 (m, 2H), 5.46 (s, 2H), 4.27 (s, 2H), 3.21 (t, *J* = 7.6 Hz, 1H), 1.69-1.66 (m, 2H), 1.47 (sep, *J* = 6.6 Hz, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.8, 166.3, 143.5, 138.7, 134.7, 130.8, 130.6, 130.2 (2C), 128.8 (2C), 128.4 (2C), 123.3, 119.5 (2C), 52.4, 49.9, 38.0, 27.3, 25.7, 22.4, 22.3. HRMS-ESI+ (m/z): calcd. for C₂₃H₂₇ClN₅O₃S [M + H]⁺ 488.1523, found 488.1490.

### N-[4-[[4-[(4-Chlorophenyl)methyl]triazol-1-yl]methyl]phenyl]-2-(hydroxycarbamoyl)-4-methyl-pentanamide (137)

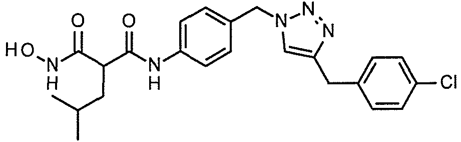

Compound **137** was synthesized according to the general procedure D, using ester **129** (75 mg, 0.16 mmol), KCN (3.1 mg, 0.05 mmol) and NH₂OH (1.5 mL, 50% w/w in water) in MeOH (1.5 mL) for 72 h. The crude product was purified by preparative HPLC (H₂O+0.05%FA / ACN+0.05%FA: 95:5 to 5:95) affording compound **137** as a white solid after lyophilization (11 mg, 15%). Purity: 100%, LC tr = 4.04 min, MS (ESI+): m/z = 456 and 458 [M + H]⁺. ¹H NMR (500 MHz, DMSO-*d₆*) *δ* ppm: 10.53 (br s, 1H), 9.81 (s, 1H), 8.97 (br s, 1H), 7.84 (s, 1H), 7.56-7.54 (m, 2H), 7.34-7.32 (m, 2H), 7.26-7.24 (m, 4H), 5.46 (s, 2H), 3.96 (s, 2H), 3.20 (t, *J* = 7.6 Hz, 1H), 1.67 (t, *J* = 7.0 Hz, 2H), 1.47 (sep, *J* = 6.7 Hz, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.8, 166.3, 146.0, 138.7, 138.6, 130.9, 130.8, 130.4 (2C), 128.6 (2C), 128.3 (2C), 122.5, 119.5 (2C), 52.4, 49.9, 38.0, 30.5, 25.7, 22.4, 22.3. HRMS-ESI+ (m/z): calcd. for C₂₃H₂₇ClN₅O₃ [M + H]⁺ 456,1802, found 456.1773.

### 2-(Hydroxycarbamoyl)-N-[4-[[4-(4-iodophenyl)triazol-1-yl]methyl]phenyl]-4-methyl-pentanamide (138)

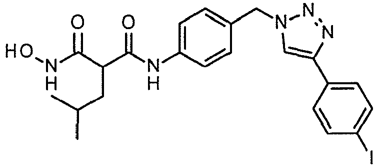

Compound **138** was synthesized according to the general procedure D, using ester **130** (140 mg, 0.25 mmol), KCN (4.96 mg, 0.08 mmol) and NH₂OH (2.5 mL, 50% w/w in water) in MeOH (2.5 mL) for 72 h. The crude product was purified by flash chromatography on silica gel (CH₂Cl₂ to CH₂Cl₂/MeOH: 95/5) affording compound **138** as a white solid after lyophilization (28 mg, 20%). Purity: 100%, LC tr = 4.31 min, MS (ESI+): m/z = 534 [M + H]⁺. ¹H NMR (500 MHz, DMSO-*d₆*) *δ* ppm: 10.52 (br s, 1H), 9.83 (s, 1H), 8.98 (br s, 1H), 8.63 (s, 1H), 7.81-7.79 (m, 2H), 7.67-7.59 (m, 4H), 7.33-7.32 (m, 2H), 5.58 (s, 2H), 3.20 (t, *J* = 7.3 Hz, 1H), 1.67 (t, *J* = 6.9 Hz, 2H), 1.51-1.43 (m, 1H), 0.88-0.85 (m, 6H). ¹³C NMR (126 MHz, DMSO-*d₆*) *δ* ppm: 167.9, 166.3, 145.7, 138.8, 137.7 (2C), 130.6, 130.3, 128.7 (2C), 127.2 (2C), 121.7, 119.6 (2C), 93.8, 52.7, 50.0, 38.0, 25.7, 22.4, 22.3. HRMS-ESI+ (m/z): calcd. for C₂₂H₂₅IN₅O₃ [M + H]⁺ 534.1002, found 534.0970.

### 2-(Hydroxycarbamoyl)-N-[3-[[4-[[(4-iodophenyl)sulfonylamino]methyl]triazol-1-yl]methyl]phenyl]-4-methyl-pentanamide (114)

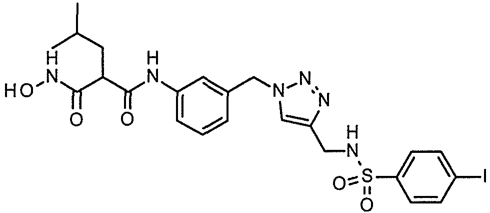

Compound **114** was synthesized according to the general procedure D, using ester **113** (206 mg, 0.32 mmol), KCN (4.15 mg, 0.06 mmol) and NH₂OH (3.5 mL, 50% w/w in water) in MeOH (3.5 mL) for 96 h. The crude product was purified by flash chromatography on silica gel (CH₂Cl₂ to CH₂Cl₂/MeOH: 95/5) affording compound **114** as a white solid after lyophilization (13 mg, 6%). Purity: 95%, LC tr = 3.84 min, MS (ES+): m/z= 627 [M + H]⁺. ¹H NMR (500 MHz, DMSO-*d₆*) *δ* ppm: 10.52 (br s, 1H), 9.83 (s, 1H), 8.96 (s, 1H), 8.21 (br s, 1H), 7.94-7.92 (m, 2H), 7.89 (s, 1H), 7.56-7.51 (m, 4H), 7.32-7.29 (m, 1H), 6.96-6.95 (m, 1H), 5.49 (s, 2H), 4.04 (s, 2H), 3.21 (t, *J* = 7.5 Hz, 1H), 1.70-1.64 (m, 2H), 1.47 (sep, *J* = 6.6 Hz, 1H), 0.87 (d, *J* = 6.4 Hz, 3H), 0.86 (d, J = 6.4 Hz, 3H). ¹³C NMR (126 MHz, DMSO-*d₆*) *δ* ppm: 167.8, 166.3, 143.5, 140.0, 139.1, 138.0 (2C), 136.4, 129.2, 128.3 (2C), 123.4, 123.0, 119.1, 118.9, 100.4, 52.7, 49.9, 38.0, 37.9, 25.7, 22.4, 22.3. HRMS-ESI+ (m/z): calcd. for C₂₃H₂₈IN₆O₅S [M + H]⁺ 627.0887, found 627.0879.

### N-[4-[[4-[[(4-Fluorophenyl)sulfonylamino]methyl]triazol-1-yl]methyl]phenyl]-2-(hydroxycarbamoyl)-4-methyl-pentanamide (160)

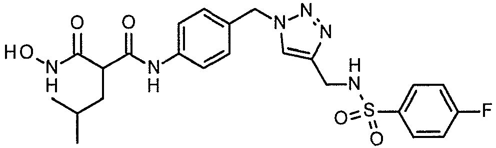

Compound **160** was synthesized according to the general procedure D, using ester **150** (127 mg, 0.23 mmol), KCN (4.62 mg, 0.07 mmol) and NH₂OH (3 mL, 50% w/w in water) in MeOH (3 mL) for 72 h. The crude product was purified by flash chromatography on silica gel (CH₂Cl₂ to CH₂Cl₂/MeOH: 95/5) affording compound **160** as a white solid after lyophilization (96 mg, 78%). Purity: 100%, LC tr = 3.46 min, MS (ESI+): m/z = 519 [M + H]⁺. ¹H NMR (500 MHz, DMSO-*d₆*) *δ* ppm: 10.51 (br s, 1H), 9.83 (s, 1H), 8.97 (br s, 1H), 8.19 (br s, 1H), 7.84 (s, 1H), 7.82-7.79 (m, 2H), 7.57-7.56 (m, 2H), 7.38-7.34 (m, 2H), 7.23-7.22 (m, 2H), 5.45 (s, 2H), 4.04 (s, 2H), 3.21 (t, *J* = 7.5 Hz, 1H), 1.68 (t, *J* = 7.2 Hz, 2H), 1.47 (sep, *J* = 6.6 Hz, 1H), 0.87 (d, *J* = 6.3 Hz, 3H), 0.86 (d, J = 6.3 Hz, 3H). ¹³C NMR (126 MHz, DMSO-*d₆*) *δ* ppm: 167.8, 166.3, 164.1 (d, *J* = 250.8 Hz), 143.5, 138.7, 136.8 (d, *J* = 2.8 Hz), 130.7, 129.6 (d, *J* = 9.2 Hz, 2C), 128.6 (2C), 123.2, 119.6 (2C), 116.2 (d, *J* = 22.8 Hz, 2C), 52.4, 49.9, 38.1, 38.0, 25.7, 22.4, 22.3. HRMS-ESI+ (m/z): calcd. for C₂₃H₂₈FN₆O₅S [M + H]⁺: 519.1826, found 519.1819.

### N-[4-[[4-[[(4-Chlorophenyl)sulfonylamino]methyl]triazol-1-yl]methyl]phenyl]-2-(hydroxycarbamoyl)-4-methyl-pentanamide (161)

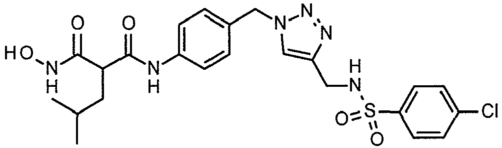

Compound **161** was synthesized according to the general procedure D, using ester **151** (128 mg, 0.23 mmol), KCN (4.52 mg, 0.07 mmol) and NH₂OH (2.8 mL, 50% w/w in water) in MeOH (2.8 mL) for 72 h. The crude product was purified by flash chromatography on silica gel (CH₂Cl₂ to CH₂Cl₂/MeOH: 9/1) affording compound **161** as a white solid after lyophilization (59 mg, 47%). Purity: 100%, LC tr = 3.64 min, MS (ESI+): m/z = 535 and 537 [M + H]⁺. ¹H NMR (500 MHz, DMSO-*d₆*) *δ* ppm: 10.53 (s, 1H), 9.81 (s, 1H), 8.98 (s, 1H), 8.22 (t, *J* = 6.0 Hz, 1H), 7.85 (s, 1H), 7.75-7.73 (m, 2H), 7.60-7.56 (m, 4H), 7.24-7.22 (m, 2H), 5.44 (s, 2H), 4.05 (d, *J* = 3.6 Hz, 2H), 3.21 (t, *J* = 7.5 Hz, 1H), 1.68 (t, *J* = 6.9 Hz, 2H), 1.47 (sep, *J* = 6.6 Hz, 1H), 0.88-0.86 (m, 6H). ¹³C NMR (126 MHz, DMSO-*d₆*) δ ppm: 169.7, 167.1, 143.4, 139.3, 138.7, 137.2, 130.9, 129.2 (2C), 128.8 (2C), 128.5 (2C), 123.2, 119.4 (2C), 52.3, 50.9, 38.0, 37.4, 25.6, 22.6, 21.9. HRMS-ESI+ (m/z): calcd. for C₂₃H₂₈ClN₆O₅S [M + H]⁺ 535.1530, found 535.1526.

### 2-(Hydroxycarbamoyl)-N-[4-[[4-[[(4-methoxyphenyl)sulfonylamino]methyl] triazol-1-yl]methyl]phenyl]-4-methyl-pentanamide (162)

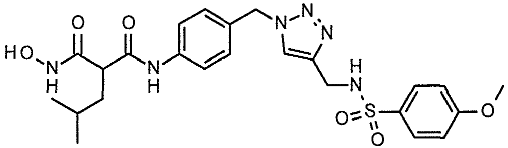

Compound **162** was synthesized according to the general procedure D, using ester **152** (131 mg, 0.24 mmol), KCN (4.66 mg, 0.07 mmol) and NH₂OH (3 mL, 50% w/w in water) in MeOH (3 mL) for 72 h. The crude product was purified by flash chromatography on silica gel (CH₂Cl₂ to CH₂Cl₂/MeOH: 9/1) affording compound **162** as a white solid after lyophilization (34 mg, 26%). Purity: 100%, LC tr = 3.43 min, MS (ESI+): m/z = 531 [M + H]⁺. Purity: 100%, LC tr = 3.43 min, MS (ESI+): m/z = 531 [M + H]⁺. ¹H NMR (500 MHz, DMSO-*d₆*) *δ* ppm: 10.53 (br s, 1H), 9.83 (s, 1H), 8.97 (br s, 1H), 7.92 (br s, 1H), 7.84 (s, 1H), 7.71-7.69 (m, 2H), 7.57-7.56 (m, 2H), 7.24-7.22 (m, 2H), 7.07-7.05 (m, 2H), 5.45 (s, 2H), 3.98 (s, 2H), 3.82 (s, 3H), 3.21 (t, *J* = 7.6 Hz, 1H), 1.68 (t, *J* = 7.2 Hz, 2H), 1.47 (sep, *J* = 6.6 Hz, 1H), 0.88-0.86 (m, 6H). ¹³C NMR (126 MHz, DMSO-*d₆*) *δ* ppm: 167.8, 166.3, 162.1, 143.7, 138.7, 131.9, 130.8, 128.7 (2C), 128.6 (2C), 123.2, 119.5 (2C), 114.3 (2C), 55.6, 52.4, 49.9, 38.1, 38.0, 25.7, 22.4, 22.3. HRMS-ESI+ (m/z): calcd. for C₂₄H₃₁N₆O₆S [M + H]⁺: 531.2026, found 531.1997.

### N-[4-[[4-[[(2-Chlorophenyl)sulfonylamino]methyl]triazol-1-yl]methyl]phenyl]-2-(hydroxycarbamoyl)-4-methyl-pentanamide (163)

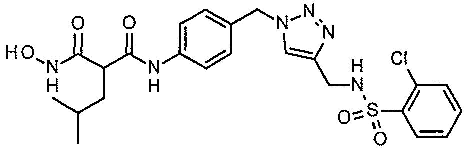

Compound **163** was synthesized according to the general procedure D, using ester **153** (160 mg, 0.29 mmol), KCN (5.65 mg, 0.09 mmol) and NH₂OH (3 mL, 50% w/w in water) in MeOH (3 mL) for 72 h. The crude product was purified by flash chromatography on silica gel (CH₂Cl₂ to CH₂Cl₂/MeOH: 9/1) affording compound **163** as a white solid after lyophilization (12 mg, 7%). Purity: 100%, LC tr = 3.43 min, MS (ESI+): m/z = 535 and 537 [M + H]⁺. ¹H NMR (DMSO-*d₆*) *δ* ppm: 10.55 (br s, 1H), 9.84 (s, 1H), 8.99 (br s, 1H), 8.37 (br s, 1H), 7.90-7.89 (m, 1H), 7.74 (s, 1H), 7.59-7.57 (m, 2H), 7.55-7.49 (m, 2H), 7.45-7.42 (m, 1H), 7.22-7.21 (m, 2H), 5.42 (s, 2H), 4.16 (s, 2H), 3.22 (t, *J* = 7.6 Hz, 1H), 1.69 (t, *J* = 7.0 Hz, 2H), 1.49 (sep, *J* = 6.6 Hz, 1H), 0.89 (d, *J* = 6.4 Hz, 3H), 0.88 (d, *J* = 6.4 Hz, 3H). ¹³C NMR (126 MHz, DMSO-*d₆*) *δ* ppm: 167.9, 166.3, 143.7, 138.7, 138.0, 133.9, 131.6, 130.8, 130.6, 130.4, 128.7 (2C), 127.5, 123.1, 119.6 (2C), 52.3, 50.0, 38.0, 37.9, 25.8, 22.5, 22.3. HRMS-ESI+ (m/z): calcd. for C₂₃H₂₈ClN₆O₅S [M+H]⁺ 535,1530, found 535.1500.

### N-[4-[[4-[[(3-Chlorophenyl)sulfonylamino]methyl]triazol-1-yl]methyl]phenyl]-2-(hydroxycarbamoyl)-4-methyl-pentanamide (164)

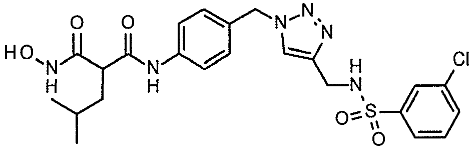

Compound **164** was synthesized according to the general procedure D, using ester **154** (125 mg, 0.23 mmol), KCN (4.41 mg, 0.07 mmol) and NH₂OH (2.5 mL, 50% w/w in water) in MeOH (2.5 mL) for 72 h. The crude product was purified by flash chromatography on silica gel (CH₂Cl₂ to CH₂Cl₂/MeOH: 9/1) affording compound **164** as a white solid after lyophilization (67 mg, 55%). Purity: 100%, LC tr = 3.66 min, MS (ESI+): m/z = 535 and 537 [M + H]⁺. ¹H NMR (500 MHz, DMSO-*d₆*) *δ* ppm: 10.53 (br s, 1H), 9.82 (s, 1H), 8.98 (s, 1H), 8.29 (br s, 1H), 7.84 (s, 1H), 7.75-7.74 (m, 1H), 7.70-7.66 (m, 2H), 7.58-7.55 (m, 3H), 7.24-7.22 (m, 2H), 5.45 (s, 2H), 4.09 (s, 2H), 3.21 (t, *J* = 7.6 Hz, 1H), 1.71-1.67 (m, 2H), 1.48 (sep, *J* = 6.7 Hz, 1H), 0.88 (d, *J* = 6.6 Hz, 3H), 0.87 (d, *J* = 6.6 Hz, 3H). ¹³C NMR (126 MHz, DMSO-*d₆*) *δ* ppm: 167.9, 166.3, 143.3, 142.4, 138.8, 133.7, 132.4, 131.2, 130.7, 128.7 (2C), 126.2, 125.2, 123.2, 119.6 (2C), 52.4, 50.0, 38.1, 38.0, 25.8, 22.4, 22.3. HRMS-ESI+ (m/z): calcd. for C₂₃H₂₈ClN₆O₅S [M + H]⁺ 535,1530, found 535.1496.

### N-[4-[[4-[[(3,4-Dichlorophenyl)sulfonylamino]methyl]triazol-1-yl]methyl]phenyl]-2-(hydroxycarbamoyl)-4-methyl-pentanamide (165)

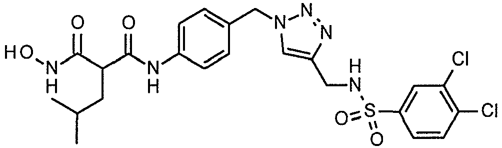

Compound **165** was synthesized according to the general procedure D, using ester **155** (108 mg, 0.18 mmol), KCN (3.59 mg, 0.06 mmol) and NH₂OH (1.8 mL, 50% w/w in water) in MeOH (1.8 mL) for 72 h. The crude product was purified by flash chromatography on silica gel (CH₂Cl₂ to CH₂Cl₂/MeOH: 95/5) affording compound **165** as a white solid after lyophilization (19 mg, 18%). Purity: 95%, LC tr = 3.94 min, MS (ESI-): m/z= 569, 571 and 573 [M + H]⁺. ¹H NMR (500 MHz, DMSO-*d₆*) δ ppm: 10.54 (br s, 1H), 9.82 (s, 1H), 8.97 (s, 1H), 8.35 (br s, 1H), 7.91-7.90 (m, 1H), 7.88 (s, 1H), 7.79-7.78 (m, 1H), 7.68-7.67 (m, 1H), 7.58-7.56 (m, 2H), 7.24-7.22 (m, 2H), 5.44 (s, 2H), 4.10 (s, 2H), 3.21 (t, *J* = 7.6 Hz, 1H), 1.69-1.66 (m, 2H), 1.47 (sep, *J* = 6.7 Hz, 1H), 0.87 (d, *J* = 6.6 Hz, 3H), 0.86 (d, *J* = 6.6 Hz, 3H). ¹³C NMR (126 MHz, DMSO-*d₆*) δ ppm: 167.8, 166.3, 143.2, 140.9, 138.7, 135.4, 131.9, 131.5, 130.7, 128.6 (2C), 128.4, 126.7, 123.3, 119.9 (2C), 52.4, 50.0, 38.0, 38.0, 25.7, 22.4, 22.3. HRMS-ESI+ (m/z): calcd. for C₂₃H₂₇Cl₂N₆O₅S [M + H]⁺ 569.1141, found 569.1099.

### N-[4-[[4-[[(4-Chlorophenyl)methylsulfonylamino]methyl]triazol-1-yl]methyl] phenyl]-2-(hydroxycarbamoyl)-4-methyl-pentanamide (166)

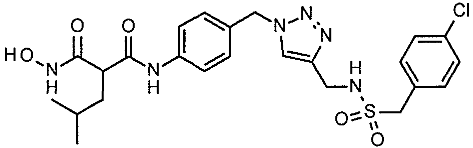

Compound **166** was synthesized according to the general procedure D, using ester **156** (58 mg, 0.1 mmol), KCN (2 mg, 0.03 mmol) and NH₂OH (1.2 mL, 50% w/w in water) in MeOH (1.2 mL) for 72 h. The crude product was purified by flash chromatography on silica gel (CH₂Cl₂ to CH₂Cl₂/MeOH: 9/1) affording compound **166** as a white solid after lyophilization (13 mg, 23%). Purity: 100%, LC tr = 3.74 min, MS (ESI+): m/z = 549 and 551 [M + H]⁺. ¹H NMR (500 MHz, DMSO-*d₆*) *δ* ppm: 10.52 (s, 1H), 9.81 (s, 1H), 8.97 (s, 1H), 7.97 (s, 1H), 7.64 (t, *J* = 5.9 Hz, 1H), 7.58-7.56 (m, 2H), 7.38-7.37 (m, 2H), 7.30-7.28 (m, 4H), 5.52 (s, 2H), 4.34 (s, 2H), 4.17 (d, *J* = 5.8 Hz, 2H), 3.20 (t, *J* = 7.6 Hz, 1H), 1.67 (t, *J* = 6.2 Hz, 2H), 1.47 (sep, *J* = 6.6 Hz, 1H), 0.87 (d, *J* = 6.4 Hz, 3H), 0.86 (d, *J* = 6.4 Hz, 3H). ¹³C NMR (126 MHz, DMSO-*d₆*) *δ* ppm: 167.8, 166.3, 148.7, 138.7, 132.9, 132.5 (2C), 130.9, 129.3, 128.7 (2C), 128.3 (2C), 123.2, 119.5 (2C), 56.7, 52.4, 49.9, 38.0, 38.0, 25.7, 22.4, 22.2. HRMS-ESI+ (m/z): calcd. for C₂₄H₃₀ClN₆O₅S [M + H]⁺ 549.1687, found 549.1653.

### N-[4-[[4-(Benzenesulfonamidomethyl)triazol-1-yl]methyl]phenyl]-2-(hydroxycarbamoyl)-4-methyl-pentanamide (167)

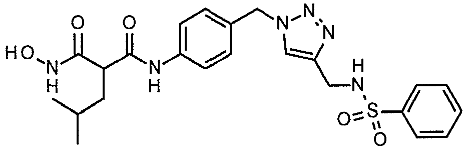

Compound **167** was synthesized according to the general procedure D, using ester **157** (113 mg, 0.22 mmol), KCN (4.25 mg, 0.07 mmol) and NH₂OH (2.5 mL, 50% w/w in water) in MeOH (2.5 mL) for 72h. The crude product was purified by flash chromatography on silica gel (CH₂Cl₂ to CH₂Cl₂/MeOH: 9/1) affording compound **167** as a white solid after lyophilization (19 mg, 17%). Purity: 100%, LC tr = 3.36 min, MS (ESI+): m/z = 501 [M + H]⁺. ¹H NMR (500 MHz, DMSO-*d₆*) *δ* ppm: 10.50 (br s, 1H), 9.83 (s, 1H), 8.96 (br s, 1H), 8.13 (br s, 1H), 7.80 (s, 1H), 7.76-7.75 (m, 2H), 7.61-7.51 (m, 5H), 7.23-7.21 (m, 2H), 5.44 (s, 2H), 4.03 (s, 2H), 3.21 (t, *J* = 7.6 Hz, 1H), 1.68 (t, *J* = 7.2 Hz, 2H), 1.47 (sep, *J* = 6.6 Hz, 1H), 0.88 (d, *J* = 6.5 Hz, 3H), 0.87 (d, *J* = *6.5* Hz, 3H). ¹³C NMR (126 MHz, DMSO-*d₆*) *δ* ppm: 167.9, 166.3, 143.6, 140.4, 138.7, 132.4, 130.7, 129.1 (2C), 128.7 (2C), 126.5 (2C), 123.1, 119.6 (2C), 52.4, 50.0, 38.1, 38.0, 25.7, 22.4, 22.3. HRMS-ESI+ (m/z): calcd. for C₂₃H₂₉N₆O₅S [M + H]⁺ 501.1920, found 501.1889.

### N-[4-[[4-[(Cyclohexylsulfonylamino)methyl]triazol-1-yl]methyl]phenyl]-2-(hydroxycarbamoyl)-4-methyl-pentanamide (168)

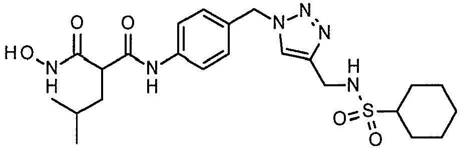

Compound **168** was synthesized according to the general procedure D, using ester **158** (50 mg, 0.1 mmol), KCN (1.9 mg, 0.03 mmol) and NH₂OH (1.2 mL, 50% w/w in water) in MeOH (1.2 mL) for 48 h. The crude product was purified by flash chromatography on silica gel (CH₂Cl₂ to CH₂Cl₂/MeOH: 9/1) affording compound **168** as a white solid after lyophilization (7 mg, 14%). Purity: 100%, LC tr = 3.36 min, MS (ESI+): m/z = 501 [M + H]⁺. ¹H NMR (500 MHz, DMSO-*d₆*) δ ppm: 10.53 (br s, 1H), 9.83 (s, 1H), 8.96 (br s, 1H), 7.99 (s, 1H), 7.58-7.56 (m, 2H), 7.50 (t, *J* = 5.8 Hz, 1H), 7.28-7.27 (m, 2H), 5.52 (s, 2H), 4.18 (d, *J* = 5.8 Hz, 2H), 3.20 (t, *J* = 7.6 Hz, 1H), 2.75-2.70 (m, 1H), 1.90-1.88 (m, 2H), 1.69-1.66 (m, 4H), 1.55-1.54 (m, 1H), 1.47 (sep, *J* = 6.6 Hz, 1H), 1.27-1.20 (m, 2H), 1.07-1.03 (m, 3H), 0.87-0.86 (m, 6H). ¹³C NMR (126 MHz, DMSO-*d₆*) δ ppm: 167.8, 166.2, 144.9, 138.7, 130.9, 128.6 (2C), 123.1, 119.5 (2C), 59.4, 52.4, 49.9, 38.0, 37.7, 25.9 (2C), 25.7, 24.8, 24.5 (2C), 22.4, 22.3. HRMS-ESI+ (m/z): calcd. for C₂₃H₃₅N₆O₅S [M + H]⁺ 507.2390, found 507.2356.

### 2-(Hydroxycarbamoyl)-N-[4-[[4-[(isobutylsulfonylamino)methyl]triazol-1-yl]methyl]phenyl]-4-methyl-pentanamide (169)

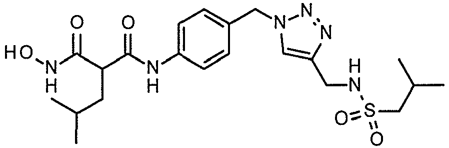

Compound **169** was synthesized according to the general procedure D, using ester **159** (75 mg, 0.15 mmol), KCN (2.94 mg, 0.05 mmol) and NH₂OH (1.5 mL, 50% w/w in water) in MeOH (1.5 mL) overnight. The crude product was purified by flash chromatography on silica gel (CH₂Cl₂ to CH₂Cl₂/MeOH: 95/5) affording compound **169** as a white solid after lyophilization (35 mg, 48%). Purity: 100%, LC tr = 3.33 min, MS (ESI+): m/z= 481 [M + H]⁺. ¹H NMR (500 MHz, DMSO-*d₆*) *δ* ppm: 10.52 (s, 1H), 9.80 (s, 1H), 8.97 (s, 1H), 8.00 (s, 1H), 7.57-7.55 (m, 2H), 7.53 (t, *J* = 6.0 Hz, 1H), 7.28-7.26 (m, 2H), 5.52 (s, 2H), 4.18 (d, *J* = 6.0 Hz, 2H), 3.20 (t, *J* = 7.6 Hz, 1H), 2.82 (d, *J* = 6.4 Hz, 2H), 1.97 (sep, *J* = 6.7 Hz, 1H), 1.68-1.66 (m, 2H), 1.47 (sep, *J* = 6.7 Hz, 1H), 0.92 (d, *J* = 6.6 Hz, 6H), 0.87 (d, *J* = 6.6 Hz, 3H), 0.86 (d, *J* = 6.6 Hz, 3H). ¹³C NMR (126 MHz, DMSO-*d₆*) *δ* ppm: 167.9, 166.3, 144.6, 138.7, 130.9, 128.6 (2C), 123.2, 119.5 (2C), 59.2, 52.4, 50.0, 38.0, 37.6, 25.7, 24.2, 22.4, 22.3, 22.2 (2C). HRMS-ESI+ (m/z): calcd. for C₂₁H₃₃N₆O₅S [M + H]⁺ 481.2233, found 481.2202.

### 2-Benzyl-3-(hydroxyamino)-N-[4-[[4-[[(4-iodophenyl)sulfonylamino]methyl] triazol-1-yl]methyl]phenyl]-3-oxo-propanamide (179)

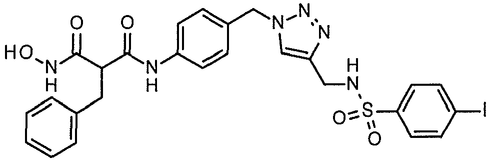

Compound **179** was synthesized according to the general procedure D, using ester **177** (85 mg, 0.13 mmol), KCN (2.44 mg, 0.04 mmol) and NH₂OH (1.8 mL, 50% w/w in water) in MeOH (1.8 mL) for 96 h. The crude product was purified by flash chromatography on silica gel (CH₂Cl₂ to CH₂Cl₂/MeOH: 9/1) affording compound **179** as a white solid after lyophilization (67 mg, 81%). Purity: 100%, LC tr = 3.85 min, MS (ESI+): m/z = 661 [M + H]⁺. ¹H NMR (500 MHz, DMSO-*d₆*) *δ* ppm: 10.50 (br s, 1H), 9.86 (s, 1H), 8.97 (br s, 1H), 8.17 (br s, 1H), 7.93-7.91 (m, 2H), 7.83 (s, 1H), 7.55-7.50 (m, 4H), 7.27-7.23 (m, 6H), 7.18-7.15 (m, 1H), 5.44 (s, 2H), 4.03 (s, 2H), 3.46 (t, *J* = 7.5 Hz, 1H), 3.16-3.06 (m, 2H). ¹³C NMR (126 MHz, DMSO-*d₆*) *δ* ppm: 167.0, 165.4, 143.4, 140.0, 138.9, 138.7, 138.0 (2C), 130.7, 128.8 (2C), 128.7 (2C), 128.3 (2C), 128.2 (2C), 126.3, 123.2, 119.5 (2C), 100.4, 53.1, 52.4, 38.0, 34.4. HRMS-ESI+ (m/z): calcd. for C₂₆H₂₆IN₆O₅S [M + H]⁺ 661.0730, found 661.0683.

### 2-(Hydroxycarbamoyl)-N-[4-[[4-[[(4-iodophenyl)sulfonylamino]methyl]triazol-1-yl]methyl]phenyl]-3-methyl-butanamide (182)

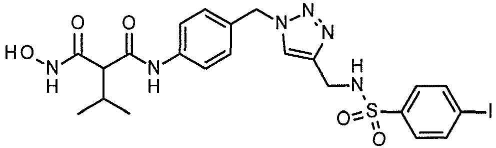

Compound **182** was synthesized according to the general procedure E, using azide **180** (35 mg, 0.12 mmol), alkyne **93** (38.6 mg, 0.12 mmol), copper (II) sulfate pentahydrate (6.0 mg, 0.02 mmol) and sodium ascorbate (11.9 mg, 0.06 mmol) in DMF (2.5 mL) and H₂O (1.5 mL) overnight. The crude product was purified by flash chromatography on silica gel (CH₂Cl₂ to CH₂Cl₂/MeOH: 95/5) affording compound **182** as a white solid after lyophilization (41 mg, 55%). Purity: 100%, LC tr = 3.51 min, MS (ESI+): m/z = 613 [M + H]⁺. ¹H NMR (500 MHz, DMSO-*d₆*) *δ* ppm: 10.56 (s, 1H), 9.77 (s, 1H), 9.02 (s, 1H), 8.19 (t, *J* = 5.8 Hz, 1H), 7.93-7.91 (m, 2H), 7.84 (s, 1H), 7.59-7.57 (m, 2H), 7.51-7.49 (m, 2H), 7.25-7.23 (m, 2H), 5.44 (s, 2H), 4.03 (d, *J* = 5.7 Hz, 2H), 2.71 (d, *J* = 10.5 Hz, 1H), 2.39-2.32 (m, 1H), 0.89-0.87 (m, 6H). HRMS-ESI+ (m/z): calcd. for C₂₂H₂₆IN₆O₅S [M + H]⁺ 613.0730, found 613.0689.

### 2-(Cyclopropylmethyl)-3-(hydroxyamino)-N-[4-[[4-[[(4-iodophenyl) sulfonylamino]methyl]triazol-1-yl]methyl]phenyl]-3-oxo-propanamide (183)

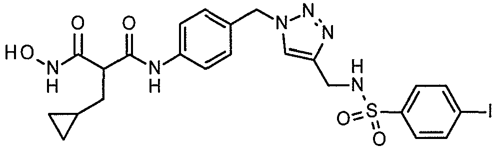

Compound **183** was synthesized according to the general procedure E, using azide **181** (50 mg, 0.17 mmol), alkyne **93** (52.9 mg, 0.12 mmol), copper (II) sulfate pentahydrate (8.2 mg, 0.03 mmol) and sodium ascorbate (16.3 mg, 0.08 mmol) in DMF (3 mL) and H₂O (2 mL) overnight. The crude product was purified by flash chromatography on silica gel (CH₂Cl₂ to CH₂Cl₂/MeOH: 95/5) affording compound 183 as a white solid after lyophilization (49 mg, 47%). Purity: 100%, LC tr = 3.64 min, MS (ESI+): m/z = 625 [M + H]⁺. ¹H NMR (500 MHz, DMSO-*d₆*) *δ* ppm: 10.51 (s, 1H), 9.85 (s, 1H), 8.96 (s, 1H), 8.20-8.17 (m, 1H), 7.93-7.91 (m, 2H), 7.84 (s, 1H), 7.58-7.50 (m, 4H), 7.25-7.23 (m, 2H), 5.44 (s, 2H), 4.03-4.02 (m, 2H), 3.21 (t, *J* = 6.9 Hz, 1H), 1.69 (t, *J* = 6.7 Hz, 2H), 0.65-0.62 (m, 1H), 0.37-0.35 (m, 2H), 0.08-0.07 (m, 2H). ¹³C NMR (126 MHz, DMSO-*d₆*) *δ* ppm: 167.8, 166.2, 143.4, 140.0, 138.8, 138.0 (2C), 130.7, 128.7 (2C), 128.3 (2C), 123.2, 119.5 (2C), 100.4, 52.4, 52.0, 38.0, 34.1, 9.0, 4.3, 4.3. HRMS-ESI+ (m/z): calcd. for C₂₃H₂₆IN₆O₅S [M + H]⁺ 625.0730, found 625.0691.

### 2-(Hydroxycarbamoyl)-4-methyl-N-[4-[[4-[(1,1,3-trioxo-1,2-benzothiazol-2-yl)methyl]triazol-1-yl]methyl]phenyl]pentanamide (139)

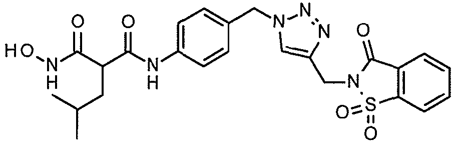

Compound **139** was synthesized according to the general procedure E, using azide **Z9** (50 mg, 0.26 mmol), alkyne **117** (145 mg, 0.66 mmol), copper (II) sulfate pentahydrate (8.2 mg, 0.03 mmol) and sodium ascorbate (51.9 mg, 0.26 mmol) in DMF (5.5 mL) and H₂O (4.5 mL) for 72 h. The crude product was purified by preparative HPLC (H₂O+0.05%FA / ACN+0.05%FA 95:5 to 5:95) affording compound **139** as a white solid after lyophilization (21 mg, 15%). Purity: 100%, LC tr = 2.55 min, MS (ESI+): m/z = 527 [M + H]⁺. ¹H NMR (500 MHz, DMSO-*d₆*) *δ* ppm: 10.53 (s, 1H), 9.81 (s, 1H), 8.96 (s, 1H), 8.32-8.30 (m, 1H), 8.14-8.12 (m, 2H), 8.07-7.99 (m, 2H), 7.56-7.54 (m, 2H), 7.25-7.24 (m, 2H), 5.51 (s, 2H), 4.97 (s, 2H), 3.20 (t, *J* = 7.6 Hz, 1H), 1.67 (t, *J* = 7.2 Hz, 2H), 1.46 (sep, *J* = 6.6 Hz, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.8, 166.3, 158.3, 141.4, 138.7, 136.9, 136.0, 135.3, 130.8, 128.6 (2C), 126.2, 125.2, 124.0, 121.6, 119.5 (2C), 52.5, 49.9, 38.0, 33.5, 25.7, 22.4, 22.3. HRMS-ESI+ (m/z): calcd. for C₂₄H₂₇N₆O₆S [M + H]⁺ 527.1712, found 527.1619.

### Ethyl 2-[[4-[[5-[(tert-butoxycarbonylamino)methyl]triazol-1-yl]methyl]phenyl] carbamoyl]-4-methyl-pentanoate (184)

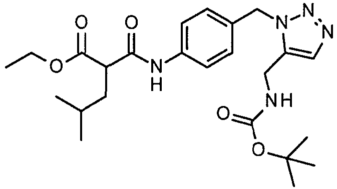

To a microwave tube filled with dioxane (6.0 mL) and degassed with N₂ during 10 min was added Cp*RuCl(COD) (16.7 mg, 0.04 mmol, 0.05 eq) and the mixture was stirred under N₂ at room temperature during 2 min. Then, **83** (280 mg, 0.88 mmol) and *N*-Boc-propargylamine (136 mg, 0.88 mmol) were added, the tube was sealed, and the reaction medium was heated at 80°C and stirred overnight. After, dioxane was evaporated under reduced pressure, and the crude was purified through flash chromatography on silica gel column (CH₂Cl₂ to CH₂Cl₂/MeOH: 95/5) affording compound **184** as brown oil (295 mg, 70%). LC tr = 4.40 min, MS (ESI+): m/z= 947 [2M + H]⁺, 474 [M + H]⁺, 418 [M - tBu + H]⁺, 374 [M - Boc + H]⁺. ¹H NMR (500 MHz, DMSO-*d₆*) *δ* ppm: 10.28 (s, 1H), 7.58-7.56 (m, 2H), 7.53 (s, 1H), 7.46 (t, *J* = 5.5 Hz, 1H), 7.17-7.16 (m, 2H), 5.54 (s, 2H), 4.18 (d, *J* = 5.7 Hz, 2H), 4.13-4.06 (m, 2H), 3.56 (dd, *J* = 8.7 and 6.1 Hz, 1H), 1.78-1.72 (m, 1H), 1.68-1.62 (m, 1H), 1.53-1.44 (m, 1H), 1.36 (s, 9H), 1.16 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 155.5, 138.5, 135.7, 132.7, 130.7, 128.0 (2C), 119.4 (2C), 78.4, 60.6, 50.9, 50.2, 37.4, 32.9, 28.1 (3C), 25.6, 22.7, 21.9, 14.0.

### tert-butyl N-[[3-[[4-[[2-(hydroxycarbamoyl)-4-methyl-pentanoyl]amino]phenyl] methyl]triazol-4-yl]methyl]carbamate (185)

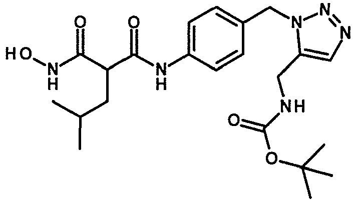

To a solution of ethyl 2-[[4-[[5-[(tert-butoxycarbonylamino)methyl]triazol-1-yl]methyl]phenyl]carbamoyl]-4-methyl-pentanoate (70 mg, 0.15 mmol) in methanol (1.5 mL) was added aqueous hydroxylamine (1.5 mL, 50% w/w in water) and KCN (2.86 mg, 0.04 mmol, 0.3 eq.). The mixture was stirred 48h. Then, the solvents were removed under reduced pressure and the residue was purified through preparative HPLC (H₂O+0.05%FA/ ACN+0.05%FA: 95:5 to 5:95) to give the expected product as a white solid after lyophilization (18 mg, 26%). Purity: 100%, LC tr = 3.46 min, MS (ESI+): m/z= 921 [2M + H]⁺, 461 [M + H]⁺, 405 [M - tBu + H]⁺, 361 [M - Boc + H]⁺. ¹H NMR (500 MHz, DMSO-*d₆*) δ: 10.53 (s, 1H), 9.82 (s, 1H), 8.96 (s, 1H), 7.57-7.55 (m, 2H), 7.51 (s, 1H), 7.46 (t, *J =* 5.3 Hz, 1H), 7.16-7.14 (m, 2H), 5.53 (s, 2H), 4.16 (d, *J* = 5.7 Hz, 2H), 3.20 (t, *J* = 7.6 Hz, 1H), 1.69-1.65 (m, 2H), 1.47 (sep, *J* = 6.7 Hz, 1H), 1.36 (s, 9H), 0.87 (d, *J* = 6.5 Hz, 3H), 0.86 (d, *J* = 6.5 Hz, 3H). ¹³C NMR (126 MHz, DMSO-*d₆*) δ: 167.8, 166.2, 155.5, 138.5, 135.7, 132.7, 130.5, 128.0 (2C), 119.5 (2C), 78.4, 50.2, 49.9, 38.0, 32.9, 28.1 (3C), 25.7, 22.4, 22.3. HRMS-ESI+ (m/z): calcd. for C₂₂H₃₃N₆O₅ [M + H]⁺ 461.2512, found 461.2480.

### [3-[[4-[(2-Ethoxycarbonyl-4-methyl-pentanoyl)amino]phenyl]methyl]triazol-4-yl]methylammonium;chloride (186)

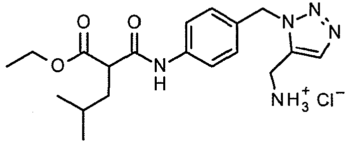

Compound **184** (290 mg, 0.61 mmol) was dissolved in ethanol and dichloromethane (5 mL, 2.5:2.5 v/v) and 4 N HCl in dioxane (2.5 mL) was added. The mixture was stirred overnight at room temperature. Then, solvents were evaporated to give compound **186** as a yellowish oil (251 mg, yield considered quantitative). LC tr = 3.04 min, MS (ESI+): m/z = 374 [M + H]⁺. ¹H NMR (500 MHz, DMSO-*d₆*) δ: 10.47 (s, 1H), 8.74 (br s, 3H), 7.89 (s, 1H), 7.61-7.59 (m, 2H), 7.21-7.19 (m, 2H), 5.68 (s, 2H), 4.15-4.05 (m, 4H), 1.76-1.71 (m, 1H), 1.67-1.61 (m, 1H), 1.48 (sep, *J* = 6.7 Hz, 1H), 1.15 (t, *J* = 7.1 Hz, 3H), 0.88 (d, *J* = 6.6 Hz, 3H), 0.86 (d, *J* = 6.6 Hz, 3H). ¹³C NMR (126 MHz, DMSO-*d₆*) δ: 169.8, 167.3, 138.8, 134.3, 131.1, 130.3, 128.2 (2C), 119.5 (2C), 60.6, 50.9, 50.6, 37.4, 31.1, 25.7, 22.7, 22.0, 14.0.

### Ethyl 2-[[4-[[5-[[(4-iodophenyl)sulfonylamino]methyl]triazol-1-yl]methyl]phenyl] carbamoyl]-4-methyl-pentanoate (187)

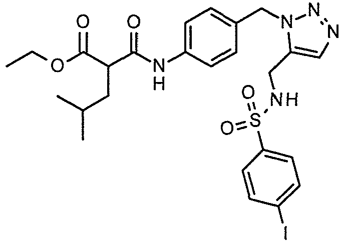

Compound **187** was synthesized according to the general procedure C, using **186** (70 mg, 0.17 mmol), 4-iodobenzenesulfonyl chloride (62 mg, 0.21 mmol) and *N,N-*diisopropylethylamine (89 µL, 0.51 mmol) in DMF (2 mL) overnight. The crude product was purified by flash chromatography on silica gel (CH₂Cl₂ to CH₂Cl₂/MeOH: 95/5) affording compound **187** as brown oil (71 mg, 64%). LC tr = 4.68 min, MS (ESI+): m/z = 640 [M + H]⁺. ¹H NMR (500 MHz, DMSO-*d₆*) *δ* ppm: 10.30 (s, 1H), 8.39 (t, *J* = 5.5 Hz, 1H), 7.96-7.94 (m, 2H), 7.57-7.55 (m, 2H), 7.50 (s, 1H), 7.48-7.46 (m, 2H), 7.13-7.11 (m, 2H), 5.48 (s, 2H), 4.14-4.04 (m, 4H), 3.56 (dd, *J* = 8.7 and 6.2 Hz, 1H), 1.78-1.73 (m, 1H), 1.68-1.62 (m, 1H), 1.49 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (DMSO-*d₆*) *δ* ppm: 169.7, 167.1, 139.4, 138.6, 138.2 (2C), 133.5, 133.4, 130.3, 128.2 (2C), 128.2 (2C), 119.4 (2C), 100.9, 60.7, 50.9, 50.3, 37.4, 35.3, 25.6, 22.7, 21.9, 14.0.

### Ethyl 2-[[4-[[5-[[(4-fluorophenyl)sulfonylamino]methyl]triazol-1-yl]methyl] phenyl]carbamoyl]-4-methyl-pentanoate (188)

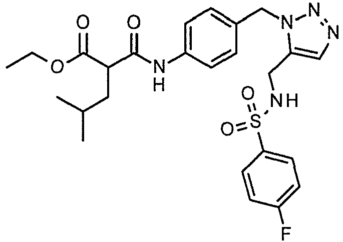

Compound **188** was synthesized according to the general procedure C, using **186** (70 mg, 0.17 mmol), 4-fluorobenzenesulfonyl chloride (39.9 mg, 0.21 mmol) and *N,N-*diisopropylethylamine (89 µL, 0.51 mmol) in DMF (2 mL) overnight. The crude product was purified by flash chromatography on silica gel (CH₂Cl₂ to CH₂Cl₂/MeOH: 95/5) affording compound **188** as pale yellow oil (65 mg, 70%). LC tr = 4.41 min, MS (ESI+): m/z= 532 [M + H]⁺. ¹H NMR (500 MHz, DMSO-*d₆*) *δ* ppm: 10.29 (s, 1H), 8.35 (s, 1H), 7.78-7.75 (m, 2H), 7.56-7.55 (m, 2H), 7.49 (s, 1H), 7.41-7.38 (m, 2H), 7.13-7.12 (m, 2H), 5.48 (s, 2H), 4.12-4.06 (m, 4H), 3.56 (dd, *J* = 8.7 and 6.2 Hz, 1H), 1.78-1.72 (m, 1H), 1.68-1.62 (m, 1H), 1.49 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.2, 164.2 (*J* = 251.4 Hz), 138.6, 136.2 (*J* = 2.8 Hz), 133.5, 133.2, 130.3, 129.6 (*J* = 9.2 Hz, 2C), 128.2 (2C), 119.4 (2C), 116.4 (*J* = 23.0 Hz, 2C), 60.7, 50.9, 50.3, 37.4, 35.3, 25.6, 22.6, 21.9, 14.0.

### Ethyl 2-[[4-[[5-[(isobutylsulfonylamino)methyl]triazol-1-yl]methyl]phenyl] carbamoyl]-4-methyl-pentanoate (189)

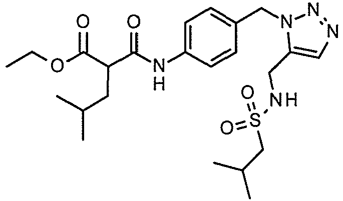

Compound **189** was synthesized according to the general procedure C, using **186** (100 mg, 0.24 mmol), 4-fluorobenzenesulfonyl chloride (45.9 mg, 0.29 mmol) and *N,N-*diisopropylethylamine (127 µL, 0.73 mmol) in DMF (2 mL) overnight. The crude product was purified by flash chromatography on silica gel (CH₂Cl₂ to CH₂Cl₂/MeOH: 95/5) affording compound **189** as yellow oil (80 mg, 65%). LC tr = 4.30 min, MS (ESI+): m/z = 494 [M + H]⁺. ¹H NMR (500 MHz, DMSO-*d₆*) *δ* ppm: 10.29 (s, 1H), 7.72 (t, *J* = 5.2 Hz, 1H), 7.67 (s, 1H), 7.58-7.56 (m, 2H), 7.21-7.19 (m, 2H), 5.56 (s, 2H), 4.22 (d, *J* = 5.2 Hz, 2H), 4.14-4.04 (m, 2H), 3.55 (dd, *J* = 8.8 and 6.2 Hz, 1H), 2.82 (d, *J* = 6.5 Hz, 2H), 2.01 (sep, *J* = 6.7 Hz, 1H), 1.77-1.71 (m, 1H), 1.67-1.61 (m, 1H), 1.48 (sep, *J* = 6.7 Hz, 1H), 1.15 (t, *J* = 7.1 Hz, 3H), 0.96 (d, *J* = 6.7 Hz, 6H), 0.88 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 138.6, 134.4, 133.4, 130.5, 128.2 (2C), 119.4 (2C), 60.7, 58.9, 50.9, 50.3, 37.4, 34.9, 25.6, 24.2, 22.7, 22.2 (2C), 21.9, 14.0.

### 2-(Hydroxycarbamoyl)-N-[4-[[5-[[(4-iodophenyl)sulfonylamino]methyl]triazol-1-yl]methyl]phenyl]-4-methyl-pentanamide (190)

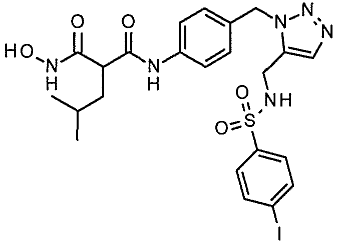

Compound **190** was synthesized according to the general procedure D, using ester **187** (64 mg, 0.1 mmol), KCN (1.94 mg, 0.03 mmol) and NH₂OH (1.2 mL, 50% w/w in water) in MeOH (1.2 mL) for 48 h. The crude product was purified through preparative HPLC (H₂O+0.05%FA / ACN+0.05%FA: 95:5 to 5:95) to give compound **190** as a white solid after lyophilization (18 mg, 29%). Purity: 100%, LC tr = 3.82 min, MS (ESI+): m/z = 627 [M + H]⁺. ¹H NMR (500 MHz, DMSO-*d₆*) *δ* ppm: 10.54 (br s, 1H), 9.82 (s, 1H), 8.97 (s, 1H), 8.39 (br s, 1H), 7.97-7.95 (m, 2H), 7.56-7.54 (m, 2H), 7.49-7.47 (m, 3H), 7.11-7.10 (m, 2H), 5.47 (s, 2H), 4.05 (s, 2H), 3.21 (t, *J* = 7.6 Hz, 1H), 1.70-1.66 (m, 2H), 1.48 (sep, *J* = 6.6 Hz, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.9, 166.3, 139.4, 138.6, 138.2 (2C), 133.5, 133.4, 130.2, 128.2 (2C), 128.1 (2C), 119.6 (2C), 100.9, 50.3, 50.0, 38.0, 35.3, 25.7, 22.4, 22.3. HRMS-ESI+ (m/z): calcd. for C₂₃H₂₈IN₆O₅S [M + H]⁺: 627.0887, found 627.0881.

### N-[4-[[5-[[(4-Fluorophenyl)sulfonylamino]methyl]triazol-1-yl]methyl]phenyl]-2-(hydroxycarbamoyl)-4-methyl-pentanamide (191)

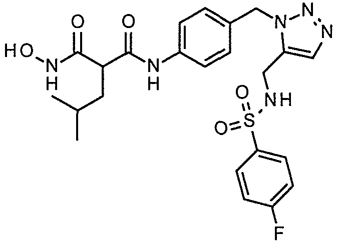

Compound **191** was synthesized according to the general procedure D, using ester **188** (58 mg, 0.11 mmol), KCN (2.11 mg, 0.03 mmol) and NH₂OH (1.2 mL, 50% w/w in water) in MeOH (1.2 mL) for 48 h. The crude product was purified through preparative HPLC (H₂O+0.05%FA / ACN+0.05%FA: 95:5 to 5:95) to give compound **191** as a white solid after lyophilization (21 mg, 37%). Purity: 100%, LC tr = 3.50 min, MS (ESI+): m/z = 519 [M + H]⁺. ¹H NMR (500 MHz, DMSO-*d₆*) *δ* ppm: 10.53 (br s, 1H), 9.82 (s, 1H), 8.96 (s, 1H), 8.35 (br s, 1H), 7.79-7.76 (m, 2H), 7.56-7.54 (m, 2H), 7.48 (s, 1H), 7.42-7.39 (m, 2H), 7.12-7.10 (m, 2H), 5.48 (s, 2H), 4.06 (s, 2H), 3.21 (t, *J* = 7.6 Hz, 1H), 1.70-1.66 (m, 2H), 1.47 (sep, *J* = 6.7 Hz, 1H), 0.87 (d, *J* = 6.7 Hz, 3H), 0.86 (d, *J* = 6.7 Hz, 3H). ¹³C NMR (126 MHz, DMSO-*d₆*) *δ* ppm: 167.9, 166.3, 164.2 (d, *J* = 251.1 Hz), 138.6, 136.2 (d, *J* = 2.7 Hz), 133.5, 133.3, 130.1, 129.6 (d, *J* = 9.6 Hz, 2C), 128.1 (2C), 119.5 (2C), 116.4 (d, *J* = 22.5 Hz, 2C), 50.3, 50.0, 38.0, 35.3, 25.7, 22.4, 22.3. HRMS-ESI+ (m/z): calcd. for C₂₃H₂₈FN₆O₅S [M + H]⁺ 519.1826, found 519.1821.

### 2-(Hydroxycarbamoyl)-N-[4-[[5-[(isobutylsulfonylamino)methyl]triazol-1-yl]methyl]phenyl]-4-methyl-pentanamide (192)

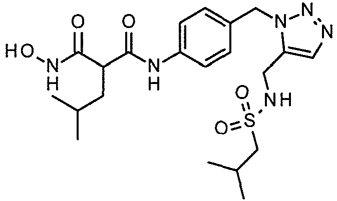

Compound **192** was synthesized according to the general procedure D, using ester **189** (70 mg, 0.14 mmol), KCN (2.74 mg, 0.04 mmol) and NH₂OH (1.5 mL, 50% w/w in water) in MeOH (1.5 mL) for 48h. The crude product was purified through preparative HPLC (H₂O+0.05%FA / ACN+0.05%FA: 95:5 to 5:95) to give compound **192** as a white solid after lyophilization (34 mg, 50%). Purity: 100%, LC tr = 3.36 min, MS (ESI+): m/z= 481 [M + H]⁺. ¹H NMR (500 MHz, DMSO-*d₆*) *δ* ppm: 10.52 (s, 1H), 9.81 (s, 1H), 8.96 (s, 1H), 7.72 (t, *J* = 5.4 Hz, 1H), 7.67 (s, 1H), 7.57-7.55 (m, 2H), 7.19-7.17 (m, 2H), 5.55 (s, 2H), 4.22 (d, *J* = 5.1 Hz, 2H), 3.20 (t, *J* = 7.6 Hz, 1H), 2.84 (d, *J* = 6.4 Hz, 2H), 2.02 (sep, *J* = 6.7 Hz, 1H), 1.69-1.65 (m, 2H), 1.47 (sep, *J* = 6.7 Hz, 1H), 0.97 (d, *J* = 6.7 Hz, 6H), 0.87 (d, *J* = 6.7 Hz, 3H), 0.86 (d, *J* = 6.7 Hz, 3H). ¹³C NMR (126 MHz, DMSO-*d₆*) *δ* ppm: 167.8, 166.3, 138.6, 134.4, 133.4, 130.4, 128.1 (2C), 119.5 (2C), 58.9, 50.3, 50.0, 38.0, 35.0, 25.7, 24.2, 22.4, 22.3, 22.2 (2C). HRMS-ESI⁻ (m/z): calcd. for C₂₁H₃₁IN₆O₅S [M - H]⁻ 479.2077, found 479.2083.

### N-[4-[[4-(Aminomethyl)triazol-1-yl]methyl]phenyl]-2-(hydroxycarbamoyl)-4-methyl-pentanamide (140)

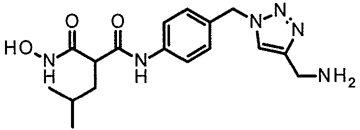

Compound **139** (30 mg, 0.07 mmol) was dissolved in CH₂Cl₂ (1 mL) and was cooled down to 0 °C with an ice-bath before an addition of 4N HCl in dioxane (1 mL). The mixture was stirred at room temperature overnight. Solvents were then evaporated under reduced pressure, and the crude product was purified through preparative HPLC (H₂O+0.05%FA / ACN+0.05%FA: 95:5 to 5:95) to give compound **140** as a beige solid after lyophilization (8 mg, 30%). Purity: 100%, LC tr = 2.29 min, MS (ESI+): m/z= 361 [M + H]⁺. ¹H NMR (500 MHz, DMSO-*d₆*) *δ* ppm: 10.33 (s, 1H), 8.62 (m, 2H), 8.10 (s, 1H), 7.63-7.61 (m, 2H), 7.30-7.28 (m, 2H), 5.56 (s, 2H), 4.04 (s, 2H), 3.59-3.15 (m, 3H), 1.65 (t, *J* = 7.2 Hz, 2H), 1.44 (sep, *J* = 6.7 Hz, 1H), 0.86 (d, *J* = 6.4 Hz, 3H), 0.85 (d, *J* = 6.6 Hz, 3H). ¹³C NMR (126 MHz, DMSO-*d₆*) *δ* ppm: 166.7, 166.1, 141.6, 139.0, 130.5, 128.7 (2C), 123.9, 119.4 (2C), 52.6, 49.3, 37.4, 34.3, 25.6, 22.5, 22.3. HRMS-ESI+ (m/z): calcd. for C₁₇H₂₅N₆O₃ [M + H]⁺ 361.1988, found 361.1958.

### tert-Butyl 4-[methoxy(methyl)carbamoyl]thiazolidine-3-carboxylate (96)

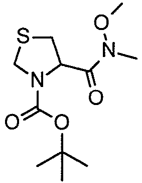

To a solution of 3-tert-butoxycarbonylthiazolidine-4-carboxylic acid (1000 mg, 4.3 mmol) in DMF (8 mL) were added triethylamine (3520 µL, 25.6 mmol), HOBt (656 mg, 4.3 mmol) and EDC.HCl (2880 mg, 15.0 mmol) and the reaction media was stirred for 20 minutes. A suspension of N-methoxymethanamine;hydrochloride (836 mg, 8.6 mmol) and triethylamine (1170 µL, 8.6 mmol) in DMF (2 mL) was then added and the mixture was stirred at room temperature overnight. Triethylamine salts were filtered; the filtrate was dissolved in water and extracted with ethyl acetate. Combined organic layers were dried over MgSO₄, filtered and concentrated under reduced pressure. The residue was finally purified through flash chromatography on silica gel (cHex to cHex/EtOAc: 5/5) affording compound **96** as a white solid (903 mg, 80%). LC tr = 3.53 min, MS (ESI+): m/z = 221 [M + H - 177 [M + H - Boc]⁺. ¹H NMR (500 MHz, CDCl₃) *δ* ppm: 5.02-5.00 (m, 0.5H), 4.83-4.81 (m, 0.4H), 4.66-4.65 (m, 0.4H), 4.59-4.58 (m, 0.6Hz), 4.45-4.44 (m, 0.4H), 4.44-4.42 (m, 0.6H), 3.71 (s, 1.6H), 3.66 (s, 1.4H), 3.36-3.26 (m, 1H), 3.13 (s, 3H), 2.99-2.93 (m, 1H), 1.38 (s, 4.9H), 1.34 (s, 4.1H). ¹³C NMR (126 MHz, CDCl₃) *δ* ppm: 171.2 (min), 170.8 (maj), 153.2 (maj), 152.9 (min), 80.8 (min), 80.7 (maj), 61.3 (maj), 61.3 (min), 59.5 (min), 59.3 (maj), 49.9 (min), 49.4 (maj), 34.5 (min), 33.4 (maj), 32.4 (min), 32.2 (maj), 28.2 (maj), 28.2 (min).

### tert-Butyl 4-formylthiazolidine-3-carboxylate (97)

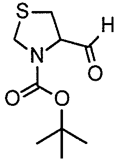

Compound **96** (903 mg, 3.27 mmol) was dissolved in anhydrous THF (30 mL) and cooled down to 0 °C before addition of a solution of LAH (2.4 M in THF, 1.7 mL, 4.08 mmol). The mixture was stirred for 30 minutes and then quenched carrefully with 1M aq. KHSO₄ at 0°C. The resulting blurry solution was poured in 1M aq. HCl and extracted with CH₂Cl₂. Combined organic layers were dried over MgSO₄, filtered and concentrated under reduced pressure. The residue was filtered through a pad of silica gel (CH₂Cl₂), affording compound **97** as yellow oil (620 mg, 87%). LC tr = not visible, MS (ESI+): m/z = no ionization. ¹H NMR (500 MHz, CDCl₃) *δ* ppm: 9.59-9.56 (m, 1H), 4.67-4.57 (m, 2H), 4.45-4.42 (m, 1H), 3.25-3.13 (m, 2H), 1.51-1.46 (m, 9H). ¹³C NMR (126 MHz, CDCl₃) *δ* ppm: 199.5 (maj), 199.4 (min), 153.9 (min), 153.3 (maj), 82.1 (maj), 81.8 (min), 67.3 (min), 67.2 (maj), 49.8 (maj), 48.9 (min), 32.3 (maj), 30.5 (min), 28.4 (min, 3C), 28.4 (maj).

### tert-Butyl 4-ethynylthiazolidine-3-carboxylate (98)

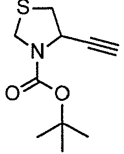

Compound **97** (610 mg, 2.1 mmol) and potassium carbonate (776 mg, 5.6 mmol, 2.0) were mixed in anhydrous methanol (8 mL) before an addition of Ohira-Bestmann reagent (809 µL, 3.4 mmol). The resulting mixture was stirred overnight at room temperature. The mixture was then diluted with CH₂Cl₂ and washed with 5% aq. NaHCO₃. The organic layer was concentrated under reduced pressure and the residue was purified through flash chromatography on silica gel column (cHex to cHex/EtOAc: 7/3) affording compound **98** as brown oil (445 mg, 74%). LC tr = 4.42 min, MS (ESI+): m/z = 158 [M + H - 114 [M + H - Boc]⁺. ¹H NMR (500 MHz, CDCl₃) *δ* ppm: 5.10-5.00 (m, 1H), 4.64 (d, *J* = 9.4 Hz, 1H), 4.40 (d, *J* = 9.4 Hz, 1H), 3.30-3.23 (m, 1H), 3.09-3.06 (m, 1H), 2.33 (d, *J* = 2.1 Hz, 1H), 1.49 (s, 9H). ¹³C NMR (126 MHz, CDCl₃) *δ* ppm: 153.0, 81.5, 77.4. 71.1, 55.9, 50.9, 47.8, 36.9, 28.4 (3C).

### (4-Ethynylthiazolidin-3-yl)-(4-fluorophenyl)methanone (99)

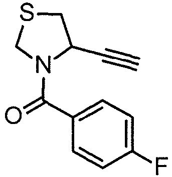

Compound **98** (335 mg, 1.6 mmol) was dissolved in methanol and dichloromethane (12.0 mL, 6:6 v/v), and the mixture was cooled down to 0 °C with an ice-bath before an addition of 4N HCl in dioxane (6 mL). The mixture was stirred at room temperature 1 h. The mixture was then cooled down to 0 °C, and triethylamine (1290 µL, 9.4 mmol) and 4-fluorobenzoyl chloride (208 µL, 1.7 mmol) were carefully added. The mixture was stirred at room temperature overnight. It was then diluted in CH₂Cl₂ and washed with brine. Combined organic layers were concentrated under reduced pressure and the residure was purified through flash chromatography on silica gel column (cHex to cHex/EtOAc: 7/3) affording compound **99** as a brown solid (256 mg, 69%). LC tr = 3.76 min, MS (ESI+): m/z = 236 [M + H]⁺. ¹H NMR (500 MHz, CDCl₃) *δ* ppm: 7.64-7.62 (m, 2H), 7.14-7.11 (m, 2H), 5.32 (br s, 2H), 4.60 (br s, 1H), 3.26-3.14 (m, 2H), 2.43 (s, 1H). ¹³C NMR (126 MHz, CDCl₃) *δ* ppm: 168.5, 164.3 (d, *J* = 251.8 Hz), 131.3 (d, *J* = 11.0 Hz), 130.2 (d, *J* = 7.6 Hz, 2C), 115.8 (d, *J* = 21.8 Hz, 2C), 81.1, 77.4, 72.5, 50.3, 37.0.

### tert-butyl N-[4-(prop-2-ynylsulfamoyl)phenyl]carbamate (193)

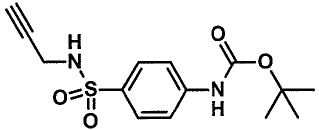

Compound **193** was synthesized according to the general procedure C, using propargylamine (72.4 µL, 1.13 mmol), tert-butyl *N*-(4-chlorosulfonylphenyl)carbamate (300 mg, 1.03 mmol) and *N*,*N*-diisopropylethylamine (359 µL, 2.06 mmol) in CH₂Cl₂ (5 mL) overnight. The crude product was purified by flash chromatography on silica gel (CH₂Cl₂ to CH₂Cl₂/MeOH: 98/2) affording compound **193** as a brown solid (237 mg, 74%). MS (ESI+): m/z = 311 [M + H]⁺. ¹H NMR (500 MHz, DMSO-*d₆*) *δ* ppm: 9.81 (s, 1H), 7.94 (t, *J* = 5.4 Hz, 1H), 7.69-7.67 (m, 2H), 7.62-7.61 (m, 2H), 3.64-3.63 (m, 2H), 3.06 (t, J = 2.4 Hz, 1H), 1.48 (s, 9H). ¹³C NMR (126 MHz, DMSO-*d₆*) *δ* ppm: 152.5, 143.3, 133.0, 127.9 (2C), 117.4 (2C), 79.8, 79.5, 74.6, 31.9, 28.0 (3C).

### 4-acetyl-N-prop-2-ynyl-benzenesulfonamide (194)

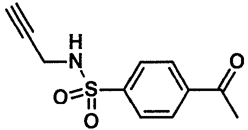

Compound **194** was synthesized according to the general procedure C, using propargylamine (72.4 µL, 1.13 mmol), 4-acetylbenzenesulfonyl chloride (300 mg, 1.03 mmol) and *N*,*N*-diisopropylethylamine (359 µL, 2.06 mmol) in CH₂Cl₂ (5 mL) overnight. The crude product was purified by flash chromatography on silica gel (CH₂Cl₂ to CH₂Cl₂/MeOH: 98/2) affording compound **194** as a brown solid (237 mg, 74%). ¹H NMR (500 MHz, DMSO-*d₆*) *δ* ppm: 8.34 (br s, 1H), 8.13-7.94 (m, 4H), 3.75 (br s, 2H), 3.04 (br s, 1H), 2.64 (s, 3H). ¹³C NMR (126 MHz, DMSO-*d₆*) *δ* ppm: 197.4, 144.3, 139.5, 128.9 (2C), 127.1 (2C), 79.1, 74.9, 31.9, 27.1.

### 4-(2-methoxyethoxy)-N-prop-2-ynyl-benzenesulfonamide (195)

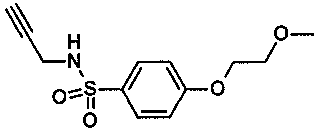

Compound **195** was synthesized according to the general procedure C, using propargylamine (42.2 µL, 0.7 mmol), 4-(2-methoxyethoxy)benzenesulfonyl chloride (150 mg, 0.6 mmol) and *N*,*N*-diisopropylethylamine (209 µL, 1.2 mmol) in CH₂Cl₂ (5 mL) overnight. The crude product was purified by flash chromatography on silica gel (CH₂Cl₂ to CH₂Cl₂/MeOH: 95/5) affording compound **195** as a colorless oil (97 mg, 60%). MS (ESI+): m/z = 270 [M + H]⁺. ¹H NMR (500 MHz, DMSO-*d₆*) *δ* ppm: 7.95 (s, 1H), 7.72-7.71 (m, 2H), 7.11-7.10 (m, 2H), 4.18-4.16 (m, 2H), 3.68-3.66 (m, 2H), 3.64 (d, *J* = 2.5 Hz, 2H), 3.31 (s, 3H), 3.04 (t, *J* = 2.5 Hz, 1H). ¹³C NMR (126 MHz, DMSO-*d₆*) *δ* ppm: 161.5, 132.1, 128.9 (2C), 114.6 (2C), 79.4, 74.6, 70.1, 67.4, 58.2, 31.9.

### methyl 4-(prop-2-ynylsulfamoyl)benzoate (196)

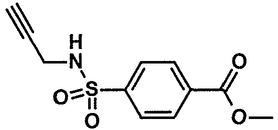

Compound **196** was synthesized according to the general procedure C, using propargylamine (150 µL, 2.34 mmol), methyl 4-chlorosulfonylbenzoate (500 mg, 2.13 mmol) and *N*,*N*-diisopropylethylamine (742 µL, 4.26 mmol) in CH₂Cl₂ (10 mL) overnight. The crude product was purified by flash chromatography on silica gel (cHex to cHex/EtOAc: 5/5) affording compound **196** as a white solid (391 mg, 72%). MS (ESI+): m/z = 254 [M + H]⁺. ¹H NMR (500 MHz, DMSO-*d₆*) *δ* ppm: 8.36 (t, *J* = 5.8 Hz, 1H), 8.14-8.13 (m, 2H), 7.95-7.94 (m, 2H), 4.18-4.16 (m, 2H), 3.89 (s, 3H), 3.76-3.75 (m, 2H), 3.01 (br s, 1H). ¹³C NMR (126 MHz, DMSO-*d₆*) *δ* ppm: 165.2, 144.7, 132.9, 129.9 (2C), 127.2 (2C), 79.0, 74.9, 52.6, 31.9.

### 4-isopropoxy-N-prop-2-ynyl-benzenesulfonamide (197)

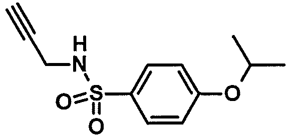

Compound **197** was synthesized according to the general procedure C, using propargylamine (90 µL, 1.41 mmol), 4-isopropoxybenzenesulfonyl chloride (300 mg, 1.28 mmol) and *N*,*N*-diisopropylethylamine (446 µL, 2.56 mmol) in CH₂Cl₂ (5 mL) overnight. The crude product was purified by flash chromatography on silica gel (cHex to cHex/EtOAc: 6/4) affording compound **197** as a pale yellow powder (152 mg, 47%). MS (ESI+): m/z = 254 [M + H]⁺. ¹H NMR (500 MHz, DMSO-*d₆*) *δ* ppm: 7.93 (t, *J* = 5.8 Hz, 1H), 7.72-7.67 (m, 2H), 7.09-7.04 (m, 2H), 4.72 (sept, *J* = 6.0 Hz, 1H), 3.66-3.61 (m, 2H), 3.06-3.03 (t, *J* = 2.3 Hz, 1H), 1.28 (d, *J* = 6.0 Hz, 6H). ¹³C NMR (126 MHz, DMSO-*d₆*) *δ* ppm: 160.6, 131.5, 128.9 (2C), 115.4 (2C), 79.5, 74.6, 69.8, 31.9, 21.6 (2C).

### 4-isopropyl-N-prop-2-ynyl-benzenesulfonamide (198)

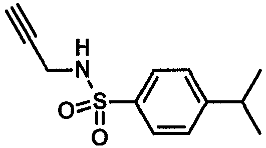

Compound **198** was synthesized according to the general procedure C, using propargylamine (195 µL, 3.02 mmol), 4-isopropylbenzenesulfonyl chloride (600 mg, 2.74 mmol) and *N*,*N*-diisopropylethylamine (955 µL, 5.48 mmol) in CH₂Cl₂ (10 mL) overnight. The crude product was purified by flash chromatography on silica gel (cHex to cHex/EtOAc: 6/4) affording compound **198** as a pale yellow powder (112 mg, 17%). MS (ESI+): m/z = 238 [M + H]⁺. ¹H NMR (500 MHz, DMSO-*d₆*) *δ* ppm: 7.95 (br s, 1H), 7.74-7.70 (m, 2H), 7.47-7.43 (m, 2H), 3.68-3.64 (m, 2H), 3.05-3.01 (m, 1H), 3.01-2.94 (m, 1H), 1.22 (d, *J* = 6.9 Hz, 6H). ¹³C NMR (126 MHz, DMSO-*d₆*) *δ* ppm: 153.8, 138.3, 127.4 (2C), 127.3 (2C), 79.9, 75.0, 33.9, 32.4, 24.0 (2C).

### ethyl 2-[[4-[[4-[[[4-(2-methoxyethoxy)phenyl]sulfonylamino]methyl]triazol-1-yl]methyl]phenyl]carbamoyl]-4-methyl-pentanoate (199)

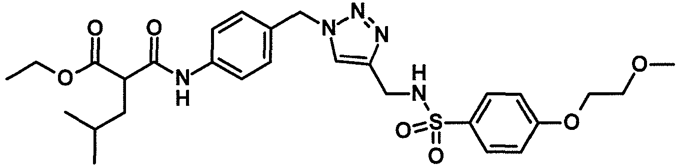

Compound **199** was synthesized according to the general procedure E, using azide **83** (100 mg, 0.31 mmol), alkyne **195** (83.5 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 95/5) affording compound **199** as a white solid (152 mg, 83%). MS (ESI+): m/z = 588 [M + H]⁺. ¹H NMR (500 MHz, DMSO-*d₆*) *δ* ppm: 10.29 (s, 1H), 7.92 (t, *J* = 5.8 Hz, 1H), 7.85 (s, 1H), 7.70-7.69 (m, 2H), 7.58-7.56 (m, 2H), 7.25-7.24 (m, 2H), 7.07-7.05 (m, 2H), 5.46 (s, 2H), 4.18-4.16 (m, 2H), 4.13-4.05 (m, 2H), 3.98 (d, *J* = 5.9 Hz, 2H), 3.68-3.66 (m, 2H), 3.31 (s, 3H), 3.56 (dd, *J* = 8.9 and 6.5 Hz, 1H), 1.77-1.72 (m, 1H), 1.68-1.62 (m, 1H), 1.48 (sep, *J* = 6.7 Hz, 1H), 1.15 (t, *J* = 7.1 Hz, 3H), 0.89 (d, *J* = 6.7 Hz, 3H), 0.87 (d, *J* = 6.7 Hz, 3H). ¹³C NMR (126 MHz, DMSO-*d₆*) *δ* ppm: 169.7, 167.1, 162.1, 143.7, 138.7, 131.9, 131.0, 128.7 (4C), 123.2, 119.4 (2C), 114.3 (2C), 74.6, 70.1, 60.7, 55.6, 52.4, 51.0, 38.1, 37.4, 25.7, 22.7, 21.9, 14.0.

### ethyl 2-[[4-[[4-[[(4-isopropoxyphenyl)sulfonylamino]methyl]triazol-1-yl]methyl]phenyl]carbamoyl]-4-methyl-pentanoate (200)

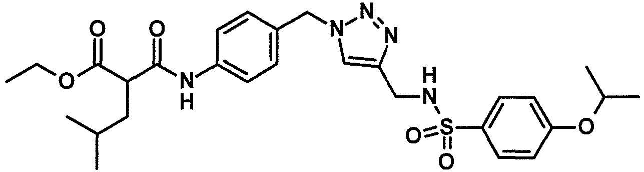

Compound **200** was synthesized according to the general procedure E, using azide **83** (75 mg, 0.24 mmol), alkyne **197** (60 mg, 0.24 mmol), copper (II) sulfate pentahydrate (12 mg, 0.05 mmol) and sodium ascorbate (24 mg, 0.12 mmol) in DMF (5 mL) and H₂O (4 mL) overnight. The crude product was purified by flash chromatography on silica gel (CH₂Cl₂ to CH₂Cl₂/MeOH: 95/5) affording compound **200** as a pale yellow powder (89 mg, 66%). MS (ESI+): m/z = 572 [M + H]⁺. ¹H NMR (500 MHz, DMSO-*d₆*) *δ* ppm: 10.29 (s, 1H), 7.90 (t, *J* = 6.0 Hz, 1H), 7.86 (s, 1H), 7.69-7.65 (m, 2H), 7.59-7.55 (m, 2H), 7.27-7.23 (m, 2H), 7.05-7.00 (m, 2H), 5.46 (s, 2H), 4.71 (sept, *J* = 6.1 Hz, 1H), 4.15-4.03 (m, 2H), 3.98 (d, *J* = 6.1 Hz, 2H), 3.59-3.52 (m, 1H), 1.78-1.61 (m, 2H), 1.53-1.44 (m, 1H), 1.29 (d, *J* = 6.0 Hz, 6H), 1.15 (t, *J* = 7.0 Hz, 3H), 0.92-0.84 (m, 6H). ¹³C NMR (126 MHz, DMSO-*d₆*) *δ* ppm: 169.7, 167.1, 160.5, 143.7, 138.7, 131.4, 131.0, 128.8 (2C), 128.7 (2C), 123.2, 119.4 (2H), 115.4 (2H), 69.8, 60.6, 52.3, 50.9, 38.1, 37.4, 25.6, 22.6, 22.0, 21.6 (2C), 14.0.

### ethyl 2-[[4-[[4-[[(4-isopropylphenyl)sulfonylamino]methyl]triazol-1-yl]methyl]phenyl]carbamoyl]-4-methyl-pentanoate (201)

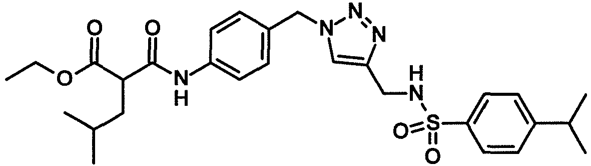

Compound **201** was synthesized according to the general procedure E, using azide **83** (75 mg, 0.24 mmol), alkyne **198** (56 mg, 0.24 mmol), copper (II) sulfate pentahydrate (12 mg, 0.05 mmol) and sodium ascorbate (24 mg, 0.12 mmol) in DMF (5 mL) and H₂O (4 mL) overnight. The crude product was purified by flash chromatography on silica gel (CH₂Cl₂ to CH₂Cl₂/MeOH: 95/5) affording compound **201** as a yellow powder (134 mg, quant. yield). MS (ESI+): m/z = 556 [M + H]⁺. ¹H NMR (500 MHz, DMSO-*d₆*) *δ* ppm: 10.29 (s, 1H), 8.01 (t, *J* = 5.3 Hz, 1H), 7.95 (s, 1H), 7.87 (s, 1H), 7.71-7.66 (m, 2H), 7.59-7.55 (m, 2H), 7.43-7.39 (m, 2H), 7.27-7.24 (m, 2H), 5.46 (s, 2H), 4.15-4.03 (m, 2H), 4.03-3.98 (m, 2H), 3.58-3.53 (m, 1H), 2.96 (sept, *J* = 6.9 Hz, 1H), 1.78-1.61 (m, 2H), 1.49 (sept, *J* = 6.9 Hz, 1H), 1.21 (d, *J* = 6.9 Hz, 6H), 1.15 (t, *J* = 7.2 Hz, 3H), 0.91-0.85 (m, 6H). ¹³C NMR (126 MHz, DMSO-*d₆*) *δ* ppm: 169.7, 167.1, 153.2, 143.7, 138.7, 137.8, 131.0, 128.7 (2C), 127.0 (2C), 126.7 (2C), 123.7, 119.4 (2C), 60.6, 52.3, 50.9, 38.1, 37.4, 33.3, 25.6, 23.5 (2C), 22.6, 21.9, 14.0.

### ethyl 2-[[4-[[4-[[[4-(tert-butoxycarbonylamino)phenyl]sulfonylamino] methyl]triazol-1-yl]methyl]phenyl]carbamoyl]-4-methyl-pentanoate (202)

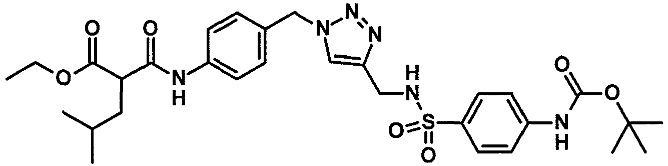

Compound **202** was synthesized according to the general procedure E, using azide **83** (100 mg, 0.31 mmol), alkyne **193** (97.5 mg, 0.31 mmol), copper (II) sulfate pentahydrate (15.7 mg, 0.06 mmol) and sodium ascorbate (31.1 mg, 0.16 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **202** as a yellow powder (160 mg, 80%). MS (ESI+): m/z= 629 [M + H]⁺, 573 [M + H - tBu]⁺, 529 [M + H - Boc]⁺. ¹H NMR (500 MHz, DMSO-*d₆*) *δ* ppm: 10.28 (s, 1H), 9.81 (s, 1H), 7.89 (t, *J* = 5.9 Hz, 1H), 7.84 (s, 1H), 7.67-7.65 (m, 2H), 7.62-7.60 (m, 2H), 7.58-7.56 (m, 2H), 7.27-7.25 (m, 2H), 5.45 (s, 2H), 4.13-4.05 (m, 2H), 3.96 (d, *J* = 5.8 Hz, 2H), 3.55 (dd, *J* = 6.2 and 8.7 Hz, 1H), 1.77-1.61 (m, 2H), 1.52-1.46 (m, 10H), 1.15 (t, *J* = 7.1 Hz, 3H), 0.89 (d, *J* = 6.8 Hz, 3H), 0.87 (d, *J* = 6.6 Hz, 3H). ¹³C NMR (126 MHz, DMSO-*d₆*) *δ* ppm: 169.7, 167.1, 152.6, 143.7, 143.3, 138.7, 132.9, 130.9, 128.8 (2C), 127.8 (2C), 123.2, 119.4 (2C), 117.6 (2C), 79.8, 60.7, 52.4, 50.9, 38.1, 37.3, 28.0, 25.6 (3C), 22.6, 21.9, 14.0.

### N-(4-[[4-[[(4-acetylphenyl)sulfonylamino]methyl]triazol-1-yl]methyl]phenyl]-2-(hydroxycarbamoyl)-4-methyl-pentanamide (203)

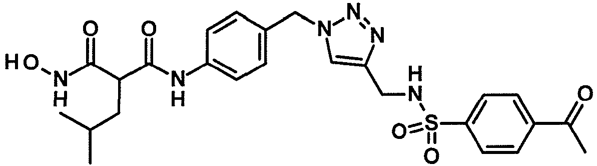

Compound **203** was synthesized according to the general procedure E, using azide **Z9** ( mg, mmol), alkyne **194** ( mg, mmol), copper (II) sulfate pentahydrate ( mg, mmol) and sodium ascorbate ( mg, mmol) in DMF (5.5 mL) and H₂O (4.5 mL) overnight. The crude product was purified by flash chromatography on silica gel (CH₂Cl₂ to CH₂Cl₂/MeOH: 98/2) affording compound **202** as a yellow powder (160 mg, 80%). Purity: 100%. MS (ESI+): m/z = 543 [M + H]⁺. ¹H NMR (500 MHz, DMSO-*d₆*) *δ* ppm: 10.51 (br s, 1H), 9.80 (s, 1H), 8.97 (s, 1H), 8.31 (br s, 1H), 8.09-8.07 (m, 2H), 7.89 (s, 1H), 7.88-7.87 (m, 2H), 7.56-7.55 (m, 2H), 7.23-7.21 (m, 2H), 5.44 (s, 2H), 4.06 (s, 2H), 3.20 (t, *J* = 7.6 Hz, 1H), 2.63 (s, 3H), 1.70-1.66 (m, 2H), 1.47 (sept, *J* = 6.7 Hz, 1H), 0.87 (d, *J* = 6.7 Hz, 3H), 0.86 (d, *J* = 6.7 Hz, 3H). ¹³C NMR (126 MHz, DMSO-*d₆*) *δ* ppm: 197.4, 167.8, 166.3, 144.1, 143.4, 139.4, 138.7, 130.7, 128.9 (2C), 128.6 (2C), 126.9 (2C), 123.2, 119.5 (2C), 52.3, 49.9, 38.0, 38.0, 27.0, 25.7, 22.4, 22.2.

### methyl 4-[[1-[[4-[[2-(hydroxycarbamoyl)-4-methyl-pentanoyl]amino]phenyl] methyl]triazol-4-yl]methylsulfamoyl]benzoate (204)

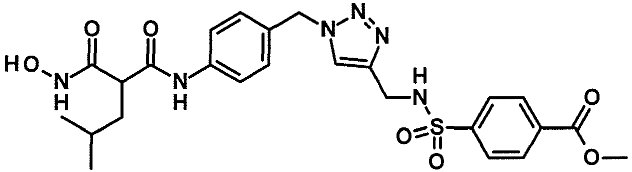

Compound **204** was synthesized according to the general procedure E, using azide **Z9** (88 mg, 0.29 mmol), alkyne **194** (73 mg, 0.29 mmol), copper (II) sulfate pentahydrate (14.5 mg, 0.06 mmol) and sodium ascorbate (28.5 mg, 0.14 mmol) in DMF (5 mL) and H₂O (4 mL) overnight. The crude product was purified by flash chromatography on silica gel (CH₂Cl₂ to CH₂Cl₂/MeOH: 95/5) affording compound **204** as a white powder (47 mg, 28%). Purity: 100%. MS (ESI+): m/z = 559 [M + H]⁺. ¹H NMR (500 MHz, DMSO-*d₆*) *δ* ppm: 10.53 (br s, 1H), 9.81 (s, 1H), 8.97 (br s, 1H), 8.34 (br s, 1H), 8.11-8.06 (m, 2H), 7.91-7.86 (m, 2H), 7.85 (s, 1H), 7.58-7.53 (m, 2H), 7.24-7.18 (m, 2H), 5.43 (s, 2H), 4.08 (s, 2H), 3.90 (s, 3H), 3.20 (t, J = 7.6 Hz, 1H), 1.68 (t, J = 7.5 Hz, 2H), 1.47 (sept, J = 6.7 Hz, 1H), 0.90-0.85 (m, 6H). ¹³C NMR (126 MHz, DMSO-*d₆*) *δ* ppm: 167.8, 166.3, 165.2, 144.5, 143.4, 138.7, 132.8, 130.7, 129.9 (2C), 128.6 (2C), 127.0 (2C), 123.2, 119.5 (2C), 52.6, 52.3, 49.9, 38.0 (2C), 25.7, 22.4, 22.2.

### 2-(hydroxycarbamoyl)-N-[4-[[4-[[[4-(2-methoxyethoxy)phenyl]sulfonylamino] methyl]triazol-1-yl]methyl]phenyl]-4-methyl-pentanamide (205)

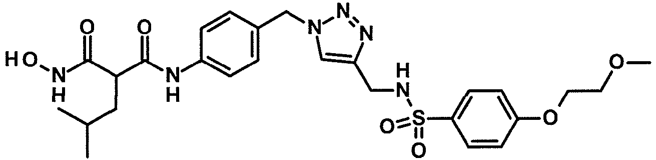

Compound **205** was synthesized according to the general procedure D, using ester **199** (145 mg, 0.25 mmol), KCN (4.9 mg, 0.08 mmol) and aq. NH₂OH (2.5 mL, 50% w/w in water) in MeOH (2.5 mL) overnight. The crude product was purified by flash chromatography on silica gel (CH₂Cl₂ to CH₂Cl₂/MeOH: 95/5) affording compound 205 as a white solid after lyophilization (65 mg, 45%). Purity: 100%. MS (ESI+): m/z = 575 [M + H]⁺. ¹H NMR (500 MHz, DMSO-*d₆*) *δ* ppm: 10.53 (br s, 1H), 9.83 (s, 1H), 8.97 (br s, 1H), 7.92 (br s, 1H), 7.84 (s, 1H), 7.71-7.69 (m, 2H), 7.57-7.56 (m, 2H), 7.24-7.22 (m, 2H), 7.07-7.05 (m, 2H), 5.45 (s, 2H), 4.18-4.16 (m, 2H), 3.98 (s, 2H), 3.68-3.66 (m, 2H), 3.31 (s, 3H), 3.21 (t, *J* = 7.6 Hz, 1H), 1.68 (t, *J* = 7.2 Hz, 2H), 1.47 (sep, J *=* 6.6 Hz, 1H), 0.88-0.86 (m, 6H). ¹³C NMR (126 MHz, DMSO-*d₆*) *δ* ppm: 167.8, 166.3, 162.1, 143.7, 138.7, 131.9, 130.8, 128.7 (2C), 128.6 (2C), 123.2, 119.5 (2C), 114.3 (2C), 74.6, 70.1, 55.6, 52.4, 49.9, 38.1, 38.0, 25.7, 22.4, 22.3.

### 2-(hydroxycarbamoyl)-N-[4-[[4-[[(4-isopropoxyphenyl)sulfonylamino]methyl] triazol-1-yl]methyl]phenyl]-4-methyl-pentanamide (206)

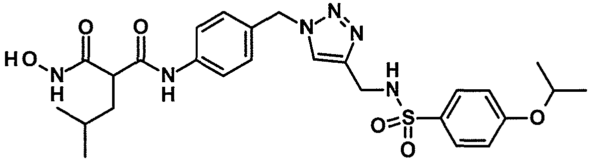

Compound **206** was synthesized according to the general procedure D, using ester **200** (90 mg, 0.16 mmol), KCN (2 mg, 0.03 mmol) and aq. NH₂OH (2 mL, 50% w/w in water) in MeOH (2 mL) overnight. The crude product was purified by flash chromatography on silica gel (CH₂Cl₂ to CH₂Cl₂/MeOH: 95/5) affording compound **206** as a white solid after lyophilization (21 mg, 24%). Purity: 100%. MS (ESI+): m/z = 559 [M + H]⁺. ¹H NMR (500 MHz, DMSO-*d₆*) *δ* ppm: 10.53 (br s, 1H), 9.82 (br s, 1H), 8.97 (br s, 1H), 7.90 (br s, 1H), 7.85 (s, 1H), 7.72-7.63 (m, 2H), 7.61-7.53 (m, 2H), 7.28-7.19 (m, 2H), 7.09-6.99 (m, 2H), 5.45 (s, 2H), 4.76-4.66 (m, 1H), 3.98 (s, 2H), 3.26-3.16 (m, 1H), 1.77-1.62 (m, 2H), 1.56-1.41 (m, 1H), 1.35-1.24 (m, 6H), 0.87 (s, 6H). ¹³C NMR (126 MHz, DMSO-*d₆*) δ ppm: 168.3, 166.7, 160.9, 144.2, 139.2, 131.9, 131.2, 129.3 (2C), 129.1 (2C), 123.6, 120.0 (2C), 115.9 (2C), 70.3, 52.8, 50.4, 38.6, 38.5, 26.2, 22.9, 22.7, 22.1 (2C).

### 2-(hydroxycarbamoyl)-N-[4-[[4-[[(4-isopropylphenyl)sulfonylamino]methyl] triazol-1-yl]methyl]phenyl]-4-methyl-pentanamide (207)

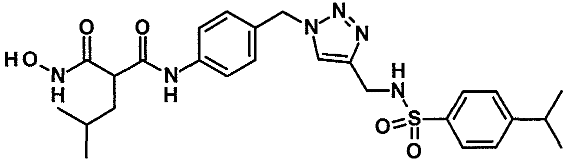

Compound **207** was synthesized according to the general procedure D, using ester **201** (125 mg, 0.22 mmol), KCN (3 mg, 0.05 mmol) and aq. NH₂OH (2.5 mL, 50% w/w in water) in MeOH (2.5 mL) overnight. The crude product was purified by flash chromatography on silica gel (CH₂Cl₂ to CH₂Cl₂/MeOH: 95/5) affording compound **207** as a white solid after lyophilization (26 mg, 21%). Purity: 97%. MS (ESI+): m/z = 543 [M + H]⁺. ¹H NMR (500 MHz, DMSO-*d₆*) *δ* ppm: 10.43 (br s, 1H), 7.85 (s, 1H), 7.74-7.65 (m, 2H), 7.60-7.52 (m, 2H), 7.45-7.38 (m, 2H), 7.27-7.19 (m, 2H), 5.44 (s, 2H), 4.00 (s, 2H), 3.11-3.03 (m, 1H), 3.01-2.91 (m, 1H), 1.73-1.54 (m, 2H), 1.54-1.44 (m, 1H), 1.29-1.14 (m, 6H), 0.86 (s, 6H). ¹³C NMR (126 MHz, DMSO-*d₆*) *δ* ppm: 169.2, 165.8, 153.1, 143.7, 138.9, 137.8, 130.4, 128.6 (2C), 127.0 (2C), 126.7 (2C), 123.2, 119.3 (2C), 52.4, 49.8, 38.9, 38.1, 33.4, 25.6, 23.5 (2C), 22.6, 22.1.

### tert-butyl N-[4-[[1-[[4-[[2-(hydroxycarbamoyl)-4-methyl-pentanoyl]amino]phenyl] methyl]triazol-4-yl]methylsulfamoyl]phenyl]carbamate (208)

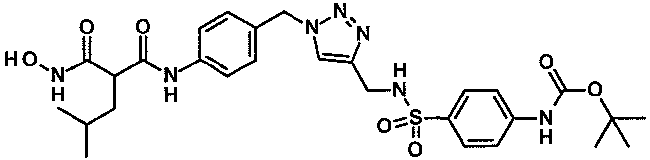

Compound **208** was synthesized according to the general procedure D, using ester **202** (150 mg, 0.24 mmol), KCN (4.6 mg, 0.07 mmol) and aq. NH₂OH (2 mL, 50% w/w in water) in MeOH (2 mL) overnight. The crude product was purified by flash chromatography on silica gel (CH₂Cl₂ to CH₂Cl₂/MeOH: 95/5) affording compound **208** as a white solid after lyophilization (72 mg, 49%). Purity: 100%. MS (ESI+): m/z = 616 [M + H]⁺, 560 [M - tBu + H]⁺, 516 [M - Boc + H]⁺. ¹H NMR (500 MHz, DMSO-*d₆*) *δ* ppm: 10.51 (br s, 1H), 9.81 (s, 1H), 9.80 (s, 1H), 8.97 (s, 1H), 7.89 (t, *J* = 5.5 Hz, 1H), 7.83 (s, 1H), 7.67-7.65 (m, 2H), 7.62-7.60 (m, 2H), 7.57-7.55 (m, 2H), 7.25-7.23 (m, 2H), 5.44 (s, 2H), 3.96 (d, *J* = 5.0 Hz, 2H), 3.20 (t, *J* = 7.6 Hz, 1H), 1.68 (t, *J* = 7.2 Hz, 2H), 1.48 (s, 9H), 1.47-1.44 (m, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.8, 166.3, 152.6, 143.7, 143.3, 138.7, 132.9, 130.7, 128.7 (2C), 127.8 (2C), 123.2, 119.6 (2C), 117.6 (2C), 79.9, 52.4, 49.9, 38.1, 38.0, 28.0 (3C), 25.7, 22.4, 22.2.

### 2-(hydroxycarbamoyl)-N-[4-[[4-[[(4-isopropylphenyl)sulfonylamino]methyl] triazol-1-yl]methyl]phenyl]-4-methyl-pentanamide (209)

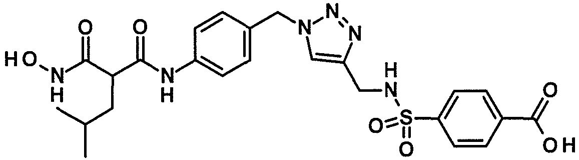

Compound **204** (35 mg, 0.06 mmol) was dissolved in THF (1 mL). A solution of LiOH (2.8 mg, 0.06 mg, 1 eq.) in H₂O (1 mL) was added dropwise. The mixture was stirred overnight. Solvents were removed under reduced pressure and the residue was dissolved in DMSO and filtered, before purification by preparative HPLC (H2O+0.05% FA/ MeCN+0.05% FA: 95:5 to 5:95) to give compound **209** as a white powder after lyophilization (22 mg, 66%). Purity: 100%. MS (ESI+): m/z = 545 [M + H]⁺. ¹H NMR (500 MHz, DMSO-*d₆*) *δ* ppm: 13.43 (br s, 1H), 10.53 (s, 1H), 9.81 (s, 1H), 8.98 (br s, 1H), 8.29 (t, J = 5.4 Hz, 1H), 8.12-8.06 (m, 2H), 7.90-7.86 (m, 2H), 7.86 (s, 1H), 7.59-7.54 (m, 2H), 7.25-7.20 (m, 2H), 5.44 (s, 2H), 4.06 (d, J = 5.8 Hz, 2H), 3.20 (t, J = 7.6 Hz, 1H), 1.68 (t, J = 7.3 Hz, 2H), 1.47 (sept, J = 6.7 Hz, 1H), 0.91-0.84 (m, 6H). ¹³C NMR (126 MHz, DMSO-*d₆*) *δ* ppm: 167.8, 166.3, 166.3, 144.0, 143.4, 138.7, 134.2, 130.7, 130.0 (2C), 128.7 (2C), 126.8 (2C), 123.2, 119.6 (2C), 52.3, 49.9, 38.1, 38.0, 25.7, 22.4, 22.2.

### N-[4-[[4-[[(4-aminophenyl)sulfonylamino]methyl]triazol-1-yl]methyl]phenyl]-2-(hydroxycarbamoyl)-4-methyl-pentanamide (210)

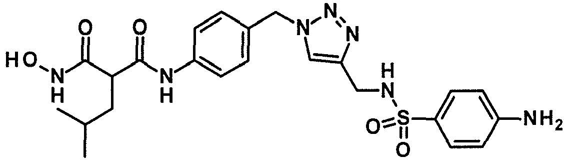

Compound **210** was synthesized according to the general procedure G, using the Boc-protected intermediate **208** (65 mg, 0.1 mmol), 4 N HCl in dioxane (2 mL) in a MeOH (2 mL) during 2 hours. The residue was purified through preparative HPLC (H₂O+0.05%FA/ ACN+0.05%FA: 95:5 to 5:95) to give compound **210** as a white solid after lyophilization (16 mg, 30%). Purity: 100%. MS (ESI⁻): m/z = 514 [M - H]⁻. ¹H NMR (500 MHz, DMSO-*d₆*) *δ* ppm: 10.53 (s, 1H), 9.81 (s, 1H), 8.97 (s, 1H), 7.81 (s, 1H), 7.57-7.54 (m, 3H), 7.41-7.39 (m, 2H), 7.26-7.24 (m, 2H), 6.60-6.58 (m, 2H), 5.94 (s, 2H), 5.45 (s, 2H), 3.90 (d, *J* = 6.1 Hz, 2H), 3.21 (t, *J* = 7.6 Hz, 1H), 1.69-1.66 (m, 2H), 1.47 (sep, J = 6.6 Hz, 1H), 0.87 (d, *J* = 6.6 Hz, 3H), 0.86 (d, *J* = 6.6 Hz, 3H). ¹³C NMR (126 MHz, DMSO-*d₆*) *δ* ppm: 167.8, 166.3, 152.6, 144.1, 138.7, 130.8, 128.7 (2C), 128.6 (2C), 125.1, 123.1, 119.6 (2C), 112.6 (2C), 52.4, 49.9, 38.2, 38.0, 25.7, 22.4, 22.2.

### 2-ethoxycarbonyl-5-methyl-hexanoic acid (211-a)

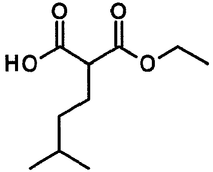

Compound **211-a** was synthesized according to the general procedure F, using diethylmalonate (2000 mg, 8.68 mmol) and NaOH (416.6 mg, 10.42 mmol) in EtOH/H₂O (20 mL, 16:4 v/v) overnight. Compound **211-a** was obtained as colorless oil (1500 mg, 85%) and was used in the next step without further purification. MS (ESI+): m/z = 203 [M + H]⁺. ¹H NMR (DMSO-*d₆*) *δ* ppm: 12.75 (br s, 1H), 4.10 (q, *J* = 7.1 Hz, 2H), 3.27 (t, J = 7.5 Hz, 1H), 1.76-1.67 (m, 2H), 1.51 (sept, J = 6.6 Hz, 1H), 1.17 (t, J = 7.1 Hz, 3H), 1.15-1.08 (m, 2H), 0.84 (d, *J* = 6.6 Hz, 6H). ¹³C NMR (DMSO-*d₆*) *δ* ppm: 170.5, 169.5, 60.6, 51.5, 35.8, 27.3, 26.3, 22.3, 22.3, 14.0.

### 3-methoxy-2-methyl-3-oxo-propanoic acid (212-a)

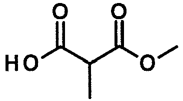

Compound **212-a** was synthesized according to the general procedure F, using dimethylmalonate (1300 mg, 8.9 mmol) and NaOH (427 mg, 10.7 mmol) in MeOH/H₂O (20 mL, 16:4 v/v) overnight. Compound **212-a** was obtained as colorless oil (1020 mg, 85%) and was used in the next step without further purification. MS (ESI+): no ionization. ¹H NMR (500 MHz, DMSO-*d₆*) *δ* ppm: 12.83 (s, 1H), 3.64 (s, 3H), 3.45 (q, *J= 10.8* Hz, 1H), 1.24 (d, *J* = 7.2 Hz, 3H). ¹³C NMR (126 MHz, DMSO-*d₆*) δ ppm: 171.2, 170.7, 52.1, 45.4, 13.6.

### ethyl 2-[[4-(azidomethyl)phenyl]carbamoyl]-5-methyl-hexanoate (211)

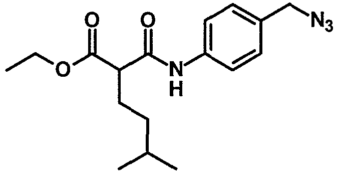

Compound **211** was synthesized according to the general procedure B, using carboxylic acid **211-a** (300 mg, 0.67 mmol), aniline **171** (148.4 mg, 0.8 mmol), HBTU (379.2 mg, 1.0 mmol) and trimethylamine (460 µL, 3.35 mmol) in DMF (3 mL) overnight. The crude product was purified by flash chromatography on silica gel (cHex to cHex/EtOAc: 8/2) affording compound **211** as a yellow oil (93 mg, 42%). MS (ESI+): m/z = 333 [M + H]⁺.

### methyl 3-[4-(azidomethyl)anilino]-2-methyl-3-oxo-propanoate (212)

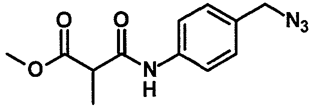

Compound **212** was synthesized according to the general procedure B, using carboxylic acid **212-a** (250 mg, 1.89 mmol), aniline **171** (419 mg, 2.27 mmol), HBTU (1080 mg, 2.84 mmol) and trimethylamine (1290 µL, 9.46 mmol) in DMF (5 mL) overnight. The crude product was purified by flash chromatography on silica gel (cHex to cHex/EtOAc: 5/5) affording compound **212** as a pale yellow solid (82 mg, 16%). MS (ESI+): m/z = 263 [M + H]⁺. ¹H NMR (500 MHz, DMSO-*d₆*) *δ* ppm: 10.28 (s, 1H), 7.62-7.60 (m, 2H), 7.33-7.31 (m, 2H), 4.38 (s, 2H), 3.64 (s, 3H), 3.63-3.60 (m, 1H), 1.31 (d, *J* = 7.1 Hz, 3H). ¹³C NMR (126 MHz, DMSO-*d₆*) *δ* ppm: 170.8, 168.1, 138.8, 130.5, 129.2 (2C), 119.3 (2C), 53.2, 52.1, 46.7, 13.9.

### N-[4-(azidomethyl)phenyl]-2-(hydroxycarbamoyl)-5-methyl-hexanamide (214)

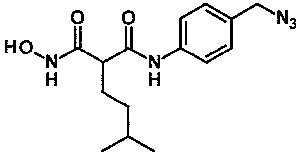

Compound **214** was synthesized according to the general procedure D, using ester **83** (85 mg, 0.26 mmol), KCN (3.4 mg, 0.05 mmol) and aq. NH₂OH (2 mL, 50% w/w in water) in MeOH (2 mL) 48 hours. The crude product was purified by flash chromatography on silica gel (CH₂Cl₂ to CH₂Cl₂/MeOH: 9/1) affording compound **214** as a beige powder (45 mg, 53%). MS (ESI+): m/z = 320 [M + H]⁺. ¹H NMR (500 MHz, DMSO-*d₆*) *δ* ppm: 10.56 (s, 1H), 9.90 (s, 1H), 8.99 (s, 1H), 7.62-7.60 (m, 2H), 7.32-7.30 (m, 2H), 4.37 (s, 2H), 3.08 (t, *J* = 7.2 Hz, 1H), 1.80-1.75 (m, 2H), 1.54-1.49 (m, 1H), 1.13-1.08 (m, 2H), 0.85 (d, *J* = 6.3 Hz, 6H). ¹³C NMR (126 MHz, DMSO-*d₆*) *δ* ppm: 167.9, 166.2, 138.8, 130.3, 129.2 (2C), 119.4 (2C), 53.3, 51.8, 36.2, 27.5, 27.1, 22.5 (2C).

### 2-(hydroxycarbamoyl)-N-[4-[[4-[[(4-iodophenyl)sulfonylamino]methyl]triazol-1-yl]methyl]phenyl]-5-methyl-hexanamide (215)

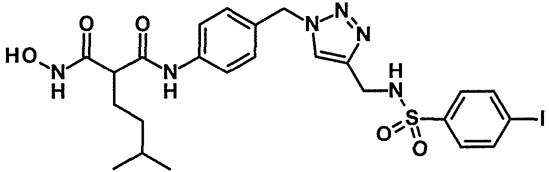

Compound **215** was synthesized according to the general procedure E, using azide **214** (40 mg, 0.13 mmol), alkyne **117** (41.7 mg, 0.13 mmol), copper (II) sulfate pentahydrate (6.5 mg, 0.03 mmol) and sodium ascorbate (12.9 mg, 0.07 mmol) in DMF (3 mL) and H₂O (2 mL) overnight. The crude product was purified by flash chromatography on silica gel (CH₂Cl₂ to CH₂Cl₂/MeOH: 9/1) affording compound **214** as a white solid after lyophilization (23 mg, 30%). Purity: 100%. MS (ESI+): m/z = 641 [M + H]⁺. ¹H NMR (500 MHz, DMSO-*d₆*) *δ* ppm: 10.51 (br s, 1H), 9.83 (s, 1H), 8.96 (s, 1H), 8.18 (br s, 1H), 7.93-7.91 (m, 2H), 7.84 (s, 1H), 7.58-7.57 (m, 2H), 7.51-7.50 (m, 2H), 7.25-7.23 (m, 2H), 5.45 (s, 2H), 4.03 (s, 2H), 3.05 (t, *J* = 7.3 Hz, 1H), 1.80-1.74 (m, 2H), 1.54-1.49 (m, 1H), 1.15-1.08 (m, 2H), 0.85 (d, *J* = 6.4 Hz, 6H). ¹³C NMR (126 MHz, DMSO-*d₆*) *δ* ppm: 167.8, 166.2, 143.4, 140.0, 138.7, 138.0 (2C), 130.7, 128.7 (2C), 128.3 (2C), 123.2, 119.5 (2C), 100.4, 52.4, 51.8, 38.0, 36.1, 27.4, 27.2, 22.4 (2C).

### methyl 3-[4-[[4-[[(4-iodophenyl)sulfonylamino]methyl]triazol-1-yl]methyl] anilino]-2-methyl-3-oxo-propanoate (216)

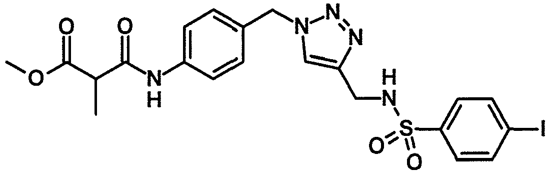

Compound **216** was synthesized according to the general procedure E, using azide **212** (70 mg, 0.27 mmol), alkyne **117** (85.6 mg, 0.27 mmol), copper (II) sulfate pentahydrate (6.5 mg, 0.03 mmol) and sodium ascorbate (24.6 mg, 0.13 mmol) in DMF (5.5 mL) and H₂O (4.5 mL) overnight. The crude product was purified by flash chromatography on silica gel (CH₂Cl₂ to CH₂Cl₂/MeOH: 98/2) affording compound **216** as a white solid (146 mg, 92%). MS (ESI+): m/z = 584 [M + H]⁺. ¹H NMR (500 MHz, DMSO-*d₆*) *δ* ppm: 10.27 (s, 1H), 8.19 (t, *J* = 5.8 Hz, 1H), 7.93-7.92 (m, 2H), 7.84 (s, 1H), 7.59-7.57 (m, 2H), 7.51-7.50 (m, 2H), 7.26-7.24 (m, 2H), 5.45 (s, 2H), 4.03 (d, *J* = 5.8 Hz, 2H), 3.63 (s, 3H), 3.61-3.58 (m, 1H), 1.30 (d, *J =* 7.1 Hz, 3H). ¹³C NMR (126 MHz, DMSO-*d₆*) δ ppm: 170.7, 168.1, 143.4, 140.0, 138.8, 138 (2C), 130.8, 128.8 (2C), 128.3 (2C), 123.2, 119.4 (2C), 100.4, 52.4, 52.1, 46.7, 38.0, 13.9.

### 3-(hydroxyamino)-N-[4-[[4-[[(4-iodophenyl)sulfonylamino]methyl]triazol-1-yl] methyl]phenyl]-2-methyl-3-oxo-propanamide (217)

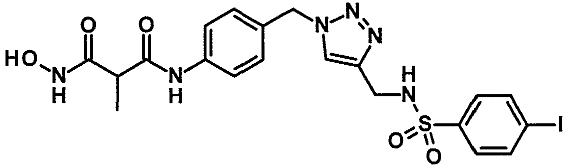

Compound **217** was synthesized according to the general procedure D, using ester **216** (139 mg, 0.24 mmol), KCN (3.1 mg, 0.05 mmol) and aq. NH₂OH (2 mL, 50% w/w in water) in MeOH (2 mL) overnight. The crude product was purified by flash chromatography on silica gel (CH₂Cl₂ to CH₂Cl₂/MeOH: 9/1) affording compound **217** as a beige powder (72 mg, 52%). Purity: 96%. MS (ESI+): m/z = 585 [M + H]⁺. ¹H NMR (500 MHz, DMSO-*d₆*) *δ* ppm: 9.99 (s, 1H), 9.31 (br s, 2H), 8.26 (s, 1H), 7.93-7.91 (m, 2H), 7.83 (s, 1H), 7.59-7.57 (m, 2H), 7.51-7.49 (m, 2H), 7.24-7.23 (m, 2H), 5.44 (s, 2H), 4.03 (m, 2H), 3.29-3.25 (m, 1H), 1.26 (d, *J* = 7.1 Hz, 3H). ¹³C NMR (126 MHz, DMSO-*d₆*) *δ* ppm: 168.6, 166.9, 143.4, 140.1, 139.0, 138.0 (2C), 130.5, 128.7 (2C), 128.3 (2C), 123.2, 119.4 (2C), 100.4, 52.4, 45.2, 38.0, 14.3.

### II. Biology

### In vitro LasB activity assay:

The LasB activity assay was performed as described previously (Nishino N, Powers JC. Pseudomonas aeruginosa elastase. Development of a new substrate, inhibitors, and an affinity ligand. J Biol Chem. 1980 Apr 25;255(8):3482-6) using the fluorogenic substrate 2-Aminobenzoyl-L-Alanyl-Glycyl-L-Leucyl-L-Alanyl-para-Nitro-Benzyl-Amide, purchased from Peptides International (Louisville, KY, USA) and vivitide, LLC (Gardner, MA, USA).

Fluorescence intensity was measured for 60 min at 37 °C in black 384-well microtiter plates (Greiner BioOne, Kremsmünster, Austria) using a CLARIOstar microplate reader (BMG Labtech, Ortenberg, Germany) with an excitation wavelength of 340 ± 15 nm and an emission wavelength of 415 ± 20 nm. The assay was performed in a final volume of 50 µL of assay buffer (50 mM Tris, pH 7.2, 2.5 mM CaCl₂, 0.075% Pluronic F-127, 5% DMSO) containing LasB at a final concentration of or 0.3 nM and the substrate at 150 µM. Before substrate addition, compounds were preincubated with the enzyme at 37 °C for 15 min. Experiments were performed in duplicates and repeated for at least two times. Blank controls without enzyme were performed. After blank subtraction, the slope of samples containing inhibitors (v) was divided by the slope of a simultaneously started uninhibited enzymatic reaction (v0). IC₅₀ values were determined with nonlinear regression using GraphPad Prism 5 (Graph Pad Software, San Diego, CA, USA) and are given as mean values ± standard deviation (SD). The slope factor was constrained to 1.

**Table1: Activity against LasB**

| **Compound** | **IC₅₀ LasB (nM)** |
|---|---|
| Z9 | 16.9 ± 0.6 |
| Hit L3 | 13.9 ± 0.3 |
| Hit L5 | 13.8 ± 0.3 |
| Hit L4 | 6.6 ± 0.2 |
| Hit L2 | 5.3 ± 0.1 |
| Hit L1 | 2.4 ± 0.1 |
| 131 | 2.3 ± 0.1 |
| 139 | 19.3 ± 0.6 |
| 132 | 22.8 ± 0.1 |
| 133 | 9.8 ± 0.4 |
| 140 | 55.9 ± 2.3 |
| 134 | 8.4 ± 0.5 |
| 135 | 9.8 ± 0.3 |
| 136 | 1.4 ± 0.1 |
| 137 | 16.9 ± 1.3 |
| 138 | 37.8 ± 2.9 |
| 160 | 3.8 ± 0.1 |
| 161 | 1.7 ± 0.1 |
| 162 | 1.9 ± 0.1 |
| 163 | 4.3 ± 0.1 |
| 164 | 2.4 ± 0.1 |
| 165 | 1.6 ± 0.1 |
| 166 | 5.2 ± 0.3 |
| 167 | 5.5 ± 0.3 |
| 168 | 2.7 ± 0.2 |
| 169 | 5.2 ± 0.2 |
| 182 | 1.9 ± 0.1 |
| 183 | 9.9 ± 0.3 |
| 179 | 1.0 ± 0.6 |
| 185 | 6.5 ± 0.2 |
| 190 | 7.8 ± 0.1 |
| 191 | 7.9 ± 0.1 |
| 192 | 6.8 ± 0.1 |
| 203 | 7.9 ± 0.2 |
| 204 | 13.0 ± 0.4 |
| 205 | 29.5 ± 0.7 |
| 206 | 7.8 ± 0.3 |
| 207 | 3.1 ± 0.1 |
| 208 | 4.2 ± 0.2 |
| 209 | 44.6 ± 1.1 |
| 210 | 7.7 ± 0.2 |
| 215 | 48.5 ± 1.4 |
| 217 | 278.3 ± 9.7 |

### MIC determination assay:

Assays regarding the determination of the minimum inhibitory concentration (MIC) were performed as described previously (Elgaher WAM, Fruth M, Groh M, Haupenthal J, Hartmann RW. Expanding the scaffold for bacterial RNA polymerase inhibitors: design, synthesis and structure-activity relationships of ureido-heterocyclic-carboxylic acids. RSC Adv. 2013 Dec 3;4(5):2177-94). The experiments were based on the P. *aeruginosa* strain PA14. For the case that no MIC value could be determined due to activity reasons, percentage (%) inhibition at 100 µM (or lower, depending on the solubility of the compounds) was calculated. The OD₆₀₀ was measured 0 and 16 h after inhibitor addition in a CLARIOstar Platereader (BMG Labtech, Ortenberg, Germany).

### Kinetic Turbidimetric solubility assay:

The desired compounds were sequentially diluted in DMSO in a 96-well plate. 1.5 µL of each well were transferred into another 96-well plate and mixed with 148.5 µL of PBS. Plates were shaken for 5 min at 600 rpm at room temperature, and the absorbance at 620 nm was measured. Absorbance values were normalized by blank subtraction and plotted using GraphPad Prism 8.4.2 (GraphPad Software, San Diego, CA, USA). Solubility (S) was determined based on the First X value of AUC function using a threshold of 0.005.

### Metabolic stability in Mouse Liver S9 Fraction assay:

For the evaluation of combined phase I and phase II metabolic stability, the compound (1 µM) was incubated with 1 mg/mL pooled liver S9 fraction (Xenotech), 2 mM NADPH, 1 mM UDPGA, 10 mM MgCl2, 5 mM GSH and 0.1 mM PAPS at 37 °C for 0, 5, 15, 30 and 60 min. The metabolic stability of Testosterone (1 µM), verapamil (1 µM) and ketoconazol (1 µM) were determined in parallel to confirm the enzymatic activity of the S9 fraction. The incubation was stopped by precipitation of S9 enzymes with 2 volumes of cold acetonitrile containing internal standard (150 nM Diphenhydramine). Samples were stored on ice for 10 min and precipitated protein was removed by centrifugation (15 min, 4°C, 4,000 rpm). The remaining test compound at different time points was analyzed by LC-MS/MS (TSQ Quantum Access MAX, Thermo Fisher, Dreieich, Germany) and used to determine half-life (t1/2).

**Table 2: Comparison study between Hit L1, 131 and 136 according to their activity, solubility and metabolic stability in mouse**

| **Compound** | **Structure** | **IC₅₀ LasB (nM)** | **Solubility (µM)** | **Met Stab in mouse liver S9 t_{1/2} [min]** |
|---|---|---|---|---|
| **Hit L1** | 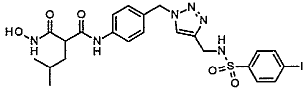 | 2.4 ± 0.1 | 106 ± 19 | 30.1 ± 1.5 |
| **131** | 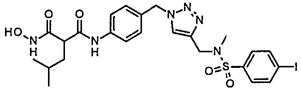 | 2.3 ± 0.1 | < 200 | 37.5 ± 2.5 |
| **136** | 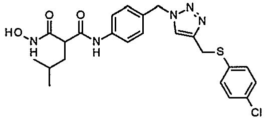 | 1.4 ± 0.1 | < 200 | 3.3 ± 0.2 |

**Table 3: Comparative study between Hit L1, 160, 161, 165 and 169 according to their activity, solubility, clogP and metabolic stability in mouse. *cLogP values were calculated using Datawarrior.**

| 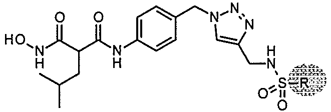 | | | | | |
|---|---|---|---|---|---|
| **Compound** | **R** | **IC₅₀ LasB (nM)** | **cLogP*** | **Solubility [µM]** | **Met Stab in mouse liver S9 t_{1/2} [min]** |
| **Hit L1** | 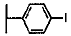 | 2.4 ± 0.1 | 1.5 | 142 | 30.1 ± 1.5 |
| **161** | 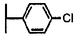 | 1.7 ± 0.1 | 1.6 | > 200 | 33.9 ± 1.0 |
| **165** | 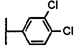 | 1.6 ± 0.1 | 2.2 | 117 | 10.7 ± 0.6 |
| **160** | 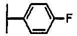 | 3.8 ± 0.1 | 1.1 | > 200 | 65.5 ± 0.7 |
| **169** | 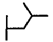 | 5.2 ± 0.2 | 0.9 | > 200 | > 120 |

### Selectivity assay:

### Inhibition of MMPs

MMPs -1, -2, -3, -7, -8 and -14 along with the SensoLyte 520 Generic MMP Activity Kit*Fluorimetric* were purchased from AnaSpec (Fremont, CA, USA). The assay was performed as described previously (Schönauer E, Kany AM, Haupenthal J, Hüsecken K, Hoppe IJ, Voos K, et al. Discovery of a Potent Inhibitor Class with High Selectivity toward Clostridial Collagenases. J Am Chem Soc. 2017 Sep 13;139(36):12696-703) using batimastat as a positive control according to the guidelines of the manufacturer.

### HDAC inhibition

HDAC3 and HDAC8 Inhibitor Screening Assay kits were purchased from Sigma-Aldrich. The assay was performed according to the guidelines of the manufacturer using trichostatin as a positive control. Fluorescence signals were measured in a CLARIOstar plate reader (BMG Labtech).

### TACE

The ADAM-17 (TACE) Inhibitor Screening Assay Kit was purchased from Sigma-Aldrich. The assay was performed according to the guidelines of the manufacturer using ilomastat as a positive control. Fluorescence signals were measured in a CLARIOstar plate reader (BMG Labtech).

### COX-1

COX-1 Inhibitor Screening Assay Kit was purchased from Abcam. The assay was performed according to the guidelines of the manufacturer. Fluorescence signals were measured in a CLARIOstar plate reader (BMG Labtech).

### Cytotoxicity assay:

HepG2, HEK293 or A549 cells (2 × 10⁵ cells per well) were seeded in 24-well, flat-bottomed plates. Culturing of cells, incubations, and OD measurements were performed as described previously (Int. J. Cancer 2007, 121, 206-210). Twenty-four hours after seeding the cells, the incubation was started by the addition of test compound in a final DMSO concentration of 1%. The living cell mass was determined after 48 h. At least two independent measurements were performed for each compound.

**Table 4: Selectivity and cytotoxicity profiles of Hit L1, 160, 169 and Z9 with their antibacterial activities against P.aeruginosa PA-14.**

| | | | **Hit L1** | **160** | **169** | **Z9** |
|---|---|---|---|---|---|---|
| **Selectivity IC₅₀ (µM)** | | **MMP1** | > 100 | > 100 | > 100 | > 100 |
| | | **MMP2** | > 100 | > 100 | > 100 | > 100 |
| | | **MMP3** | > 100 | > 100 | > 100 | > 100 |
| | | **HDAC3** | > 100 | > 100 | > 100 | > 100 |
| | | **HDAC8** | > 100 | > 100 | > 100 | > 100 |
| | | **TACE** | > 100 | > 100 | > 100 | 1.6 ± 0.2 |
| | | **COX-1** | > 100 | > 100 | > 100 | > 100 |
| **Cytotoxicity IC₅₀ (µM)** | | **HepG2** | > 100 | > 100 | > 100 | > 100 |
| | | **HEK293** | > 100 | > 100 | > 100 | > 100 |
| | | **A549** | > 100 | > 100 | > 100 | > 100 |
| **Antibacterial activity IC₅₀ (µM)** | | **PA-14** | > 100 | > 100 | > 100 | > 100 |

### Transepithelial Electric Resistance (TEER) Experiments

*With Calu-3 cell line.* Human lung cancer cell line (Calu-3) (HTB-55^{™}; ATCC) passaged 19 to 25 were seeded at a density of 3 × 10⁴ cells/mL onto a hanging cell culture insert at 37 °C for 10 days with 5% CO₂. Every 2 days, the medium (minimum essential medium containing 1% non-essential amino acids (NEAA, 40035),1 mM sodium pyruvate (11360070), 100 U/ml penicillin/streptomycin, 10% fetal calf serum (FCS)) was changed.

A concentration of 20% (v/v) of PAO1 culture supernatant was added together with 3 µM of compounds 169, Hit L1 or 160 into the inner compartment. The TEER of the cells was measured with Millicell ERS-2 (Electrical Resistance System) over time. Three readings were recorded for each well, Ohmic resistance values were corrected for the area of the insert (0.3 cm2) as well as the related value of a blank and reported relative to the control (no inhibitor, no supernatant treatments).

*With hAELVi cell line.* Human alveolar epithelial lentivirus immortalized hAELVi (Arlo) were seeded according to the reported procedure (Anna Kuehn, S. K.-W.-D.-M. (2016). Human alveolar epithelial cells expressing tight junctions to model the air-blood barrier. ALTEX.). The treatment with the PAO1 supernatant and compounds was performed as mentioned before.

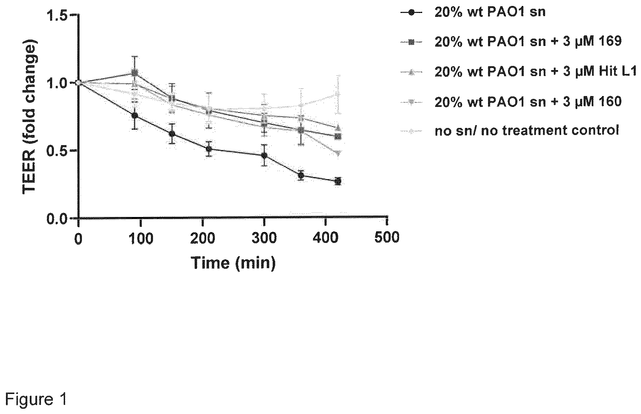

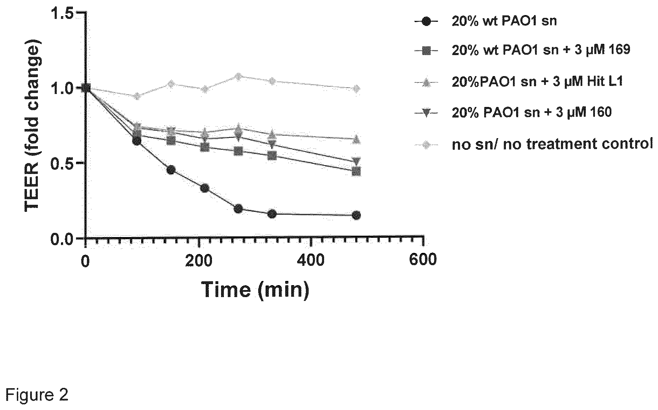

*LasB inhibitors can rescue the TEER value of Calu3 cells.* The hydroxamic acid-based compounds inhibited the activity of LasB and maintained the TEER of the challenged cells with PAO1 supernatant as shown in Figures 1 and 2.

Figure 1 shows the change in the transepithelial electrical resistance (TEER) of human lung cancer cell line (Calu3) cells challenged with 20% (v/v) PAO1 culture supernatant and treated with or without hydroxamic acid LasB inhibitors. Each curve represents the average ± standard deviation of at least three independent experiments.

Figure 2 shows the change in the transepithelial electrical resistance (TEER) of human alveolar epithelial lentivirus immortalized (hALVi) cells challenged with 20% (v/v) PAO1 culture supernatant and treated with or without hydroxamic acid LasB inhibitors. Each curve represents the average ± standard deviation of data derived from one experiment.

### In vitro evaluation of compound 169 through an A549 cell-based assay

The human lung adenocarcinoma cell line (A549) was cultured in Dulbecco's Modified Eagle Medium (DMEM), containing 10% Fetal Bovine Serum (FBS) and 1% penicillin-streptomycin mixture. Cells were maintained according to standard cell culture procedures.

To conduct the assay, 2.5-3.5*10³ cells/well were seeded into a flat bottom 96-well plate (Corning^{™} Costar^{™}) and incubated at 37°C + 5% CO₂ for 24 hours. On the following day, several concentrations of the compound **169** were tested against 10% (v/v) *Pseudomonas aeruginosa* PAO1 (DSM 22644, ATCC 15692) culture supernatant diluted in DMEM starting from 1.25 µM. The compound was initially dissolved in 99.9% Dimethyl sulfoxide (DMSO) and a final assay concentration of 0.5% (v/v) was applied to minimize the proteolytic effect of DMSO on the bacterial culture supernatant. In addition, cells were challenged with 10% (v/v) of *ΔIasB* PAO1 to confirm the toxic effect derived from LasB, and DMEM was included as a control without any treatment. Plates were incubated at 37°C + 5% CO₂ for 24 hours prior to the MTT assay.

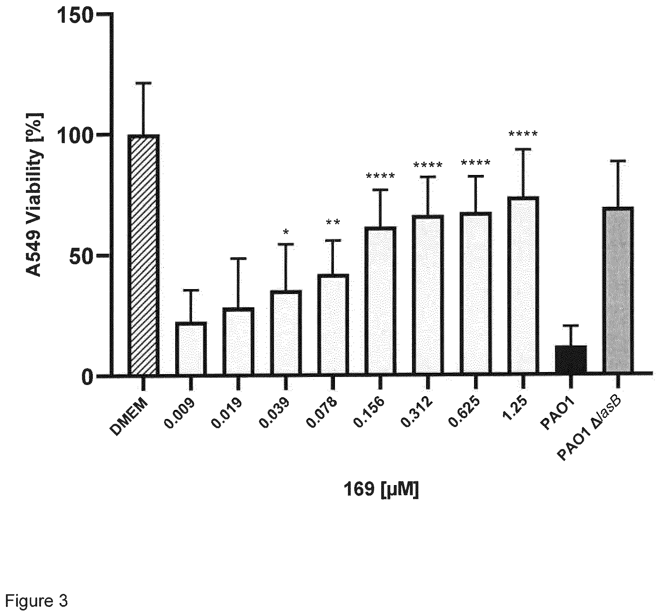

The ability of the hydroxamic acid **169** to reduce the LasB-related cytotoxicity in the A549 cell line was evaluated. This compound demonstrated excellent potency in PAO1 csn-treated cells (Figure 3). An average cell viability of 12% and 69% was observed when cells were challenged with PAO1 and *ΔIasB* PAO1 csn, respectively. As expected, improvements in cell viability were achieved within this range, proving the selectivity of the compound towards LasB. Dose-dependent inhibition of LasB in a low micromolar to nanomolar range can be observed in the application of **169.**

Figure 3 shows the dose-response inhibitory effect of **169** against 10% (v/v) P. *aeruginosa* PAO1 csn, targeting LasB. The graph represents three independent experiments ± SD. One-way ANOVA statistical analysis was performed following Dunnett's multiple comparisons test, comparing the mean value of each concentration to the mean value of PAO1 without any treatment with compounds (**** p ≤ 0.0001, ** p ≤ 0.01, * p ≤ 0.05).

### Toxicity studies of PAO1 (DSM 22644) in G. mellonella

Bacteria were grown overnight, diluted to OD₆₀₀ of 0.025 in 10 ml medium, and grown for approx. 2 hours at 37°C inside a shaker incubator until the OD₆₀₀ of 1.8-2 is reached. The culture was then serially diluted in sterile PBS (pH=7.2). Bacterial densities of 3000, 300, 30, and 3 CFU/Larva were selected for injection into groups of 15 larvae. A total volume of 5 µl was injected into each larva via the hindmost left proleg by using a syringe pump and Sterican^{®} needles (0.30 x 12 mm, 30G x ½). In addition, control group without injection for the quality of the larvae and a PBS control to monitor the effect of the injection were included in the experiment. All test groups and control groups were incubated at 37°C in the dark and monitored every 2 hours from 17 hours post-infection. Larvae were considered dead when no movement was observed in response to touch or when melanization of the cuticle occurred.

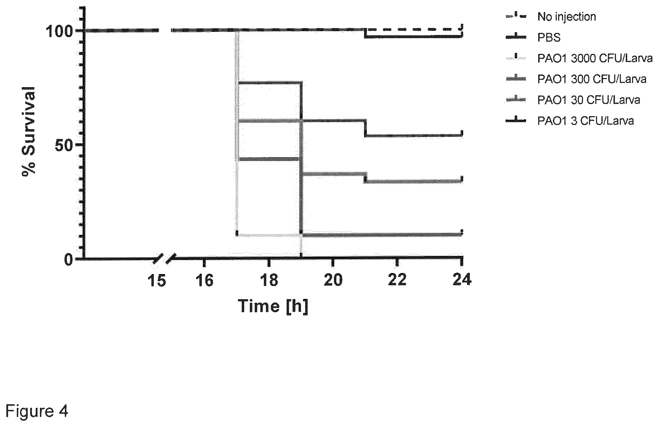

As shown in Figure 4, *P. aeruginosa* PAO1 (strain DSM22644) caused a lethal effect in a dose-response manner, proving this model to be suitable for further studies on inhibitors. The final percentage of survival of the groups infected with 3000, 300, 30, and 3 CFU/Larva was 0%, 10%, 33%, and 53%, respectively. Simultaneously, a 97-100% survival was observed in the no-injection and PBS control groups, demonstrating the significance of the survival drop caused by bacterial infection. 3 CFU/Larva was selected for further survival studies.

Figure 4 shows the Kaplan-Meier survival analysis of larvae infected with various CFUs of *P. aeruginosa* PAO1 (strain DSM22644). The graph represents three independent experiments. The experiment was conducted over 24 hours and each group was monitored every 2 hours from 17 hours post-infection (p<0.0001).

*In vivo evaluation of compounds* **162** *and* **169.** Bacteria were cultured and prepared according to the previous section. Final concentrations of 50 µM, 25 µM, and 12.5 µM in combination with 3 CFU/Larva were injected into groups of 15 larvae. In addition to no injection and PBS controls, one group was infected with 3 CFU/Larva and considered the negative control. All test groups and control groups were incubated at 37°C in the dark and monitored every 2 hours from 17 hours post-infection. Larvae were considered dead when no movement was observed in response to touch or when melanization of the cuticle occurred.

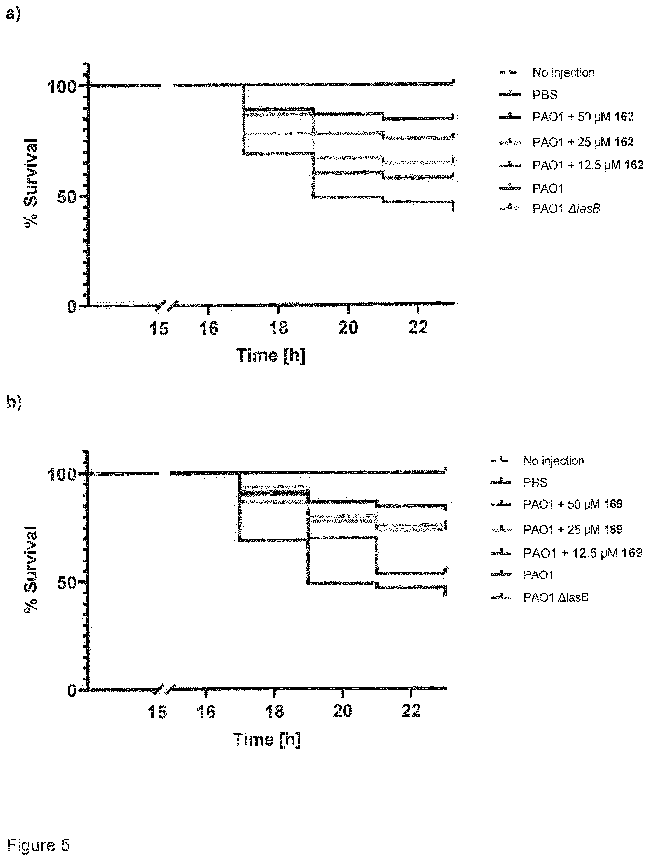

Several concentrations of compounds **162** and **169** were injected into larvae in combination with 3 CFU/Larva and the survival was monitored over the course of 24 hours (Figure 5). Treatment with 50 µM of **162** and **169** resulted in a final survival percentage of 84% and 82%, respectively. This shows a 40% improvement in the survival of the larvae compared to the negative control (3 CFU/Larva of bacteria without treatment). Treatment with further concentrations of 25 µM and 12.5 µM resulted in 64% and 58% for **162,** and 73% and 53% for **169,** respectively. In conclusion, significant survival improvement in a dose-response manner was observed in all of the test groups, suggesting both compounds as effective inhibitors against *P*. *aeruginosa.* Figure 5 shows the Kaplan-Meier survival analysis of larvae infected with 3 CFU/Larva of *P*. *aeruginosa* PAO1 (strain DSM22644) in combination with several concentrations of **162** and **169.** Each graph represents three independent experiments. The experiment was conducted over 24 h and each group was monitored every 2 h from 17 hours post-infection (p<0.0001).

### Preparation of lung homogenate

The lung homogenate was prepared from pig lung, which was stored at -80° C. After thawing the lung tissue was cut into pieces of approximately 1.5 cm diameter and 0.5 to 1 cm thickness. Subsequently the bits were flash frozen in liquid nitrogen and lyophilized for 48 h.

1.2 g dried tissue was then ball milled using an IKA Tube Drive and the respective 50 mL ball milling tubes. 20 steel balls were used with target rpm of 6000, reversing the spinning direction every 30 seconds for a total of 10 min. After 10 min the tube was detached and inverted to loosen the powder. The ball milling was repeated with the same setup twice again.

To the powdery tissue 50 mL of a 10 mM Tris buffer including 2 mM CaCl₂ with a pH of 8.0 was added and the IKA Tube Drive used again with the same settings as described before for 10 min to disperse the tissue powder in the aqueous buffer. The yielded lung homogenate was pipetted into 15 mL falcons and stored at -80° C until further use.

### Protocol of lung homogenate Assay

To perform the LasB activity/inhibition assay a compound dilution was prepared in DMSO yielding a 100 fold concentration of the final concentration in the assay. LasB was diluted from a higher stock solution to a 6 µM stock in the same buffer as used for the lung homogenate. The assay was performed in Eppis. With a stepper pipette 10 µL of the prepared LasB solution was added to the Eppis to yield a final protein concentration of 300 nM. Then 188 µL of thawed lung homogenate was added with a stepper pipette. Finally, 2 µL of the 100 fold compound dilutions were added and mixed by pipetting up and down. The Eppis were incubated at 37° C with a shaking speed of 1000 rpm for 4 h.

After 4 h, the Eppis were centrifuged at a speed of 14,000 rpm, 4° C for 10 min to separate the soluble cleaved Elastin fragments from the non-cleaved insoluble part. 100 µL of the obtained supernatant was transferred to new Eppis, and the same volume of concentrated hydrochloric acid added. For a complete hydrolysis and release of Desmosin the Eppis were again incubated at 100° C and 1000 rpm overnight.

The next day all Eppis were centrifuged for 1 minute at 14,000 rpm, 25° C and then opened and incubated at 100° C until completely dry.

To the dry Eppis 100 µL of a 100 mM ammonium formate buffer with pH 2.7 (50 % MeCN in H2O) containing 100 nM Amproliumchloride as internal standard was added. To improve solvation the Eppis were shaken for 20 min at 1500 rpm. Afterwards the solutions were centrifuged again at 14,000 rpm, 4° C for 10 min. The obtained supernatant was transferred to a 96 well plate to be submitted to LC-MSMS.

For LC-MSMS analysis a Dionex UltiMate 3000 was used consisting of a RS pump, RS autosampler and column compartment. The LC part is coupled to TSQ Quantum Access MAX Triple Quadrupole mass spectrometer. The column used is a Syncronis HILIC 50x2.1 with a particle size of 1.7 µ.

The mobile phase consisted of MS grade MeCN as B and 200 mM ammonium formate in water adjusted to pH 2.7 as A. The gradient started with 90% B for 1 min. Followed by a linear gradient from 90% B to 10% B for 6.5 min. This was followed by a plateau of 10% B for 0.5 min. From minute 8 to the end of the run minute 10 a plateau of 90% B was used to equilibrate the column for the next run.

Amprolium eluted at around 0.83 minutes, while Desmosine eluted around 4.44 minutes.

The detection of the molecules was achieved in SRM mode observing the following fragmentation patterns:
Amprolium 243.130 → 150.1
Desmosine 526.209 → 351.9
Desmosine 526.209 → 397.0
Desmosine 526.209 → 436.0
Desmosine 526.209 → 481.1

Peak areas and response ratios were calculated using Xcalibur Quan browser with Amprolium set as internal standard. The calculation of the IC50 -values was performed within Prism.

### Results of ex vivo lung homogenate assay with purified LasB

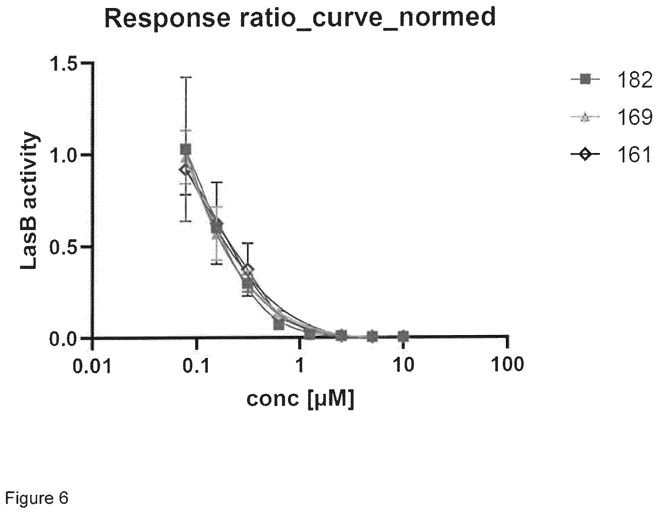

The results of the ex vivo lung homogenate assay with purified LasB are shown in Figure 6 and Table 5.

**Table 5: IC₅₀**

| **Compound** | **IC₅₀ [µM]** | **SD** |
|---|---|---|
| **182** | 0.127 | 0.088 |
| **169** | 0.077 | 0.080 |
| **161** | 0.186 | 0.058 |

## Claims

1. A compound of formula (II): 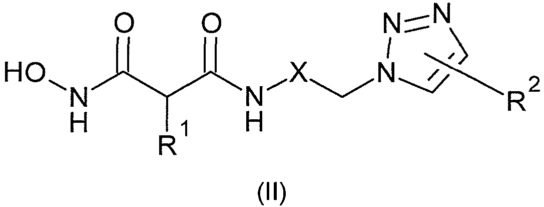 wherein
X is an optionally substituted arylene group or an optionally substituted heteroarylene group;
R¹ is a C₁₋₆ alkyl group; or a group of formula -CH₂-R¹¹, wherein R¹¹ is a phenyl group or a cyclopropyl group; and
R² is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a pharmaceutically acceptable salt thereof;
wherein the optional substituents are selected from fluorine, chlorine, bromine and iodine and OH, =O, SH, NH₂, -SO₃H, -SO₂NH₂, -COOH, -COOMe, -COMe, -NHSO₂Me, -SO₂NMe₂, -CH₂NH₂, -NHAc, -SO₂Me, -CONH₂, -CN, -NHCONH₂, -NHC(NH)NH₂, -NOHCH₃, -N₃, -NO₂, C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups.

2. A compound according to claim 1, wherein X is an optionally substituted phenylene group or an optionally substituted heteroarylene group having 5 or 6 ring atoms that are selected from C, N, O and S.

3. A compound according to claim 1, wherein X is a 1,3 phenylene group or a 1,4 phenylene group.

4. A compound according to any one of the preceding claims, wherein R¹ is a C₁₋₄ alkyl group; or a group of formula -CH₂-R¹¹, wherein R¹¹ is a phenyl group or a cyclopropyl group.

5. A compound according to any one of the preceding claims, wherein R¹ is a group of formula -CH₂CH(CH₃)₂ or a group of formula -CH(CH₃)₂.

6. A compound according to any one of the preceding claims, wherein R² is a group of formula -CH₂-NH-SO₂-R³ or -CH₂-N(CH₃)-SO₂-R³, wherein R³ is an optionally substituted phenyl group, an optionally substituted benzyl group, an optionally substituted C₃₋₁₀ cycloalkyl group, an optionally substituted -CH₂-C₃-₁₀ cycloalkyl group, a C₁₋₆ alkyl group or a C₁₋₆ heteroalkyl group.

7. A compound according to any one of the preceding claims 1 to 5, wherein R² is a group of formula -CH₂-Y-R⁴, wherein Y is selected from a bond, O, NH, S and NHCO; and R⁴ is hydrogen, a C₁₋₆ heteroalkyl group or an optionally substituted phenyl group.

8. A compound according to any one of the preceding claims 1 to 5, wherein R² is an optionally substituted phenyl group.

9. A compound according to any one of the preceding claims 1 to 5, wherein R² is selected from the following groups:

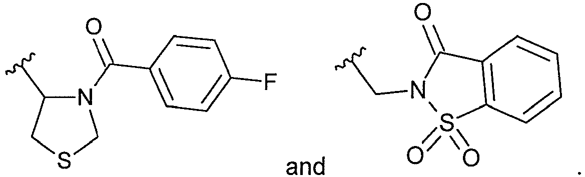

10. A pharmaceutical composition comprising a compound according to anyone of the preceding claims and optionally one or more carrier substances and/or one or more adjuvants and/or one or more further antibacterial compounds.

11. A compound according to any one of claims 1 to 9 or a pharmaceutical composition according to claim 10 for use in the treatment of bacterial infections.

12. A compound or a pharmaceutical composition for use according to claim 11, wherein the bacterial infection is caused by *P. aeruginosa.*

## Patentansprüche

1. Verbindung der Formel (II): 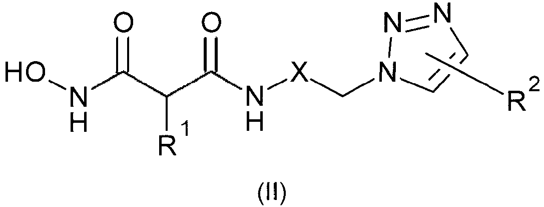 wobei
X eine optional substituierte Arylengruppe oder eine optional substituierte Heteroarylengruppe ist;
R¹ eine C₁₋₆-Alkylgruppe ist; oder eine Gruppe der Formel -CH₂-R¹¹ ist, wobei R¹¹ eine Phenylgruppe oder eine Cyclopropylgruppe ist; und
R² Wasserstoff oder eine Alkyl-, Alkenyl-, Alkinyl-, Heteroalkyl-, Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, die alle gegebenenfalls substituiert sein können;
oder ein pharmazeutisch verträgliches Salz davon ist;
wobei die optionalen Substituenten ausgewählt sind aus Fluor, Chlor, Brom und Jod sowie OH, =O, SH, NH₂, -SO₃H, -SO₂NH₂, -COOH, -COOMe, -COMe, - NHSO₂Me, -SO₂NMe₂, -CH₂NH₂, -NHAc, -SO₂Me, -CONH₂, -CN, -NHCONH₂, - NHC(NH)NH₂, -NOHCH₃, -N₃, -NO₂, C₁-C₁₀ Alkyl, C₂-C₁₀-Alkenyl, C₂-C₁₀-Alkinyl, C₁-C₁₀-Heteroalkyl, C₃-C₁₈-Cycloalkyl, C₁-C₁₇-Heterocycloalkyl, C₄-C₂₀-Alkylcycloalkyl, C₁-C₁₉-Heteroalkylcycloalkyl, C₆-C₁₈-Aryl-, C₁-C₁₇-Heteroaryl, C₇-C₂₀-Aralkyl- und C₁-C₁₉-Heteroaralkylgruppen.

2. Verbindung nach Anspruch 1, wobei X eine optional substituierte Phenylengruppe oder eine optional substituierte Heteroarylengruppe mit 5 oder 6 Ringatomen ist, die aus C, N, O und S ausgewählt sind.

3. Verbindung nach Anspruch 1, wobei X eine 1,3-Phenylengruppe oder eine 1,4-Phenylengruppe ist.

4. Verbindung nach einem der vorstehenden Ansprüche, wobei R¹ eine C₁₋₄-Alkylgruppe ist; oder eine Gruppe der Formel -CH₂-R¹¹ ist, wobei R¹¹ eine Phenylgruppe oder eine Cyclopropylgruppe ist.

5. Verbindung nach einem der vorstehenden Ansprüche, wobei R¹ eine Gruppe der Formel -CH₂CH(CH₃)₂ oder eine Gruppe der Formel -CH(CH₃)₂ ist.

6. Verbindung nach einem der vorstehenden Ansprüche, wobei R² eine Gruppe der Formel -CH₂-NH-SO₂-R³ oder -CH₂-N(CH₃)-SO₂-R³ ist, wobei R³ eine optional substituierte Phenylgruppe, eine optional substituierte Benzylgruppe, eine optional substituierte C₃₋₁₀-Cycloalkylgruppe, eine optional substituierte - CH₂-C₃₋₁₀-Cycloalkylgruppe, eine C₁₋₆-Alkylgruppe oder eine C₁₋₆-Heteroalkylgruppe ist.

7. Verbindung nach einem der vorstehenden Ansprüche 1 bis 5, wobei R² eine Gruppe der Formel -CH₂-Y-R⁴ ist, wobei Y aus einer Bindung, O, NH, S und NHCO ausgewählt ist; und R⁴ Wasserstoff, eine C₁₋₆-Heteroalkylgruppe oder eine optional substituierte Phenylgruppe ist.

8. Verbindung nach einem der vorstehenden Ansprüche 1 bis 5, wobei R² eine optional substituierte Phenylgruppe ist.

9. Verbindung nach einem der vorstehenden Ansprüche 1 bis 5, wobei R² aus den folgenden Gruppen ausgewählt ist:

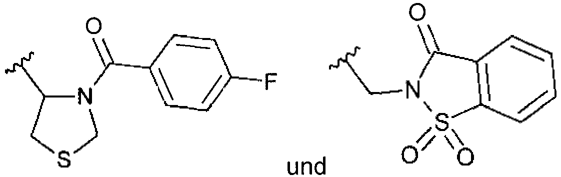

10. Pharmazeutische Zusammensetzung, die eine Verbindung nach einem der vorstehenden Ansprüche und optional eine oder mehrere Trägersubstanzen und/oder ein oder mehrere Hilfsstoffe und/oder eine oder mehrere weitere antibakterielle Verbindungen umfasst.

11. Verbindung nach einem der Ansprüche 1 bis 9 oder pharmazeutische Zusammensetzung nach Anspruch 10 zur Verwendung bei der Behandlung bakterieller Infektionen.

12. Verbindung oder pharmazeutische Zusammensetzung zur Verwendung nach Anspruch 11, wobei die bakterielle Infektion durch *P. aeruginosa* verursacht wird.

## Revendications

1. Composé de formule (II): 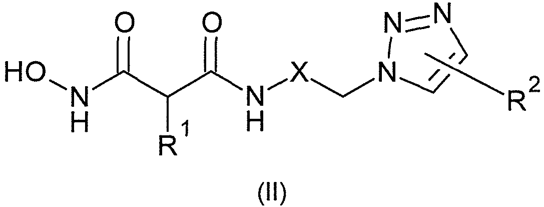 dans lequel
X représente un groupe arylène éventuellement substitué ou un groupe hétéroarylène éventuellement substitué;
R¹ est un groupe alkyle en C₁₋₆; ou un groupe de formule -CH₂-R¹¹, dans lequel R¹¹ est un groupe phényle ou un groupe cyclopropyle; et
R² représente un atome d'hydrogène ou un groupe alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle, tous pouvant être éventuellement substitués;
ou un sel pharmaceutiquement acceptable de celui-ci;
les substituants facultatifs étant choisis parmi le fluor, le chlore, le brome et l'iode, ainsi que parmi OH, =O, SH, NH₂, -SO₃H, -SO₂NH₂, -COOH, -COOMe, - COMe, -NHSO₂Me, -SO₂NMe₂, -CH₂NH₂, -NHAc, -SO₂Me, -CONH₂, -CN, - NHCONH₂, -NHC(NH)NH₂, -NOHCH₃, -N₃, -NO₂, C₁-C₁₀ alkyle, C₂-C₁₀ alcényle, C₂-C₁₀ alcynyle, C₁-C₁₀ hétéroalkyle, C₃-C₁₈ cycloalkyle, C₁-C₁₇ hétérocycloalkyle, C₄-C₂₀ alkylcycloalkyle, C₁-C₁₉ hétéroalkylcycloalkyle, C₆-C₁₈ aryle, C₁-C₁₇ hétéroaryle, C₇-C₂₀ aralkyle et C₁-C₁₉ hétéroaralkyle.

2. Composé selon la revendication 1, dans lequel X est un groupe phénylène éventuellement substitué ou un groupe hétéroarylène éventuellement substitué comportant 5 ou 6 atomes cycliques choisis parmi C, N, O et S.

3. Composé selon la revendication 1, dans lequel X est un groupe 1,3-phénylène ou un groupe 1,4-phénylène.

4. Composé selon l'une quelconque des revendications précédentes, dans lequel R¹ est un groupe alkyle en C₁₋₄; ou un groupe de formule -CH₂-R¹¹, dans lequel R¹¹ est un groupe phényle ou un groupe cyclopropyle.

5. Composé selon l'une quelconque des revendications précédentes, dans lequel R¹ est un groupe de formule -CH₂CH(CH₃)₂ ou un groupe de formule -CH(CH₃)₂.

6. Composé selon l'une quelconque des revendications précédentes, dans lequel R² est un groupe de formule -CH₂-NH-SO₂-R³ ou -CH₂-N(CH₃)-SO₂-R³, où R³ est un groupe phényle éventuellement substitué, un groupe benzyle éventuellement substitué, un groupe cycloalkyle en C₃₋₁₀ éventuellement substitué, un groupe -CH₂-C₃₋₁₀ cycloalkyle éventuellement substitué, un groupe alkyle en C₁₋₆ ou un groupe hétéroalkyle en C₁₋₆.

7. Composé selon l'une quelconque des revendications 1 à 5 précédentes, dans lequel R² est un groupe de formule -CH₂-Y-R⁴, où Y est choisi parmi une liaison, O, NH, S et NHCO; et R⁴ est un atome d'hydrogène, un groupe hétéroalkyle en C₁₋₆ ou un groupe phényle éventuellement substitué.

8. Composé selon l'une quelconque des revendications 1 à 5 précédentes, dans lequel R² est un groupe phényle éventuellement substitué.

9. Composé selon l'une quelconque des revendications 1 à 5 précédentes, dans lequel R² est choisi parmi les groupes suivants:

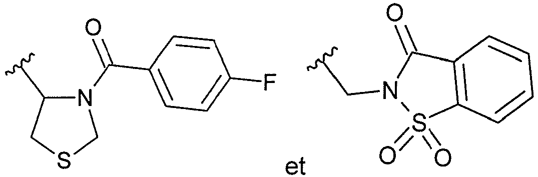

10. Composition pharmaceutique comprenant un composé selon l'une quelconque des revendications précédentes et eventuellement une ou plusieurs substances véhicules et/ou un ou plusieurs adjuvants et/ou un ou plusieurs autres composés antibactériens.

11. Composé selon l'une quelconque des revendications 1 à 9 ou composition pharmaceutique selon la revendication 10, destiné à être utilisé dans le traitement d'infections bactériennes.

12. Composé ou composition pharmaceutique destiné à être utilisé selon la revendication 11, dans lequel l'infection bactérienne est causée par P. *aeruginosa.*
